# FASCICULE DE BREVET EUROPEEN

(11) **EP 1 979 321 B1**
(45) Date de publication et mention de la délivrance du brevet: **29.07.2009**
(21) Numéro de dépôt: 07730888.0
(22) Date de dépôt: 30.01.2007
(51) Int. Cl.: C07D 213/65, C07D 401/10, A61K 31/4418, A61P 25/00

(54) **NOUVEAUX COMPOSES PYRIDINYLAMINOALKYLENE- ET PYRIDINYLOXYALKYLENE- CYCLOPROPANAMINES, LEUR PROCEDE DE PREPARATION ET LES COMPOSITIONS PHARMACEUTIQUES QUI LES CONTIENNENT**
NEUARTIGE PYRIDINYLAMINALKYLEN- UND PYRIDINYLOXALKYLEN-CYCLOPROPANAMINE, HERSTELLUNGSVERFAHREN DAFÜR UND PHARMAZEUTISCHE ZUSAMMENSETZUNGEN DAMIT
NOVEL PYRIDINYLAMINOALKYLENE- AND PYRIDINYLOXYALKYLENE-CYCLOPROPANAMINES, PROCESS FOR THE PREPARATION THEREOF AND PHARMACEUTICAL COMPOSITIONS CONTAINING THEM

(30) Priorité: 30.01.2006 FR 0600784
(43) Date de publication de la demande: 15.10.2008
(73) Titulaire: Les Laboratoires Servier, 92415 Courbevoie Cedex (FR)
(72) Inventeur: GOLDSTEIN, Solo, F-92150 Suresnes (FR); GUILLONNEAU, Claude, F-92140 Clamart (FR); CHARTON, Yves, F-92330 Sceaux (FR); LOCKHART, Brian, F-78810 Feucherolles (FR); LESTAGE, Pierre, F-78170 La Celle St Cloud (FR)
(86) Numéro de dépôt international: PCT/FR2007/000170
(87) Numéro de publication internationale: WO 2007/085750

(56) Documents cités:
- EP-A1- 1 170 281
- WO-A-00/75110
- WO-A-20/04078752
- WO-A2-01/70733

## Description

La présente invention concerne de nouveaux composés pyridinylaminoalkylene- et pyridinyloxyalkylene- cyclopropanamines polysubstitués, leur procédé de préparation et les compositions pharmaceutiques qui les contiennent.

Les composés de la présente invention sont particulièrement intéressants d'un point de vue pharmacologique pour leur interaction spécifique avec les récepteurs nicotiniques centraux de type α4β2, trouvant leur application dans le traitement des neuropathologies associées au vieillissement cérébral, des troubles de l'humeur, de la douleur et du sevrage tabagique.

Le vieillissement de la population par augmentation de l'espérance de vie à la naissance a entraîné parallèlement un large accroissement de l'incidence des neuropathologies liées à l'âge et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence. Il est largement démontré que parmi les différents neurotransmetteurs, l'acétylcholine tient une place prépondérante dans les fonctions de mémoire et que les voies neuronales cholinergiques sont dramatiquement détruites lors de certaines maladies neurodégénératives ou en déficit d'activation lors du vieillissement cérébral. C'est pourquoi, de nombreuses approches thérapeutiques ont visé à empêcher la destruction du neuromédiateur *via* l'inhibition de l'acétylcholine-estérase ou ont cherché à se substituer au neuromédiateur déficitaire. Dans ce dernier cas, les agonistes cholinergiques proposés ont été de type muscarinique, spécifiques pour les récepteurs post-synaptiques M1.

Récemment, il a été montré que l'atteinte cholinergique liée à la maladie d'Alzheimer touchait davantage les neurones portant les récepteurs nicotiniques que ceux portant les récepteurs muscariniques (Schroder et Coll., « Alzheimer disease : therapeutic strategies », Birkhauser Boston, 1994, 181-185). De plus, de nombreuses études ont démontré que la nicotine possède des propriétés facilitatrices de la mémoire (Prog. Neuropsychopharmacol., 1992, 16, 181-191) et que ces propriétés s'exercent tout autant sur les fonctions mnésiques (Psychopharmacol., 1996, 123, 88-97) que sur les facultés d'attention et de vigilance (Psychopharmacol., 1995, 118, 195-205). Par ailleurs, la nicotine exerce des effets neuroprotecteurs vis-à-vis d'agents excitotoxiques tel que le glutamate (Brain Res., 1994, 644, 181-187).

L'ensemble de ces données est très probablement à relier avec les études épidémiologiques qui ont montré une moindre incidence de maladie d'Alzheimer ou de Parkinson chez les sujets fumeurs. De plus, plusieurs études ont montré l'intérêt de la nicotine dans le traitement des troubles de l'humeur tels que les états dépressifs, anxieux ou schizophréniques. Enfin, il a été montré que la nicotine possède des propriétés antalgiques. L'ensemble des propriétés thérapeutiques de la nicotine, ainsi que celles décrites pour d'autres agents nicotiniques, est sous tendu par une activité vis-à-vis de récepteurs centraux qui diffèrent structurellement et pharmacologiquement des récepteurs périphériques (muscle et ganglion). Les récepteurs centraux de type α4β2 sont les plus représentés dans le système nerveux central et ont été impliqués dans la plupart des effets thérapeutiques de la nicotine (Life Sci., 1995, 56, 545-570).

Plusieurs documents tels que Synlett., 1999, 7, 1053-1054 ; J. Med. Chem, 1985, 28(12), 1953-1957 et 1980, 23(3), 339-341 ; 1970, 13(5), 820-826 ; 1972, 15(10), 1003-1006 ; J. Am. Chem. Soc., 1987, 109(13), 4036-4046, ou quelques brevets ou demandes de brevets comme DE 36 08 727, EP 124 208 ou WO 94/10158 décrivent et revendiquent des composés comportant un motif cyclopropanique 1,1 ou 1,2-disubstitué. Aucunes de ces références ne décrivent ou ne suggèrent pour ces composés une activité pharmacologique spécifique vis-à-vis des récepteurs nicotiniques et plus particulièrement vis-à-vis des récepteurs nicotiniques centraux de type α4β2, propriété originale des composés décrits par la Demanderesse. La demande de brevet EP 1 170 281 décrit des composés cyclopropaniques 1,1 et 1,2-disubstitués qui sont des ligands nicotiniques.

Les composés de la présente invention sont donc nouveaux et constituent de puissants ligands nicotiniques sélectifs du sous-type réceptoriel α4β2 central. De ce fait, ils sont utiles dans le traitement des déficits de mémoire associés au vieillissement cérébral et aux maladies neurodégénératives telles que la maladie d'Alzheimer, la maladie de Parkinson, la maladie de Pick, la maladie de Korsakoff et les démences frontales et sous-corticales, ainsi que pour le traitement des troubles de l'humeur, du syndrome de Tourette, du syndrome d'hyperactivité avec déficits attentionnels, du sevrage tabagique et de la douleur. Plus particulièrement, la présente invention concerne les composés de formule (I) : 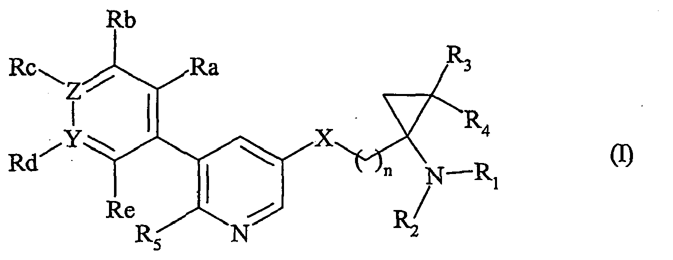 dans laquelle :
- n: représente un nombre entier compris entre 1 et 6 inclus,
- X: représente un atome d'oxygène ou un groupement NR₆,
- Y: représente un atome de carbone ou un atome d'azote, étant entendu que lorsque Y représente un atome d'azote Rd est absent,
- Z: représente un atome de carbone ou un atome d'azote, étant entendu que lorsque Z représente un atome d'azote, Rc est absent,
- R₁ et R₂,: identiques ou différents, indépendamment l'un de l'autre, représentent un atome d'hydrogène, ou un groupement alkyle (C₁-C₆) linéaire ou ramifié ou arylalkyle (C₁-C₆) linéaire ou ramifié,
- R₃ et R₄,: identiques ou différents, indépendamment l'un de l'autre, représentent un atome d'hydrogène, ou un groupement alkyle (C₁-C₆) linéaire ou ramifié,
- R₅: représente un atome d'hydrogène, ou un groupement alkyle (C₁-C₆) linéaire ou ramifié, halogène, hydroxy, alkoxy (C₁-C₆) linéaire ou ramifié, cyano, nitro, acyle (C₂-C₆) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, amino éventuellement substitué par un ou deux groupements alkyle (C₁-C₆) linéaire ou ramifié, aryle ou hétéroaryle,
- R₆: représente un atome d'hydrogène, ou un groupement alkyle (C₁-C₆) linéaire ou ramifié ou arylalkyle (C₁-C₆) linéaire ou ramifié,
- Ra, Rb, Rc, Rd et Re,: identiques ou différents, indépendamment l'un de l'autre, représentent un atome d'hydrogène, ou un groupement alkyle (C₁-C₆) linéaire ou ramifié, halogène, halogénoalkyle (C₁-C₆) linéaire ou ramifié, hydroxy, alkoxy (C₁-C₆) linéaire ou ramifié, hydroxyalkyle (C₁-C₆) linéaire ou ramifié, cyano, nitro, carboxy, isothiocyanate, acyle (C₂-C₆) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, alkylthio (C₁-C₆) linéaire ou ramifié, alkyle(C₁-C₆)carbonylamino la partie alkyle étant linéaire ou ramifié, halogénoalkyle(C₁-C₆)carbonylamino la partie alkyle étant linéaire ou ramifié, aminocarbonyle, amino éventuellement substitué par un ou deux groupements alkyle (C₁-C₆) linéaire ou ramifié, tétrazolyle,
par groupement aryle, on comprend un groupement phényle, biphényle, naphtyle, dihydronaphtyle, tétrahydronaphtyle, indanyle et indényle, chacun de ces groupements étant éventuellement substitués par un ou plusieurs groupements, identiques ou différents, choisis parmi halogène, alkyle (C₁-C₆) linéaire ou ramifié, hydroxy, cyano, nitro, alkoxy (C₁-C₆) linéaire ou ramifié, acyle (C₂-C₇) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, et amino éventuellement substitué par un ou deux groupements alkyle (C₁-C₆) linéaire ou ramifié,
par groupement hétéroaryle, on comprend un système monocyclique aromatique ou bicyclique de 5 à 12 chaînons, contenant de un à trois hétéroatomes, identiques ou différents, choisis parmi oxygène, azote et soufre, et dont l'un des cycles, dans le cas d'un système bicyclique, possède un caractère aromatique, l'autre cycle pouvant être aromatique ou partiellement hydrogéné, chacun de ces groupements pouvant être éventuellement substitué par un ou plusieurs groupements identiques ou différents, choisis parmi les substituants précédemment définis dans le cas d'un groupement aryle.

Selon une variante avantageuse de l'invention, les composés préférés sont les composés de formule (I/A) : 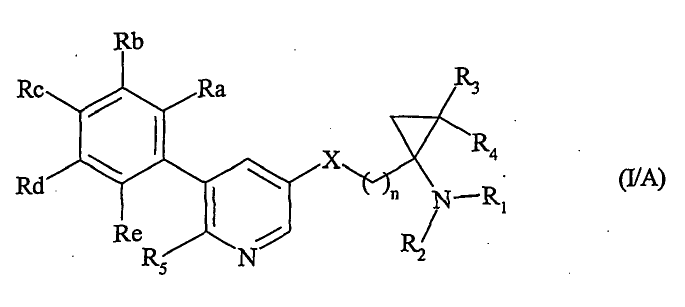 dans laquelle R₁, R₂, R₃, R₄, R₅, Ra, Rb, Rc, Rd, Re, X et n sont tels que définis précédemment.

Selon une deuxième variante avantageuse de l'invention, les composés préférés sont les composés de formule (I/B) : 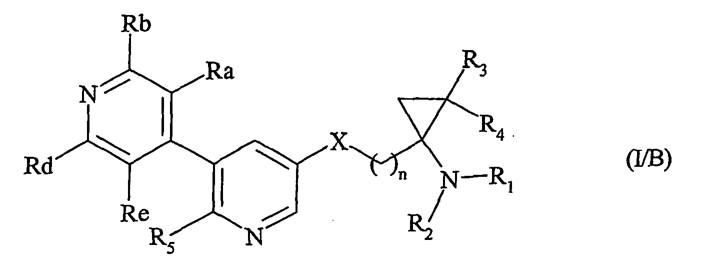 dans laquelle R₁, R₂, R₃, R₄, R₅, Ra, Rb, Rd, Re, X et n sont tels que définis précédemment.

Selon une troisième variante avantageuse de l'invention, les composés préférés sont les composés de formule (I/C) : 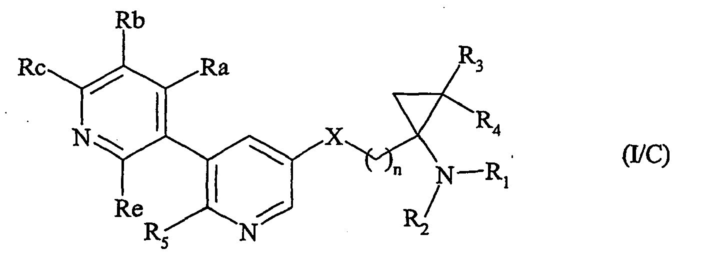 dans laquelle R₁, R₂, R₃, R₄, R₅, Ra, Rb, Rc, Re, X et n sont tels que définis précédemment.

Des composés préférés de l'invention sont les composés pour lesquels n est un entier prenant la valeur 1.

Les substituants R₁ et R₂ préférés selon l'invention sont l'atome d'hydrogène et le groupement alkyle (C₁-C₆) linéaire ou ramifié.

Plus préférentiellement, les substituants R₁ et R₂ préférés selon l'invention sont l'atome d'hydrogène et le groupement méthyle.

Les substituants R₃ et R₄ préférés selon l'invention sont l'atome d'hydrogène et le groupement méthyle.

Le substituant R₅ préféré selon l'invention est l'atome d'hydrogène, l'atome d'halogène et le groupement alkyle (C₁-C₆) linéaire ou ramifié.

Le substituant R₆ préféré selon l'invention est l'atome d'hydrogène et le groupement méthyle.

D'une façon avantageuse, les composés préférés de l'invention sont les composés pour lesquels Y représente un atome d'azote et Z représente un atome de carbone éventuellement substitué par Rc.

D'une façon avantageuse, les composés préférés de l'invention sont les composés pour lesquels Y représente un atome d'azote, Z représente un atome de carbone, Ra représente un atome d'hydrogène, Rb représente un atome d'hydrogène, Rc représente un atome d'hydrogène et Re représente un atome d'hydrogène.

D'une façon très avantageuse, les composés préférés de l'invention sont les composés pour lesquels Y représente un atome de carbone éventuellement substitué par Rd et Z représente un atome d'azote.

D'une façon avantageuse, les composés préférés de l'invention sont les composés pour lesquels Y représente un atome de carbone, Z représente un atome d'azote, Ra représente un atome d'hydrogène, Rb représente un atome d'hydrogène, Rd représente un atome d'hydrogène et Re représente un atome d'hydrogène.

La notation (1*S*,2*S*), (1*R*,2*R*) suivi du nom du composé signifie que le produit obtenu est un mélange racémique et donc, que les deux configurations sont présentes.
A titre d'exemple :
(1*S*,2*S*), (1*R*,2*R*)-2-Méthyl-1-[(3-pyridinyloxy)méthyl]cylopropanamine signifie que le produit obtenu, le mélange racémique, contient le (1*S*,2*S*)-2-méthyl-1-[(3-pyridinyloxy) méthyl]cyclopropanamine et le (1*R*,2*R*)-2-éthyl-1-[(3-pyridinyloxy)méthyl]cylopropanamine

La notation (*R ou S*) suivi du nom du composé signifie que le produit obtenu est un énantiomère optiquement pur. La présence de (-) et/ou (+) indique le signe du pouvoir rotatoire.

La notation (*R,S*) suivi du nom du composé signifie que le produit obtenu est un mélange racémique et donc, que les deux configurations sont présentes.

La notation (1*S*,2*S*) ou (1*R*,2*R*) suivi du nom du composé signifie que le produit obtenu est un énantiomère optiquement pur. La présence de (-) et/ou (+) indique le signe du pouvoir rotatoire.
A titre d'exemple :
Dichlorhydrate de (1*S*,2*S*) ou (1*R*,2*R*)-(-)-*N*,2-diméthyl-1-[(3-pyridinyloxy)méthyl] cyclopropanamine signifie que le produit obtenu, l'énantiomère optiquement pur, est le dichlorhydrate de (1*S*,2*S*)-(-)-*N*,2-diméthyl-1-[(3-pyridinyloxy)méthyl]cylopropanamine
ou le dichlorhydrate de (1*R*,2*R*)-(-)-*N*,2-diméthyl-1-[(3-pyridinyloxy)méthyl] cyclopropanamine

Par énantiomère α et énantiomère β, on entend les énantiomères optiquement purs du mélange racémique correspondant.

D'une façon particulièrement avantageuse, les composés préférés de l'invention sont le :
Dichlorhydrate de [1-({[5-(3-méthoxyphényl)pyridin-3-yl]oxy}méthyl)cyclopropyl]-méthylamine,
Dichlorhydrate de [1-({[6-chloro-5-(3-méthoxyphényl)pyridin-3-yl]oxy}méthyl)-cyclopropyl]méthylamine,
Dichlorhydrate de [1-({[5-(4-méthoxyphényl)pyridin-3-yl]oxy}méthyl)cyclopropyl]-méthylamine,
Chlorhydrate de [1-({[5-(4-chlorophényl)pyridin-3-yl]oxy}méthyl)cyclopropyl]-méthylamine,
Chlorhydrate de [1-({[6-chloro-5-(4-fluorophényl)pyridin-3-yl]oxy}méthyl)-cyclopropyl]méthylamine,
Dichlorhydrate de {1-[({6-chloro-5-[4-(méthylthio)phényl]pyridin-3-yl}oxy)méthyl]-cyclopropyl} méthylamine,
Chlorhydrate de [1-({[6-chloro-5-(3,5-dichlorophényl)pyridin-3-yl]oxy}méthyl)-cyclopropyl]méthylamine,
Chlorhydrate de N-[3-(2-chloro-5-{[1-(méthylamino)cyclopropyl]méthoxy}pyridin-3-yl)-phényl]acétamide,
Dichlorhydrate de 4-(2-chloro-5-{[1-(méthylamino)cyclopropyl]méthoxy}-pyridin-3-yl)benzoate d'éthyle,
Chlorhydrate de 4-(2-chloro-5-{[1-(méthylamino)cyclopropyl]méthoxy}pyridine-3-yl)benzamide,
Chlorhydrate de l'acide 4-(2-chloro-5-{[1-(méthylamino)cyclopropyl]méthoxy}pyridin-3-yl)benzoique,
Dichlorhydrate de (1-{[(2-chloro-3,4'-bipyridin-5-yl)oxy]méthyl}cyclopropyl)-méthylamine,
Dichlorhydrate de {1-[({6-chloro-5-[4-(2H-tétrazol-5-yl)phényl]pyridin-3-yl}oxy)-méthyl]cyclopropyl}méthylamine,
Dichlorhydrate de [1-({[5,6-bis(4-chlorophényl)pyridin-3-yl]oxy}méthyl)cyclopropyl]-méthylamine,
Trichlorhydrate de 5-(4-aminophényl)-6-méthyl-N-{[1-(méthylamino)cyclopropyl]-méthyl}pyridin-3-amine,

Les énantiomères et diastéréoisomères ainsi que leurs sels d'addition à un acide ou à une base pharmaceutiquement acceptable des composés préférés font partie intégrante de l'invention.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

La présente invention concerne également le procédé de préparation des composés de formule (I), caractérisé en ce qu'on utilise comme produit de départ un composé de formule (II) : 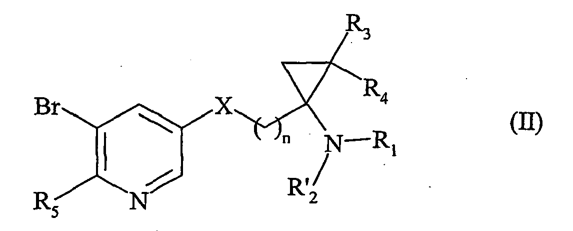 dans laquelle R'₂ représente un atome d'hydrogène, un groupement méthyle ou un groupement tert-butoxycarbonyl et R₁, R₃, R₄, R₅, X et n sont tels que définis dans la formule (I), composés de formule (II) qui sont mis à réagir avec un composé de formule (III) : 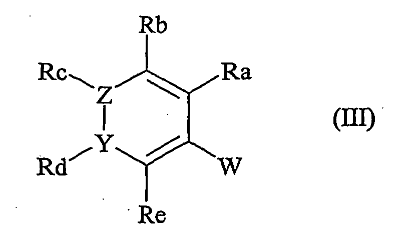 dans laquelle W représente un groupement -Sn(C₄H₉)₃, -B(OH)₂ et 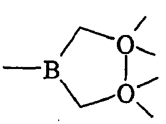 et Ra, Rb, Rc, Rd, Re, Y et Z sont tels que définis dans la formule (I), en présence de Pd(PPh₃)₄ en milieu basique pour conduire aux composés de formule (IV) : 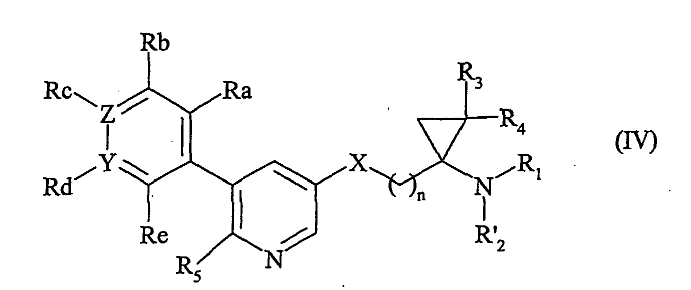 dans laquelle R₁, R'₂, R₃, R₄, R₅, X, Y, Z, Ra, Rb, Rc, Rd, Re et n sont tels que définis précédemment,
composés de formule (IV) qui sont mis en présence d'acide chlorhydrique, dans le cas où R'₂ représente un groupement tert-butoxycarbonyl, pour conduire aux composés de formule (I/a), cas particulier des composés de formule (I) : 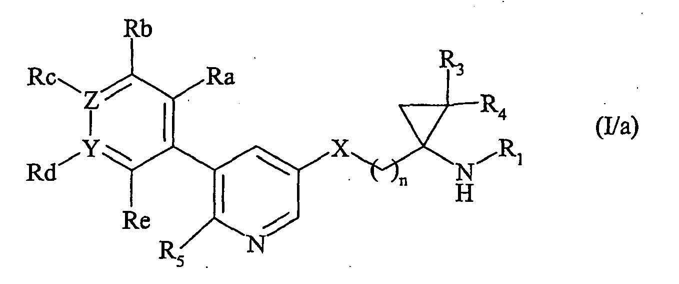 dans laquelle R₁, R₃, R₄, R₅, X, Y, Z, Ra, Rb, Rc, Rd, Re et n sont tels que définis précédemment,
composés de formule (I/a) qui sont mis à réagir avec un composé de formule (V) :

R"₂-L₂ (V)

dans laquelle R"₂ représente un groupement alkyle (C₁-C₆) linéaire ou ramifié ou arylalkyle (C₁-C₆) linéaire ou ramifié, et L₂ représente un groupement partant usuel de la chimie organique, en milieu basique pour conduire aux composés de formule (I/b), cas particulier des composés de formule (I) : 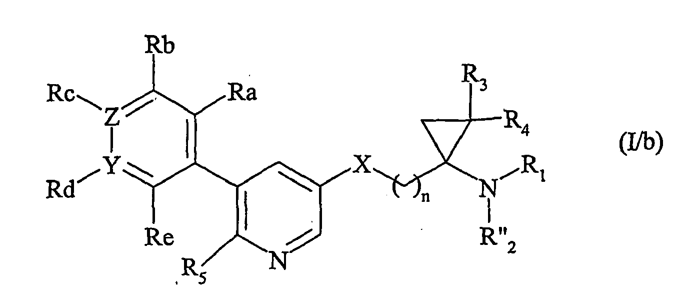 dans laquelle R₁, R"₂, R₃, R₄, R₅, X, Y, Z, Ra, Rb, Rc, Rd, Re et n sont tels que définis précédemment,
l'ensemble des composés de formules (I/a) et (I/b) formant l'ensemble des composés de l'invention qui sont purifiés, le cas échéant, selon des techniques classiques de purification, qui peuvent être séparés en leurs différents isomères, selon une technique classique de séparation et qui sont transformés, le cas échéant, en leurs sels d'addition à un acide ou à une base pharmaceutiquement acceptable.

Selon une variante de l'invention, les composés de formule (II), dans le cas où X représente un atome d'oxygène, R₃ et R₄ représentent chacun un atome d'hydrogène et R'₂ représente un groupement tert-butoxycarbonyl, de formule (II/a) : 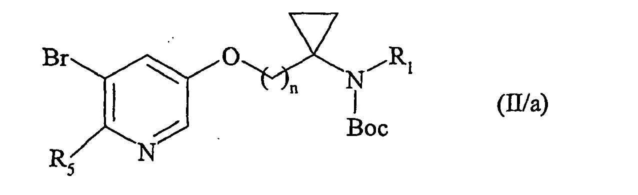 dans laquelle Boc représente un groupement tert-butoxycarbonyl et R₁, R₅ et n sont tels que définis précédemment,
peuvent être préparés à partir d'un composé de formule (VI) : 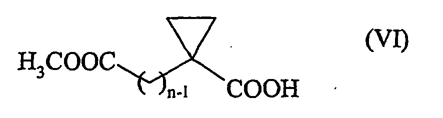 dans laquelle n est tel que défini précédemment, qui est mis à réagir avec du diphénylphosphoryle azide en milieu basique puis, mis en présence de tert-butanol pour conduire aux composés de formule (VII) : 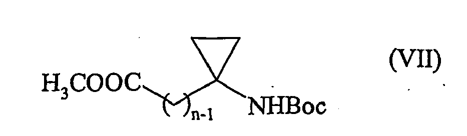 dans laquelle n et Boc sont tels que définis précédemment,
composés de formule (VII) qui sont mis à réagir avec un groupement de formule (VIII) :

R'₁-L₁ (VIII)

dans laquelle R'₁ représente un groupement alkyle (C₁-C₆) linéaire ou ramifié ou arylalkyle (C₁-C₆) linéaire ou ramifié, et L₁ représente un groupement partant usuel de la chimie organique, en milieu basique pour conduire aux composés de formule (IX) : 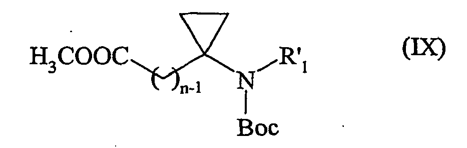 dans laquelle R'₁, Boc et n sont tels que définis précédemment, les composés de formules (VII) et (IX) formant les composés de formule (X) : 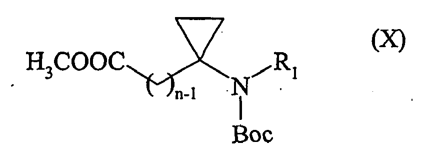 dans laquelle R₁ est tel que défini dans la formule (I) et Boc et n sont tels que définis précédemment,
composés de formule (X) qui sont mis en présence d'un agent réducteur pour conduire aux composés de formule (XI) : 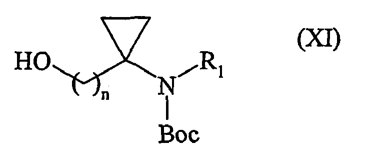 dans laquelle R₁, Boc et n sont tels que définis précédemment,
composés de formule (XI) qui sont mis en présence de tetrabromure de carbone et de triphénylphosphine pour conduire aux composés de formule (XII) : 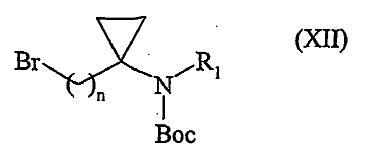 dans laquelle R₁, Boc et n sont tels que définis précédemment, composés de formule (XII) qui sont mis à réagir avec un composé de formule (XIII) : 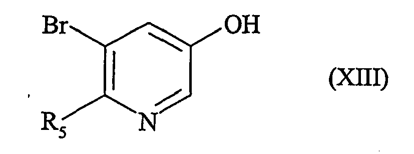 dans laquelle R₅ est tel que défini dans la formule (I), en milieu basique pour conduire aux composés de formule (II/a) tel que défini précédemment.

Selon une autre variante de l'invention, les composés de formule (II), dans le cas où X représente un atome d'oxygène, n prend la valeur 1, R'₂ représente un groupement méthyle et, l'un des groupements R₃ ou R₄ représente un groupement méthyle et l'autre groupement R₃ ou R₄ représente un atome d'hydrogène, de formule (II/b) : 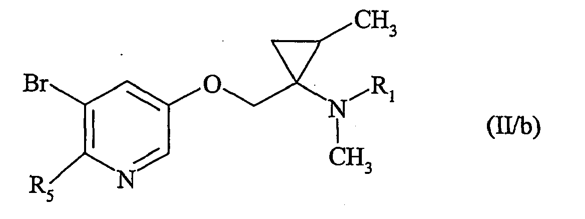 dans laquelle R₁ et R₅ sont tels que définis précédemment, peuvent être préparés à partir de 1,2-dibromopropane et d'isocyanate d'éthyle en milieu basique pour conduire au composé de formule (XIV) : 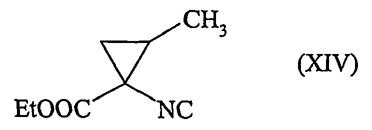 composé de formule (XIV) qui est mis en présence d'un agent réducteur, pour conduire au composé de formule (XV) : 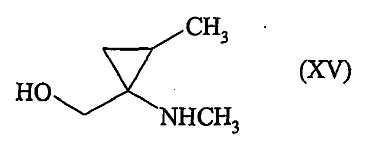 composé de formule (XV) qui est mis à réagir avec un composé de formule (XVI) : 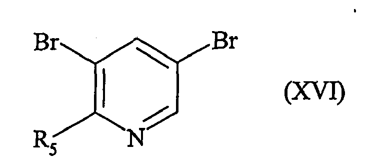 dans laquelle R₅ est tel que défini précédemment, en milieu basique pour conduire aux composés de formule (XVII) : 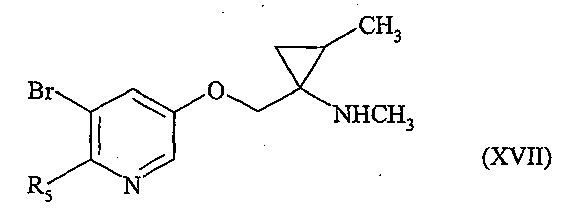 dans laquelle R₅ est tel que défini précédemment,
composés de formule (XVII) qui sont mis à réagir avec un composé de formule (VIII), tel que défini précédemment, dans les mêmes conditions que les composés de formule (VII), pour conduire aux composés de formule (XVIII) : 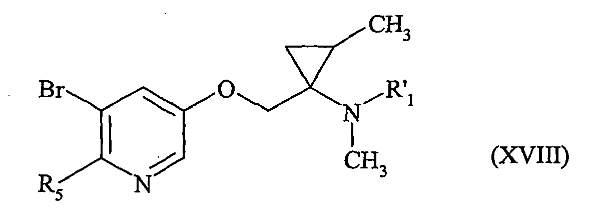 dans laquelle R'₁ et R₅ sont tels que définis précédemment,
les composés de formules (XVII) et (XVIII) formant les composés de formule (II/b), tel que défini précédemment.

Selon une autre variante de l'invention, les composés de formule (II), dans le cas où X représente un groupement NR₆ et R'₂ représente un groupement tert-butoxycarbonyl, de formule (II/c) : 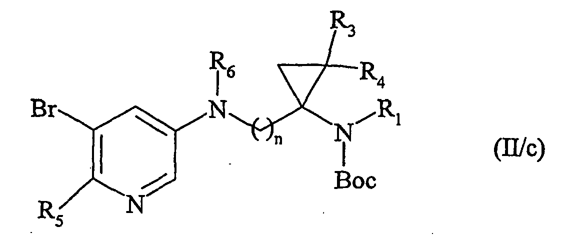 dans laquelle R₁, R₃, R₄, R₅, R₆, Boc et n sont tels que définis précédemment, peuvent être préparés à partir du composé de formule (XI), tel que défini précédemment, qui est mis en présence de chlorure d'oxalyle et de DMSO pour conduire aux composés de formule (XIX) : 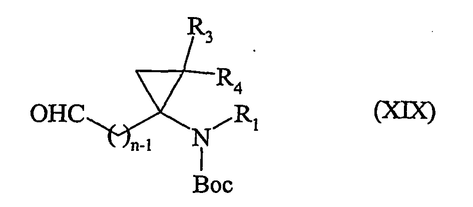 dans laquelle R₁, R₃, R₄, Boc et n sont tels que définis précédemment,
composés de formule (XIX) qui sont mis à réagir avec un composé de formule (XX) : 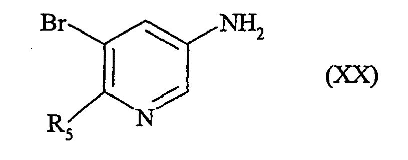 dans laquelle R₅ est tel que défini précédemment, en présence d'acide acétique suivi de cyanoborohydrure de sodium, pour conduire aux composés de formule (XXI) : 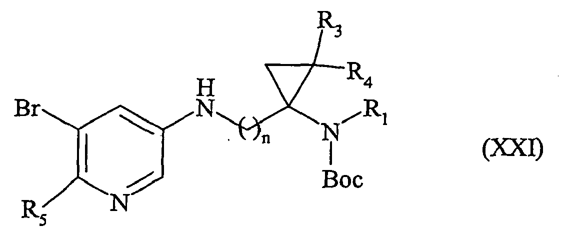 dans laquelle R₁, R₃, R₄, R₅, Boc et n sont tels que définis précédemment,
composés de formule (XXI) qui sont :
- soit mis en présence d'acide formique et d'anhydride acétique pour conduire aux composés de formule (XXII) : 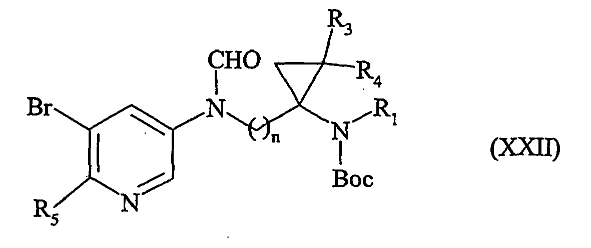
dans laquelle R₁, R₃, R₄, R₅, Boc et n sont tels que définis précédemment,
composés de formule (XXII) qui sont mis en présence de borane diméthylsulfure complexe pour conduire aux composés de formule (XXIII) : 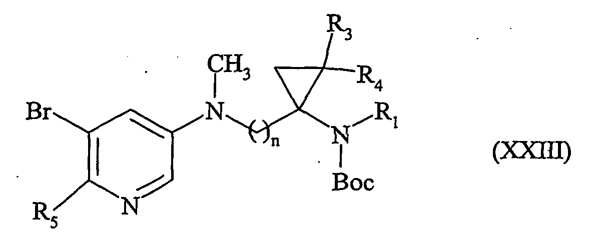 dans laquelle R₁, R₃, R₄, R₅, Boc et n sont tels que définis précédemment,
- soit mis à réagir avec un composé de formule (XXIV) :

   R'₆ - L₆ (XXIV)

   dans laquelle R'₆ représente un groupement alkyle (C₁-C₆) linéaire ou ramifié et arylalkyle (C₁-C₆) linéaire ou ramifié, et L₆ représente un groupement partant usuel de la chimie organique, en milieu basique pour conduire aux composés de formule (XXV) : 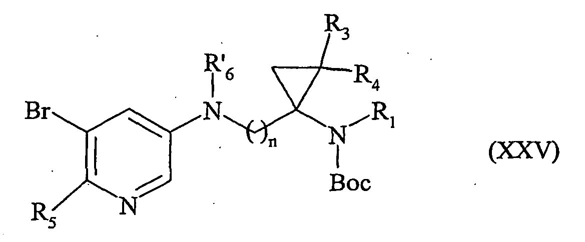
dans laquelle R₁, R₃, R₄, R₅, R'₆, Boc et n sont tels que définis précédemment, les composés de formules (XXI), (XXIII) et (XXV) formant les composés de formules (II/c), tel que défini précédemment.

Les composés de la présente invention, de part leurs propriétés pharmacologiques de ligands nicotiniques, et sélectivement du sous-type réceptoriel α4β2, sont utiles dans le traitement des déficits de mémoire associés au vieillissement cérébral et aux maladies neurodégénératives telles que la maladie d'Alzheimer, la maladie de Parkinson, la maladie de Pick, la maladie de Korsakoff et les démences frontales et sous-corticales, ainsi que pour le traitement des troubles de l'humeur, du syndrome de Tourette, du syndrome d'hyperactivité avec déficits attentionnels, du sevrage tabagique et de la douleur.

La présente invention a également pour objet les compositions pharmaceutiques renfermant comme principe actif au moins un composé de formule (I), un de ses isomères, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 1 mg à 500 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utile pour la préparation des composés de l'invention.

Les structures des composés décrits dans les exemples et dans les préparations ont été déterminées selon les techniques spectrophotométriques usuelles (infrarouge, résonance magnétique nucléaire, spectrométrie de masse, ...).

Les points de fusion ont été déterminés soit à la platine chauffante de Kofler, soit à la platine chauffante sous microscope.

### PREPARATION 1 :

### (1-{[(5-Bromopyridin-3-yl)oxy]méthyl}cyclopropyl)methylcarbamate de tert-butyle

### Stade 1 : 1-[(tert-Butoxycarbonyl)amino]cyclopropanecarboxylate de méthyle

Une solution de 80 g d'acide 1-(méthoxycarbonyl)cyclopropanecarboxylique, 78 ml de triéthylamine dans 550 ml de toluène, additionnée de 152 g de diphénylphosphoryle azide est chauffée à 80°C. Après arrêt de dégagement gazeux, la température est ramenée à 50°C et 61 g de *tert*-butanol sont ajoutés. Après 7 heures de réaction à 80°C, le milieu est concentré. Le résidu est repris à l'éther, lavé par une solution saturée en Na₂CO₃ puis par une solution d'acide chlorhydrique 1N, puis par une solution de NaHCO₃. Après séchage, et évaporation de la phase organique, le résidu est repris par 300 ml de cyclohexane, puis concentré à sec. Le résidu obtenu est trituré dans le pentane, filtré puis séché permettant d'isoler le produit attendu.

### Stade 2 : 1-[(tert-Butoxycarbonyl)(méthyl)amino]cyclopropanecarboxylate de méthyle

A une solution refroidie à 5°C de 99,7 g du composé obtenu au stade 1 précédent dans 1,71 de diméthylformamide anhydre sont ajoutés par fractions 24,7 g d'hydrure de sodium à 60 %. Après 15 minutes à 5°C puis 3 heures à température ambiante, 38,2 ml d'iodure de méthyle sont additionnés goutte à goutte. Après 20 heures de réaction, le milieu est évaporé. Le résidu est repris dans l'éther puis traité de façon classique. Une chromatographie sur gel de silice (dichlorométhane) permet d'isoler le produit attendu.

### Stade 3 : 1-(Hydroxyméthyl)cyclopropyl(méthyl)carbamate de tert-butyle

A une solution de 23 g du composé obtenu au stade 2 précédent dans 100 ml de tétrahydrofurane est additionnée une solution de 100 ml de borohydrure de lithium 2M dans le tétrahydrofurane. Après 20 heures d'agitation à température ambiante, puis 8 heures au reflux, le milieu réactionnel est refroidi à 0°C, hydrolysé, dilué à l'éther, décanté, séché et concentré. Une chromatographie sur gel de silice du résidu (dichlorométhane/tétrahydrofurane : 95/5) permet d'isoler le produit attendu.

### Stade 4 : 1-(bromométhyl)cyclopropyl(méthyl)carbamate de tert-butyle

A une solution de 4 g du composé obtenu au stade 3 précédent dans 100 ml d'éther sont additionnés à 20°C 7,9 g de triphénylphosphine puis 9,9 g de tetrabromométhane. Après 24 heures d'agitation, filtration et concentration à sec, une chromatographie sur gel de silice (dichlorométhane) permet d'isoler le produit attendu.
Point de fusion : 62-64°C

### Stade 5 : (1-{[(5-Bromopyridin-3-yl)oxy]méthyl}cyclopropyl)méthylcarbamate de tert-butyle

12,3 g d'hydroxyde de potassium en poudre sont ajoutés à une solution de 13,1 g de 5-bromopyridin-3-ol dans 375 ml de DMF. Le milieu réactionnel est agité 40 minutes puis une solution de 24,3 g du composé obtenu au stade 4 précédent dans 115 ml de DMF est additionnée en 20 minutes. On porte 8 heures à 85 °C puis le DMF est évaporé. Le résidu est repris par une solution aqueuse à 10 % de chlorure de lithium et extrait plusieurs fois à l'acétate d'éthyle, séché sur sulfate de sodium puis évaporé. La chromatographie sur silice (dichlorométhane/tétrahydrofurane : 98/2) permet d'obtenir 23,9 g du produit attendu.

### PREPARATION 2 :

### (1-{[(5-Bromo-6-chloropyridin-3-yl)oxy]méthyl}cyclopropyl)méthylcarbamate de tert-butyle

9,5 g de carbonate de césium sont ajoutés à une solution de 7,3 g du composé obtenu au stade 4 de la préparation 1 et de 7,5 g de 5-bromo-6-chloropyridin-3-ol dans 200 ml de 2-butanone. Le milieu réactionnel est porté 20 heures au reflux puis la butanone est évaporée. Le résidu est repris par une solution aqueuse saturée de carbonate de sodium puis extrait plusieurs fois à l'éther. Les phases éthérées réunies sont ensuite lavées avec des solutions aqueuses saturées de carbonate de sodium et de chlorure de sodium puis séchées sur sulfate de sodium et concentrées pour obtenir 10,6 g du produit attendu.

### PREPARATION 3 :

### (1-{[(5-Bromo-6-méthylpyridin-3-yl)oxy]méthyl}cyclopropyl)méthylcarbamate de tert-butyle

13 g de carbonate de césium sont ajoutés à une solution de 10,5 g du composé obtenu au stade 4 de la préparation 1 et de 7,5 g de 5-bromo-6-méthylpyridin-3-ol dans 300 ml de 2-butanone. Le milieu réactionnel est porté 20 heures au reflux. Après retour à température ambiante, les minéraux sont filtrés et la butanone évaporée. Une chromatographie sur silice (dichlorométhane/butanone : 95/5) permet d'obtenir 14,8 g du produit attendu.

### PREPARATION 4 :

### (1-{[(5-Bromo-6-fluoropyridin-3-yl)oxy]méthyl}cyclopropyl)méthylcarbamate de tert-butyle

12,9 g de carbonate de césium sont ajoutés à une solution de 10,5 g du composé obtenu au stade 4 de la préparation 1 et 5,8 g 5-bromo-6-fluororopyridin-3-ol dans 300 ml de butanone. Le milieu réactionnel est chauffé 20 h au reflux puis filtré et concentré. Le résidu est repris dans le dichlorométhane. Après lavage avec une solution saturée de chlorure de sodium et séchage sur sulfate de sodium, la phase organique est concentrée. Une chromatographie sur silice (dichlorométhane/butanone : 98/2) permet d'obtenir 10,7 g de produit attendu.

### PREPARATION 5 :

### (1-{[(5-Bromo-6-chloropyridin-3-yl)oxy]méthyl}cyclopropyl)carbamate de tert-butyle

### Stade 1 : 1-(Hydroxyméthyl)cyclopropylcarbamate de tert-butyle

A une solution de 23 g du composé du stade 1 de la préparation 1 dans 100 ml de tétrahydrofurane est additionnée une solution de 100 ml de borohydrure de lithium 2M dans le tétrahydrofurane. Après 20 heures d'agitation à température ambiante, puis 8 heures au reflux, le milieu réactionnel est refroidi à 0°C, hydrolysé, dilué à l'éther, décanté, séché et concentré. Une chromatographie sur gel de silice du résidu (dichlorométhane/tétrahydrofurane : 95/5) permet d'isoler le produit attendu.
Point de fusion : 80-82°C

### Stade 2 : [1-(Bromométhyl)cyclopropyl]carbamate de tert-butyle

Une solution de 92,5 g de tétrabromure de carbone dans 150 ml d'éther est ajoutée à température ambiante à une solution de 34,5 g du composé du stade 1 précédent et de 73,5 g de triphénylphosphine dans 750 ml d'éther. Après 20 h d'agitation le milieu réactionnel est filtré et concentré. Une chromatographie sur silice (dichlorométhane/cyclohexane : 50/50) permet d'obtenir 15 g de produit attendu.

### Stade 3 : (1-{[(5-Bromo-6-chloropyridin-3-yl)oxy]méthyl}cyclopropyl)carbamate de tert-butyle

Le composé est obtenu selon le procédé de la préparation 3 en utilisant le composé du stade 2 précédent et en remplaçant la 5-bromo-6-méthylpyridin-3-ol par la 5-bromo-6-chloropyridin-3-ol.

### PREPARATION 6 :

### (1S,2R), (1R,2S)-1-{[(5-Bromo-3-pyridinyl)oxy]méthyl}-N,2-diméthylcyclopropanamine

### Stade 1 : (1R,2S), (1S,2R)-1-Isocyano-2-méthylcyclopropanecarboxylate d'éthyle

Une solution de 2,5 g d'isocyanate d'éthyle, 2,3 cm³ de 1,2-dibromopropane, 25 cm³ de diméthyle sulfoxyde et 60 cm³ d'éther est additionnée goutte à goutte, en une heure, à une suspension de 1,93 g d'hydrure de sodium à 60 % dans l'huile dans 20 cm³ d'éther. Après 2 heures de chauffage au reflux, le milieu réactionnel est refroidi et coulé sur un mélange de 50 cm³ d'eau glacée et 50 cm³ d'éther. La phase aqueuse est décantée et extraite de nouveau à l'éther (3 x 40 cm³). Les phases organiques réunies sont lavées avec une solution aqueuse de chlorure de sodium, séchées sur sulfate de sodium et évaporées. La chromatographie sur silice (dichlorométhane/tétrahydrofuranne : 97/3), permet d'obtenir 4,88 g de produit attendu.
Rapport diastéréoisomérique : 90/10.

### Stade 2 : [(1R,2S), (1S,2R)-2-Méthyl-1-(méthylamino)cyclopropyl]méthanol

Une solution de 4,88 g du composé obtenu au stade 1 précédent dans 85 cm³ d'éther est additionnée goutte à goutte à une suspension de 3,73 g d'hydrure d'aluminium lithium dans 250 cm³ d'éther. Le mélange réactionnel est porté 4 heures au reflux puis agité 16 heures à température ambiante. Le milieu réactionnel est refroidi dans un bain de glace avant ajout de sulfate de sodium imprégné d'eau. Après deux heures d'agitation les minéraux sont filtrés, la phase éthérée est séchée sur sulfate de sodium puis évaporée pour obtenir 2,75 g du produit attendu.
Rapport diastéréoisomérique : 90/10.

### Stade 3 : (1S,2R), (1R,2S)-1-{[(5-Bromo-3-pyridinyl)oxy]méthyl}-N,2-diméthylcyclopropanamine

1,7 g d'hydrure de sodium à 60% dans l'huile sont ajoutés à 4,6 g du composé obtenu au stade 2 précédent dans 160 cm³ de diméthylformamide. Le milieu réactionnel est agité une heure à température ambiante puis 10,2 g de 3,5-dibromopyridine sont additionnés goutte à goutte. Le milieu réactionnel est chauffé 16 heures à 60°C puis le diméthylformamide est évaporé. Le résidu est repris dans 300 cm³ d'éther. La phase organique est lavée avec une solution aqueuse de chlorure de lithium puis séchée sur sulfate de sodium et concentrée. La chromatographie sur silice (dichlorométhane/méthanol : 96/4), permet d'obtenir 4,86 g du composé attendu.
Spectrométrie de masse (ESI) : m/z = 271,1 Th ([M+H]⁺)

### PREPARATION 7 :

### 1-(Formyl)cyclopropyl(méthyl)carbamate de tert-butyle

A une solution contenant 25,8 g de chlorure d'oxalyle dans 430 ml de dichloromethane, on ajoute, à -60°C, 33,5 g de diméthylsulfoxyde en 20 minutes Après 20 minutes d'agitation à -60°C, on ajoute un mélange contenant 34,3 g du composé du stade 3 de la préparation 1 dans 100 ml de dichlorométhane en 1 heure à -60°C. Après 30 minutes d'agitation à -60°C, on coule 81 ml de triéthylamine en 20 minutes à -60°C puis on laisse remonter la température à 20°C. On coule 60 ml d'eau, décante et extrait plusieurs fois la phase aqueuse au dichlorométhane. Les phases dichlorométhane jointes sont lavées avec une solution saturée de chlorure de sodium et séchées sur sulfate de sodium puis concentré à sec. Une chromatographie sur gel de silice (dichlorométhane/tétrahydrofurane : 97/3) permet d'obtenir 31,2 g du produit attendu.

### PREPARATION 8 :

### (1-{[(5-Bromopyridin-3-yl)amino]méthyl}cyclopropyl)méthylcarbamate de tert-butyle

On ajoute 6 ml d'acide acétique à une solution contenant 6 g du composé de la préparation 7 et 5,2 g (0,03 mol) de 3-amino-5-bromopyridine dans 60 mL de méthanol. On agite 30 minutes à température ambiante. On refroidit à 5°C et on ajoute par portions 2.44 g de cyanoborohydrure de sodium. On agite 4 jours à température ambiante. On coule 6,3 ml d'eau et concentre à sec. On reprend le résidu dans 30 mL de solution saturée de carbonate de potassium dans l'eau et on extrait au dichlorométhane. Le filtrat est séché sur sulfate de sodium puis concentré. Une chromatographie sur silice (dichlorométhane/butanone : 90/10) permet d'obtenir 7,6 g du produit attendu.
Point de fusion (cap) : 96°C

### PREPARATION 9 :

### (1-{[(5-Bromopyridin-3-yl)(méthyl)amino]méthyl}cyclopropyl)méthylcarbamate de tert-butyle

### Stade 1: (1-{[(5-Bromopyridin-3-yl)(formyl)amino]méthyl}cyclopropyl)méthylcarbamate de tert-butyle

On ajoute à 7,64 g du composé de la préparation 8 à 0°C en 20 minutes 2,69 mL d'acide formique dans 5,38 mL d'anhydride acétique. On porte à 50°C pendant 2 heures. On laisse refroidir à 20°C et on ajoute 5,38 mL de tetrahydrofurane. On refroidit à -20°C. On ajoute 7.64 g du composé de la préparation 8 dissous dans 11 mL de tetrahydrofurane.On agite une 1 heure à -20°C puis on maintient 20 heures à 0°C. On concentre à sec et on reprend le résidu dans le dichlorométhane. On lave deux fois avec une solution aqueuse à 10% de carbonate de sodium, sèche sur sulfate de sodium et concentre à sec. Une chromatographie sur silice (dichlorométhane/butanone : 90/10) permet d'obtenir 8,09 g de produit attendu.

### Stade 2 : (1-{[(5-Bromopyridin-3-yl)(méthyl)amino]méthyl}cyclopropyl)méthylcarbamate de tert-butyle

On coule à 0°C 5 mL de borane diméthylsulfure complexe (BMS) dans une solution de 7,7 g (0,02 mol) du produit obtenu au stade 1 précédent dans 80 mL de tétrahydrofurane. On laisse remonter la température à 20°C puis on porte au reflux 3 heures. On refroidit à 0°C puis on ajoute 10 mL de méthanol goutte à goutte. On concentre à sec et reprend le résidu dans le dichlorométhane. On lave avec une solution aqueuse à 10% de carbonate de sodium, sèche sur sulfate de sodium et concentre à sec. Une chromatographie sur silice (dichlorométhane) permet d'obtenir 5,68 g du produit attendu.

### PREPARATION 10 :

### (1-{[(5-Bromo-6-chloropyridin-3-yl)amino]méthyl}cyclopropyl)méthylcarbamate de tert-butyle

Le composé est obtenu selon le procédé de la préparation 8 en remplaçant la 3-amino-5-bromopyridine par la 3-amino-5-bromo-6-chloropyridine.

### PREPARATION 11 :

### (1-{[(5-Bromo-6-chloropyridin-3-yl)(méthyl)amino]méthyl}cyclopropyl)méthylcarbamate de tert-butyle

### Stade 1 : (1-{[(5-Bromo-6-chloropylridin-3-yl)(formyl)amino]méthyl} cyclopropyl)méthylcarbamate de tert-butyle

Le composé est obtenu selon le procédé du stade 1 de la préparation 9 en remplaçant le composé de la préparation 8 par le composé de la préparation 10.

### Stade 2 : (1-{[(5-Bromo-6-chloropyridin-3-yl)(méthyl)maino]méthyl}cyclopropyl)méthyl carbamate de tert-butyle

Le composé est obtenu selon le procédé du stade 2 de la préparation 9 en utilisant le composé obtenu au stade 1 précédent.

### PREPARATION 12 :

### (1-{[(5-Bromo-6-méthylpyridin-3-yl)amino]méthyl}cyclopropyl)méthylcarbamate de tert-butyle

Le composé est obtenu selon le procédé de la préparation 8 en remplaçant la 3-amino-5-bromopyridine par la 3-amino-5-bromo-6-méthylpyridine.

### PREPARATION 13 :

### (1-{[(5-Bromo-6-méthylpyridin-3-yl)méthyl)amino]méthyl}cyclopropyl)méthylcarbamate de tert-butyle

### Stade 1 : (1-{[(5-Bromo-6-méthylpyridin-3-yl)(formyl)amino]méthyl}cyclopropyl)méthylcarbamate de tert-butyle

Le composé est obtenu selon le procédé du stade 1 de la préparation 9 en remplaçant le composé de la préparation 8 par le composé de la préparation 12.

### Stade 2 : (1-{[(5-Bromo-6-méthylpyridin-3-yl)méthyl)amino]méthyl}cyclopropyl)méthylcarbamate de tert-butyle

Le composé est obtenu selon le procédé du stade 2 de la préparation 9 en utilisant le composé obtenu au stade 1 précédent.

### Exemple 1 :

### Dichlorhydrate de [1-({[5-(3-méthoxyphényl)pyridin-3-yl]oxy}méthyl)cyclopropyl]-méthylamine

### Stade 1 : [1-({[5-(3-méthoxyphényl)pyridin-3-yl]oxy}méthyl)cyclopropyl]-méthylcarbamate de tert-butyle

1 g de tetrakis(triphenylphosphine)palladium sont ajoutés sous azote à une solution de 6,3 g du composé de la préparation 1 dans 120 ml de toluène. Le milieu est agité 20 minutes puis une solution de 4,09 g d'acide (3-méthoxyphényl)boronique dans 110 ml d'éthanol et 60 ml d'une solution aqueuse saturée d'hydrogénocarbonate de sodium sont ajoutés. Le milieu réactionnel est porté 4 h à 80 °C puis filtré et décanté. La phase organique est lavée avec une solution à 10 % d'hydrogénocarbonate de sodium puis avec une solution à 10 % de chlorure de sodium puis séchée sur sulfate de sodium et concentrée. Une chromatographie du résidu sur silice (dichlorométhane/tétrahydrofuranne : 97/3) permet d'obtenir 5,06 g du produit attendu.

### Stade 2 : Dichlorhydrate de [1-({[5-(3-méthoxyphényl)pyridin-3-yl]oxy}méthyl)-cycloproypl]méthylamine

50 ml d'une solution d'acide chlorhydrique 4N dans le dioxanne sont ajoutés à une solution de 5 g du composé obtenu au stade 1 précédent dans 25 ml de dioxanne. Le milieu est agité 20 h, dilué à l'éther puis filtré pour obtenir 4,6 g du produit désiré.
Point de fusion (cap) : 210-212°C
Spectrométrie de masse (ESI) m/z = 285,1582 Th ([M+H]⁺)

### Exemple 2 :

### Dichlorhydrate de méthyl[1-({[5-(4-méthylphényl)pyridin-3-yl]oxy}méthyl)cyclopropyl]amine

### Stade 1: Méthyl[1-({[5-(-4-méthylphényl)pyridin-3-yl]oxy}méthyl)cyclopropyl]carbamate-de tert-butyle

Le composé est obtenu selon le procédé du stade 1 de l'exemple 1 en remplaçant l'acide (3-méthoxyphényl)boronique par l'acide (4-méthylphényl)boronique.

### Stade 2 : Dichlorhydrate de méthyl[1-({[5-(4-méthylphényl)pyridin-3-yl]-oxy}méthyl)cyclopropyl]amine

10 ml d'une solution d'acide chlorhydrique 4N dans le dioxanne sont ajoutés à une solution de 0,78 g du composé obtenu au stade 1 précédent dans 5 ml de dioxanne. Le milieu est agité 20 h puis le solvant est évaporé. Le résidu est dissous dans l'éthanol et l'éthanol puis évaporé. Le produit cristallisé est agité en présence d'éther puis filtré et séché pour obtenir 0,7 g (98%) du produit attendu.
Point de fusion (cap) : 218-223°C
Spectrométrie de masse (ESI) m/z = 269,1643 Th ([M+H]⁺)

### Exemple 3 :

### Dichlorhydrate de [1-({[5-(4-méthoxyphényl)pyridin-3-yl]oxy}méthyl)cyclopropyl]-méthylamine

### Stade 1 : [1-({[5-(4-méthoxyphényl)pyridin-3-yl]oxy}méthyl)cyclopropyl]-méthylcarbamate de tert-butyle

Le composé est obtenu selon le procédé du stade 1 de l'exemple 1 en remplaçant l'acide (3-méthoxyphényl)boronique par l'acide (4-méthoxyphényl)boronique.

### Stade 2 : Dichlorhydrate de [1-({[5-(4-méthoxyphényl)pyridin-3-yl]oxy}méthyl) cyclopropyl]méthylamine

Le composé est obtenu selon le procédé du stade 2 de l'exemple 2 en utilisant le composé obtenu au stade 1 précédent.
Point de fusion (cap) : 215-220°C
Spectrométrie de masse (ESI) m/z = 285,1585 Th ([M+H]⁺)

### Exemple 4 :

### Dichlorhydrate de méthyl(1-{[(5-phénylpyridin-3-yl)oxy]méthyl}cyclopropyl)amine

### Stade 1 : Méthyl(1-{[(5-phénylpyridin-3-yl)oxy]méthyl}cyclopropyl)carbamate de tert-butyle

Le composé est obtenu selon le procédé du stade 1 de l'exemple 1 en remplaçant l'acide (3-méthoxyphényl)boronique par l'acide phénylboronique.

### Stade 2 : Dichlorhydrate de méthyl(1-{[(5-phénylpyridin-3-yl)oxy]méthyl}-cycloproyl)amine

Le composé est obtenu selon le procédé du stade 2 de l'exemple 2 en utilisant le composé
obtenu au stade 1 précédent.
Point de fusion (cap) : 205-210°C
Spectrométrie de masse (ESI) m/z = 255,1481 Th ([M+H]⁺)

### Exemple 5 :

### Dichlorhydrate de [1-({[5-(4-fluorophényl)pyridin-3-yl]oxy}méthyl)cyclopropyl]-méthylamine

### Stade 1 : [1-({[5-4-Fluorophényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (4-fluorophényl)boronique.

### Stade 2 : Dichlorhydrate de [1-({[5-(4-fluorophényl)pyridin-3-yl]oxy}méthyl) cyclopropyl]méthylamine

Le composé est obtenu selon le procédé du stade 2 de l'exemple 2 en utilisant le composé obtenu au stade 1 précédent.
Point de fusion (cap) : 218-223°C
Spectrométrie de masse (ESI) m/z = 273,1402 Th ([M+H]⁺)

### Exemple 6 :

### Dichlorhydrate de [1-({[5-(4-nitrophényl)pyridin-3-yl]oxy}méthyl)cyclopropyl]-méthylamine

### Stade 1 : [1-({[5-(4-Nitroyhényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (4-nitrophényl)boronique.

### Stade 2 : Dichlorhydrate de [1-({[5-(4-nitrophényl)pyridin-3-yl]oxy}méthyl)cyclopropyl]-méthylamine

Le composé est obtenu selon le procédé du stade 2 de l'exemple 2 en utilisant le composé obtenu au stade 1 précédent.
Point de fusion (cap) : 212-217°C
Spectrométrie de masse (ESI) m/z = 300,1340 Th ([M+H]⁺)

### Exemple 7 :

### Dichlorhydrate de [1-({[5-(4-chlorophényl)pyridin-3-yl]oxy}méthyl)cyclopropyl]méthylamine

### Stade 1 : [1-({[5-(4-chlorophényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (4-chlorophényl)boronique.

### Stade 2 : Dichlorhydrate de [1-({[5-(4-chlorophényl)pyridin-3-yl]oxy}méthyl)-cyclopropyl]méthylamine

Le composé est obtenu selon le procédé du stade 2 de l'exemple 2 en utilisant le composé obtenu au stade 1 précédent.
Point de fusion (cap) : 208-213°C
Spectrométrie de masse (ESI) m/z = 289,1108 Th ([M+H]⁺)

### Exemple 8 :

### Dichlorhydrate de [1-({[6-chloro-5-(3-méthoxyphényl)pyridin-3-yl]oxy}méthyl)-cyclopropyl]méthylamine

### Stade 1 : [1-({[6-Chloro-5-(3-méthoxyphényl)pyridin-3-yl]oxy}méthyl)-cyclopropyl]méthylcarbamate de tert-butyle

Le composé est obtenu selon le procédé du stade 1 de l'exemple 1 en utilisant le composé de la préparation 2 à la place du composé de la préparation 1.

### Stade 2 : Dichlorhydrate de [1-({[6-chloro-5-(3-méthoxyphényl)pyridin-3-yl]oxy}méthyl)-cyclopropyl]méthylamine

Le composé est obtenu selon le procédé du stade 2 de l'exemple 2 en utilisant le composé obtenu au stade 1 précédent.
Spectrométrie de masse (ESI) m/z = 319,1219 Th ([M+H]⁺)

### Exemple 9 :

### Dichlorhydrate de (1-{[(6-chloro-5-phénylpyridin-3-yl)oxy]méthyl}cyclopropyl)-méthylamine

### Stade 1: (1-{[(6-Chloro-5-phénylpyridin-3-yl)oxy]méthyl}cyclopropyl)méthylcarbamate de tert-bytyle

Le composé est obtenu selon le procédé du stade 1 de l'exemple 1 en utilisant le composé de la préparation 2 à la place du composé de la préparation 1 et en remplaçant l'acide (3-méthoxyphényl)boronique par l'acide phénylboronique.

### Stade 2 : Dichlorhydrate de (1-{[(6-chloro-5-phénylpyridin-3-yl)oxy]méthy/}-cyclopropyl]méthylamine

Le composé est obtenu selon le procédé du stade 2 de l'exemple 2 en utilisant le composé obtenu au stade 1 précédent.
Point de fusion (cap) : 130-135°C
Spectrométrie de masse (ESI) m/z = 289,1094 Th ([M+H]⁺)

### Exemple 10 :

### Dichlorhydrate de [1-({[6-chloro-5-(4-méthylphényl)pyridin-3-yl]oxy}méthyl)-cyclopropyl]méthylamine

### Stade 1 : [1-({[6-Chloro-5-(4-méthylphényl)pyridin-3-yl]oxy}méthyl)cyclopropyl]méthylcarbamate de tert-butyle

Le composé est obtenu selon le procédé du stade 1 de l'exemple 1 en utilisant le composé de la préparation 2 à la place du composé de la préparation 1 et en remplaçant l'acide (3-méthoxyphényl)boronique par l'acide (4-méthylphényl)boronique.

### Stade 2 : Dichlorhydrate de [1-({[6-chloro-5-(4-méthylphényl)pyridin-3-yl]oxy}méthyl)-cyclopropyl]méthylamine

Le composé est obtenu selon le procédé du stade 2 de l'exemple 2 en utilisant le composé obtenu au stade 1 précédent.
Point de fusion (cap) : 150-155°C
Spectrométrie de masse (ESI) m/z = 303,1249 Th ([M+H]⁺)

### Exemple 11 :

### Dichlorhydrate de [1-({[5-(4-méthoxyphényl)pyridin-3-yl]oxy}méthyl)cyclopropyl]-méthylamine

### Stade 1 : [1-({[6-Chloro-5-(4-méthoxyphényl)pyridin-3-yl]oxy}méthyl)cyclopropyl]-méthylcarbamate de tert-butyle

Le composé est obtenu selon le procédé du stade 1 de l'exemple 1 en utilisant le composé de la préparation 2 à la place du composé de la préparation 1 et en remplaçant l'acide (3-méthoxyphényl)boronique par l'acide (4-méthoxyphényl)boronique.

### Stade 2 : Dichlorhydrate de [1-({[5-(4-méthoxyphényl)pyridin-3-yl]oxy}méthyl)-cyclopropyl]méthylamine

Le composé est obtenu selon le procédé du stade 2 de l'exemple 2 en utilisant le composé obtenu au stade 1 précédent.
Point de fusion (cap) : 180-185°C
Spectrométrie de masse (ESI) m/z = 319,1199 Th ([M+H]⁺)

### Exemple 12 :

### Chlorhydrate de [1-({[6-chloro-5-(4-nitrophényl)pyridin-3-yl]oxy}méthyl)-cyclopropyl]méthylamine

### Stade 1: [1-({[6-Chloro-5-(4-nitrophényl)pyridin-3-yl]oxy}méthyl)cyclopropyl]méthylcarbamate de tert-butyle

Le composé est obtenu selon le procédé du stade 1 de l'exemple 1 en mettant en réaction 1,1 g du composé de la préparation 2 et 0,7 g d'acide (4-nitrophényl)boronique. Une chromatographie sur silice (dichlorométhane/tétrahydrofurane : 97/3) permet d'obtenir 0,63 g du produit attendu.

### Stade 2 : Chlorhydrate de [1-({[6-chloro-5-(4-nitrophényl)pyridin-3-yl]oxy}-méthyl)cyclopropyl]méthylamine

Le composé est obtenu selon le procédé du stade 2 de l'exemple 2 en utilisant le composé obtenu au stade 1 précédent.
Point de fusion (cap) : 145-150°C
Spectrométrie de masse (ESI) m/z = 334,0945 Th ([M+H]⁺)

### Exemple 13 :

### Chlorhydrate de [1-({[5-(4-chlorophényl)pyridin-3-yl]oxy}méthyl)cyclopropyl]-méthylamine

### Stade 1: [1-({[6-Chloro-5-(4-chlorophényl)pyridin-3-yl]oxy}méthyl)cyclopropyl]méthyl-carbamate de tert-butyle

Le composé est obtenu selon le procédé du stade 1 de l'exemple 12 en remplaçant l'acide (4-nitrophényl)boronique par l'acide (4-chlorophényl)boronique.

### Stade 2 : Chlorhydrate de [1-({[5-(4-chlorophényl)pyridin-3-yl]oxy}méthyl)-cyclopropyl]méthylamine

Le composé est obtenu selon le procédé du stade 2 de l'exemple 2 en utilisant le composé obtenu au stade 1 précédent.
Point de fusion (cap) : 160-165°C
Spectrométrie de masse (ESI) m/z = 323,0699 Th ([M+H]⁺)

### Exemple 14 :

### Dichlorhydrate de [4-(2-chloro-5-{[1-(méthylamino)cyclopropyl]méthoxy}pyridin-3-yl)phényl]méthanol

### Stade 1 : {1-[({6-Chloro-5-[4-(hydroxyméthyl)phényl]pyridin-3-yl}oxy)méthyl]cyclopropyl}méthylcarbamate de tert-butyle

Le composé est obtenu selon le procédé du stade 1 de l'exemple 1 en utilisant le composé de la préparation 2 à la place du composé de la préparation 1 et en remplaçant l'acide (3-méthoxyphényl)boronique par l'acide (4-hydroxyméthylphényl)boronique.

### Stade 2 : Dichlorhydrate de [4-(2-chloro-5-{[1-(méthylamino)-cyclopropyl]méthoxy}pyridin-3-yl)phényl]méthanol

Le composé est obtenu selon le procédé du stade 2 de l'exemple 1 en utilisant le composé obtenu au stade 1 précédent.
Point de fusion (cap) : 142-146°C
Spectrométrie de masse (ESI) m/z = 319 Th ([M+H]⁺)

### Exemple 15 :

### Dichlorhydrate de [1-({[5-(4-chlorophényl)-6-méthylpyridin-3-yl]oxy}méthyl)-cyclopropyl]méthylamine

### Stade 1 : [1-({[5-(4-Chlorophényl)-6-méthylpyridin-3-yl]oxy}méthyl)cyclopropyl]méthylcarbamate de tert-butyle

Le composé est obtenu selon le procédé du stade 1 de l'exemple 1 en utilisant le composé de la préparation 3 à la place du composé de la préparation 1 et en remplaçant l'acide (3-méthoxyphényl)boronique par l'acide (4-chlorophényl)boronique.

### Stade 2 : Dichlorhydrate de [1-({[5-(4-chlorophényl)-6-méthylpyridin-3-yl]-oxy}méthyl)cyclopropyl]méthylamine

Le composé est obtenu selon le procédé du stade 2 de l'exemple 2 en utilisant le composé obtenu au stade 1 précédent.
Point de fusion (cap) : 194-199°C
Spectrométrie de masse (ESI) m/z = 303,1 Th ([M+H]⁺)

### Exemple 16 :

### Dichlorhydrate de [1-({[5-(3-chlorophényl)-6-méthylpyridin-3-yl]oxy}méthyl)cyclopropyl]méthylamine

### Stade 1: 1-({[5-(3-Chlorophényl)-6-méthylpyridin-3-yl]oxy}méthyl)cyclopropyl]méthylcarbamate de tert-butyle

Le composé est obtenu selon le procédé du stade 1 de l'exemple 1 en utilisant le composé de la préparation 3 à la place du composé de la préparation 1 et en remplaçant l'acide (3-méthoxyphényl)boronique par l'acide (3-chlorophényl)boronique.

### Stade 2 : Dichlorhydrate de [1-({[5-(3-chlorophényl)-6-méthylpyridin-3-yl]-oxy}méthyl)cyclopropyl]méthylamine

Le composé est obtenu selon le procédé du stade 2 de l'exemple 2 en utilisant le composé obtenu au stade 1 précédent.
Point de fusion (cap) : 223-227°C
Spectrométrie de masse (ESI) m/z = 303,1 Th ([M+H]⁺)

### Exemple 17 :

### Dichlorhydrate de 3-(2-méthyl-5-{[1-(méthylamino)cyclopropyl]méthoxy}pyridin-3-yl)benzonitrile

### Stade 1 : [1-({[5-(3-Cyanophényl)-6-méthylpyridin-3-yl]oxy}méthyl)cycloproyl]méthyl-carbamate de tert-butyle

Le composé est obtenu selon le procédé du stade 1 de l'exemple 1 en utilisant le composé de la préparation 3 à la place du composé de la préparation 1 et en remplaçant l'acide (3-méthoxyphényl)boronique par l'acide (3-cyanophényl)boronique.

### Stade 2 : Dichlorhydrate de 3-(2-méthyl-5-{[1-(méthylamino)cyclopropyl]-méthoxy}pyridin-3-yl)benzonitrile

Le composé est obtenu selon le procédé du stade 2 de l'exemple 2 en utilisant le composé obtenu au stade 1 précédent.
Point de fusion (cap) : 238-242°C
Spectrométrie de masse (ESI) m/z = 294,2 Th ([M+H]⁺)

### Exemple 18 :

### Chlorhydrate de 4-(2-chloro-5-{[1-(méthylamino)cyclopropyl]méthoxy}pyridin-3-yl)benzonitrile

### Stade 1 : 1-({[6-Chloro-5-(4-cyanophényl)pyridin-3-yl]oxy}méthyl)cyclopropyl]méthyl-carbamate de tert-butyle

Le composé est obtenu selon le procédé du stade 1 de l'exemple 12 en remplaçant l'acide (4-nitrophényl)boronique par l'acide (4-cyanophényl)boronique.

### Stade 2 : Chlorhydrate de 4-(2-chloro-5-{[1-(méthylamino)cyclopropyl]-méthoxy}gyridin-3-yl)benzonitrile

8 ml d'une solution d'acide chlorhydrique 4N dans le dioxanne sont ajoutés à une solution de 0.6 g du composé obtenu au stade 1 précédent dans 100 ml d'acétonitrile. Après 16 h sous agitation le milieu réactionnel est dilué à l'éther et le précipité filtré. Le précipité est dissous dans l'éthanol, la solution est concentrée puis le résidu obtenu est dissous de nouveau dans l'éthanol. Après dilution à l'éther et filtration on obtient 0,53 g du composé attendu
Point de fusion (cap) : 215-220°C
Spectrométrie de masse (ESI) m/z = 314 Th ([M+H]⁺)

### Exemple 19 :

### Dieblorhydrate de 4-(2-méthyl-5-{[1-(méthylamino)cyclopropyl]méthoxy}pyridin-3-yl)benzonitrile

### Stade 1: [1-({[5-(4-Cyanophényl)-6-méthylpyridin-3-yl]oxy}méthyl)cyclopropyl]méthylcarbamate de tert-butyle

Le composé est obtenu selon le procédé du stade 1 de l'exemple 1 en utilisant le composé de la préparation 3 à la place du composé de la préparation 1 et en remplaçant l'acide (3-méthoxyphényl)boronique par l'acide (4-cyanophényl)boronique.

### Stade 2 : Dichlorhydrate de 4-(2-méthyl-5-{[1-(méthylainino)cyclopropyl]-méthoxy}pyridin-3-yl)benzonitrile

Le composé est obtenu selon le procédé du stade 2 de l'exemple 18 en utilisant le composé obtenu au stade 1 précédent.
Point de fusion (cap) : 220-224°C
Spectrométrie de masse (ESI) m/z = 294,1622 Th ([M+H]⁺)

### Exemple 20 :

### Dichlorhydrate de 4-(5-{[1-(méthylamulo)cyclopropyl]méthoxy}pyridin-3-yl)-benzonitrile

### Stade 1 : [1-({[5-(4-Cyanophényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (4-cyanophényl)boronique.

### Stade 2 : Dichlorhydrate de 4-(5-{[1-(méthylamino)cyclopropyl]méthoxy}pyridin-3-yl)-benzonitrile

7,5 ml d'acide trifluoroacétique sont ajoutés à une solution de 0,85 g du composé obtenu au stade 1 précédent dans 7,5 ml de dichlorométhane. Après 20 heures sous agitation le milieu réactionnel est concentré à sec et repris par un mélange de dichlorométhane et d'une solution aqueuse saturée de carbonate de sodium. La phase organique est décantée, séchée sur sulfate de sodium puis concentré. Une chromatographie du résidu sur silice (toluène/éthanol : 93/7) permet d'isoler la base du composé désiré. Après dissolution de la base dans l'éther, ajout d'une solution d'acide chlorhydrique dans l'éther, filtration et séchage, on obtient 0,52 g du produit attendu.
Point de fusion (cap) : 150-160°C
Spectrométrie de masse (ESI) m/z = 280,1439 Th ([M+H]⁺)

### Exemple 21 :

### Dichlorhydrate de 3-(5-{[1-(méthylamino)cyclopropyl]méthoxy}pyridin-3-yl)-benzonitrile

### Stade 1 : [1-({[5-(3-Cyanophényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (3-cyanophényl)boronique.

### Stade 2 : Dichlorhydrate de 3-(5-{[1-(méthylamino)cyclopropyl]méthoxy}pyridin-3-yl)-benzoizitrile

Le composé est obtenu selon le procédé du stade 2 de l'exemple 20 en utilisant le composé obtenu au stade 1 précédent.
Point de fusion (cap) : 110-120°C
Spectrométrie de masse (ESI) m/z = 280,1435 Th ([M+H]⁺)

### Exemple 22 :

### Chlorhydrate de [1-({[5-(4-chlorophényl)-6-fluoropyridin-3-yl]oxy}méthyl) cyclopropyl]méthylamine

### Stade 1: [1-({[5-(4-Chlorophényl)-6-fluoropyridin-3-yl]oxy}méthyl)cyclopropyl]méthylcarbamate de tert-butyle

Le composé est obtenu selon le procédé du stade 1 de l'exemple 1 en utilisant le composé de la préparation 4 à la place du composé de la préparation 1 et en remplaçant l'acide (3-méthoxyphényl)boronique par l'acide (4-chlorophényl)boronique.

### Stade 2 : Chlorhydrate de [1-({[5-(4-chlorophényl)-6-fluoropyridin-3-yl]oxy}-méthyl)cyclo-propyl]méthylamine

2,5 ml d'acide trifluoroacétique sont ajoutés à une solution de 0,4 g du composé du stade 1 précédent dans 5 ml de dichlorométhane. Après 20 heures sous agitation le milieu réactionnel est concentré à sec et repris par un mélange de dichlorométhane et d'une solution aqueuse saturée de carbonate de sodium. La phase organique est décantée, séchée sur sulfate de sodium puis concentrée. La base obtenue est reprise dans l'éthanol, et le chlorhydrate est précipité par ajout d'une solution d'acide chlorhydrique dans l'éther puis dilution à l'éther. Après filtration et séchage on obtient 0,24 g de produit attendu.
Point de fusion (cap) : 55-60°C
Spectrométrie de masse (ESI) m/z = 307,1043 Th ([M+H]⁺)

### Exemple 23 :

### Chlorhydrate de [1-({[5-(3-chlorophényl)-6-fluoropyridin-3-yl]oxy}méthyl)-cyclopropyl]méthylamine

### Stade 1: [1-({[5-(3-Chlorophényl)-6-fluoropyridin-3-yl]oxy}méthyl)cyclopropyl]méthyl-carbamate de tert-butyle

Le composé est obtenu selon le procédé du stade 1 de l'exemple 1 en utilisant le composé de la préparation 4 à la place du composé de la préparation 1 et en remplaçant l'acide (3-méthoxyphényl)boronique par l'acide (3-chlorophényl)boronique.

### Stade 2 : Dichlorhydrate de [1-({[5-(3-chlorophényl)-6-fluoropyridin-3-yl]oxy}méthyl)-cyclopropy]lméthylamine

Le composé est obtenu selon le procédé du stade 2 de l'exemple 22 en utilisant le composé obtenu au stade 1 précédent.
Point de fusion (cap) : 126-130°C
Spectrométrie de masse (ESI) m/z = 307,1018 Th ([M+H]⁺)

### Exemple 24 :

### Chlorhydrate de 4-(2-fluoro-5-{[1-(méthylamino)cyclopropyl]méthoxy]pyridin-3-yl) benzonitrile

### Stade 1: [1-({[6-Fluoro-5-(4-cyanophényl)pyridin-3-yl]oxy}méthyl)cyclopropyl]méthylcarbamate de tert-butyle

Le composé est obtenu selon le procédé du stade 1 de l'exemple 1 en utilisant le composé de la préparation 4 à la place du composé de la préparation 1 et en remplaçant l'acide (3-méthoxyphényl)boronique par l'acide (4-cyanophényl)boronique.

### Stade 2 : Chlorhydrate de 4-(2-fluoro-5-{[1-(méthylamino)cyclopropyl]-méthoxy}pieridin-3-yl)benzonitrile

Le composé est obtenu selon le procédé du stade 2 de l'exemple 22 en utilisant le composé obtenu au stade 1 précédent.
Point de fusion (cap) : 90-105°C
Spectrométrie de masse (ESI) m/z = 298,1370 Th ([M+M⁺)

### Exemple 25 :

### Chlorhydrate de 3-(2-fluoro-5-{[1-(méthylamino)cyclopropyl]méthoxy}pyridin-3-yl) benzonitrile

### Stade 1 : [1-({[6-Fluoro-5-(3-cyanophényl)pyridin-3-yl]oxy}méthyl)cyclopropyl]méthylcarbamate de tert-butyle

Le composé est obtenu selon le procédé du stade 1 de l'exemple 1 en utilisant le composé de la préparation 4 à la place du composé de la préparation 1 et en remplaçant l'acide (3-méthoxyphényl)boronique par l'acide (3-cyanophényl)boronique.

### Stade 2 : Chlorhydrate de 3-(2-fluoro-5-{(1-(méthylamimo)cyclonropyl] méthoxy}pyridin-3-yl)benzonitrile

Le composé est obtenu selon le procédé du stade 2 de l'exemple 22 en utilisant le composé obtenu au stade 1 précédent.
Point de fusion (cap) : 211-215°C
Spectrométrie de masse (ESI) m/z = 298,1363 Th ([M+H]⁺)

### Exemple 26 :

### Chlorhydrate de [1-({[6-chloro-5-(4-fluorophényl)pyridin-3-yl]oxy}méthyl) cyclopropyl]méthylamine

0,16 g de tetrakis(triphenylphosphine)palladium (Pd(PPh₃)₄) sont ajoutés sous argon à une solution de 1.63 g du composé de la préparation 2 dans 30 ml de toluène. Le milieu est agité 45 minutes puis une solution de 0,59 g d'acide (4-fluorophényl)boronique dans 15 ml d'éthanol et 15 ml d'une solution aqueuse saturée d'hydrogénocarbonate de sodium sont ajoutées. Le milieu réactionnel est porté 4h30 à 85°C puis filtré et décanté. La phase organique est séchée sur sulfate de sodium et concentrée pour obtenir 1,95 g de produit de couplage brut. Ce produit brut est dissous dans 15 ml de dichlorométhane puis 3,5 ml d'acide trifluoroacétique sont ajoutés. Après 20 heures d'agitation la déprotection est complète et le milieu réactionnel est concentré. Le résidu obtenu est chromatographié sur colonne RP18 12-25µ (eau/acide trifluoroacétique : 1000/2,5 à eau/acétonitrile/acide trifluoroacétique : 750/250/2.5). Les fractions de chromatographie sont analysées et réunies, puis l'acétonitrile est évaporé. La solution aqueuse résiduelle est neutralisée puis saturée avec de l'hydrogénocarbonate de sodium solide puis extraite à l'acétate d'éthyle. Après séchage sur sulfate de sodium et concentration de la phase organique, la base obtenue est mise en solution dans l'éthanol et 1,5 ml d'une solution d'acide chlorhydrique 4N dans le dioxanne est ajoutée. Après concentration et cristallisation on obtient, après lavage à l'éther et séchage, 0,86 g de produit attendu
Point de fusion (cap) : 194-196°C
Spectrométrie de masse (ESI) m/z = 307,0998 Th ([M+H]⁺)

### Exemple 27 :

### Dichlorhydrate de [1-({[5-(3-aminophényl)-6-chloropyridin-3-yl]oxy}méthyl) cyclopropyl]méthylamine

Le composé est obtenu selon le procédé de l'exemple 26 en remplaçant l'acide (4-fluorophényl)boronique par l'acide (3-aminophényl)boronique.
Spectrométrie de masse (ESI) m/z = 304,1146 Th ([M+H]⁺)

### Exemple 28 :

### Chlorhydrate de [1-({[6-chloro-5-(3-nitrophényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (3-nitrophényl)boronique.
Point de fusion (cap) : 229-232°C
Spectrométrie de masse (ESI) m/z = 334,0923 Th ([M+H]⁺)

### Exemple 29 :

### Dichlorhydrate de {1-[({6-chloro-5-[4-(méthylthio)phényl]pyridin-3-yl}oxy)méthyl]-cyclopropyl}méthylamine

Le composé est obtenu selon le procédé de l'exemple 26 en remplaçant l'acide (4-fluorophényl)boronique par l'acide (4-méthylthiophényl)boronique.
Point de fusion (cap) : 144-148°C
Spectrométrie de masse (ESI) m/z = 335,0952 Th ([M+H]⁺)

### Exemple 30 :

### Dichlorhydrate de [1-({[6-chloro-5-(4-éthylphényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (4-éthylphényl)boronique.
Point de fusion (cap) : 112-114°C
Spectrométrie de masse (ESI) m/z = 317,1382 Th ([M+H]⁺)

### Exemple 31 :

### Chlorhydrate de [1-({[6-chloro-5-(2-méthylphényl)pyridin-3-yl]oxy}méthyl) cyclopropyl]méthylamine

Le composé est obtenu selon le procédé de l'exemple 26 en remplaçant l'acide (4-fluorophényl)boronique par l'acide (2-méthylphényl)boronique.
Point de fusion (cap) : 130-132°C
Spectrométrie de masse (ESI) m/z = 303,1326 Th ([M+H]⁺)

### Exemple 32 :

### Chlorhydrate de [1-({[6-chloro-5-(3-fluorophényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (3-fluorophényl)boronique.
Point de fusion (cap) : 172-175°C
Spectrométrie de masse (ESI) m/z = 307,0976 Th ([M+H]⁺)

### Exemple 33 :

### Chlorhydrate de [1-({[6-chloro-5-(3-méthylphényl)pyridin-3-yl]oxy}méthyl)cyclopropyl]méthylamine

Le composé est obtenu selon le procédé de l'exemple 26 en remplaçant l'acide (4-fluorophényl)boronique par l'acide (3-méthylphényl)boronique.
Point de fusion (cap) : 152-154°C
Spectrométrie de masse (ESI) m/z = 303,1239 Th ([M+H]⁺)

### Exemple 34 :

### Chlorhydrate de [1-({[6-chloro-5-(3-chlorophényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (3-chlorophényl)boronique.
Point de fusion (cap) : 182-184°C
Spectrométrie de masse (ESI) m/z = 323,0724 Th ([M+H]⁺)

### Exemple 35 :

### Chlorhydrate de 3-(2-chloro-5-{[1-(méthylamino)cyclopropyl]méthoxy}pyridin-3-yl)-benzonitrile

Le composé est obtenu selon le procédé de l'exemple 26 en remplaçant l'acide (4-fluorophényl)boronique par l'acide (3-cyanophényl)boronique.
Point de fusion (cap) : 228-232°C
Spectrométrie de masse (ESI) m/z = 314,1034 Th ([M+H]⁺)

### Exemple 36 :

### Chlorhydrate de [1-({[6-chloro-5-(2,3,4-triméthoxyphényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (2,3,4-triméthoxyphényl)boronique.
Point de fusion (cap) : 168-170°C
Spectrométrie de masse (ESI) m/z = 379,1433 Th ([M+H]⁺)

### Exemple 37 :

### Chlorhydrate de [1-({[6-chloro-5-(3,4,5-triméthoxyphényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (3,4,5-triméthoxyphényl)boronique.
Point de fusion (cap) : 138-140°C
Spectrométrie de masse (ESI) m/z = 379,1436 Th ([M+H]⁺)

### Exemple 38 :

### Chlorhydrate de {1-[({5-[3,5-bis(trifluorométhyl)phényl]-6-chloropyridin-3-yl}oxy)-méthyl]cyclopropyl}méthylamine

Le composé est obtenu selon le procédé de l'exemple 26 en remplaçant l'acide (4-fluorophényl)boronique par l'acide [3,5-bis(trifluorométhyl)phényl)]boronique.
Point de fusion (cas) : 182-188°C
Spectrométrie de masse (ESI) m/z = 425,0875 Th ([M+H]⁺)

### Exemple 39 :

### Chlorhydrate de [1-({[6-chloro-5-(2,5-difluorophényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (2,5-difluorophényl)boronique.
Point de fusion (cap) : 140-142°C
Spectrométrie de masse (ESI) m/z = 325,0887 Th ([M+H]⁺)

### Exemple 40 :

### Chlorhydrate de [1-({[6-chloro-5-(2,5-dichlorophényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (2,5-dichlorophényl)boronique.
Point de fusion (cap) : 140-142°C
Spectrométrie de masse (ESI) m/z = 357,0330 Th ([M+H]⁺)

### Exemple 41 :

### Chlorhydrate de [1-({[6-chloro-5-(3,5-dichlorophényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (3,5-dichlorophényl)boronique.
Point de fusion (cap) : 186-188°C
Spectrométrie de masse (ESI) m/z = 357,0308 Th ([M+H]⁺)

### Exemple 42 :

### Chlorhydrate de [1-({[6-chloro-5-(2,6-dichlorophényl)pyridin-3-yl]oxy}méthyl)-cyclopropyl]méthylamine

Le composé est obtenu selon le procédé de l'exemple 26 en remplaçant l'acide (4-fluorophényl)boronique par l'acide (2,6-dichlorophényl)boronique.
Point de fusion (cap) : 182-186°C
Spectrométrie de masse (ESI) m/z = 357,0322 Th ([M+H]⁺)

### Exemple 43 :

### Chlorhydrate de N-[3-(2-chloro-5-{[1-(méthylamino)cyclopropyl]méthoxy}pyridin-3-yl)phényl]acétamide

Le composé est obtenu selon le procédé de l'exemple 26 en remplaçant l'acide (4-fluorophényl)boronique par l'acide ({3-[(méthylamino)carbonyl]phényl})boronique.
Point de fusion (cap) : 220-222°C
Spectrométrie de masse (ESI) m/z = 346,1324 Th ([M+H]⁺)

### Exemple 44 :

### Dichlorhydrate de [1-({[5-(3-méthylphényl)pyridin-3-yl]oxy}méthyl)cyclopropyl]-méthylamine

### Stade 1 : [1-({[5-(3-Méthylphényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (3-méthylphényl)boronique.

### Stade 2 : Dichlorhydrate de [1-({[5-(3-méthylphényl)pyridin-3-yl]oxy}méthyl) cyclopropyl]méthylarnine

25 ml d'une solution d'acide chlorhydrique 1,5 N dans l'éthanol sont ajoutés à 0,9 g du composé obtenu au stade 1 précédent Le milieu est agité 20 h puis 10 ml d'une solution d'acide chlorhydrique 6 N dans l'éthanol sont ajoutés pour compléter la déprotection. Après 20 h d'agitation supplémentaire, le milieu réactionnel est amené à 100 ml avec de l'éther et le précipité est filtré et séché pour obtenir 0,64 g du produit attendu.
Point de fusion (cap) : 215-218°C
Spectrométrie de masse (ESI) m/z = 269,1655 Th ([M+H]⁺)

### Exemple 45 :

### Dichlorhydrate de [1-({[5-(3-nitrophényl)pyridin-3-yl]oxy}méthyl)-cyclopropyl]méthylamine

### Stade 1 : [1-({[5-(3-nitrophényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (3-nitrophényl)boronique.

### Stade 2 : Dichlorhydrate de [1-({[5-(3-nitrophényl)pyridin-3-yl]oxy}méthyl)cyclopropyl]-méthylamine

Le composé est obtenu selon le procédé du stade 2 de l'exemple 44 en utilisant le composé obtenu au stade 1 précédent.
Point de fusion (cap) : 220-225°C
Spectrométrie de masse (ESI) m/z = 300,1347 Th ([M+H]⁺)

### Exemple 46 :

### Dichlorhydrate de [1-({[5-(3-chlorophényl)pyridin-3-yl]oxy}méthyl)cyclopropyl]-méthylamine

### Stade 1 : [1-({[5-(3-Chlorophényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (3-chlorophényl)boronique.

### Stade 2 : Dichlorhydrate de [1-({[5-(3-chlorophényl)pyridin-3-yl]oxy}méthyl) cyclopropyl]méthylamine

Le composé est obtenu selon le procédé du stade 2 de l'exemple 44 en utilisant le composé obtenu au stade 1 précédent.
Point de fusion (cap) : 230-235°C
Spectrométrie de masse (ESI) m/z = 289,1074 Th ([M+H]⁺)

### Exemple 47 :

### Dichlorhydrate de [1-({[5-(3-fluorophényl)pyridin-3-yl]oxy}méthyl)cyclopropyl]-méthylamine

### Stade 1 : [1-({[5-(3-Fluorophény/)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (3-fluorophényl)boronique.

### Stade 2 : Dichlorhydrate de [1-({[5-(3-fluorophényl)pyridin-3-yl]oxy}méthyl) cyclopropyl]-méthylamine

Le composé est obtenu selon le procédé du stade 2 de l'exemple 44 en utilisant le composé obtenu au stade 1 précédent.
Point de fusion (cap) : 210-214°C
Spectrométrie de masse (ESI) m/z = 273,1387 Th ([M+H]⁺)

### Exemple 48 :

### Dichlorhydrate de méthyl{1-[({5-[4-(méthylthio)phényl]pyridin-3-yl}oxy)-méthyl]cyclopropyl}amine

### Stade 1 : Méthyl{1-[({5-[4-(méthylthio)phényl]pyridin-3-yl}oxy)méthyl]cyclopropyl}carbamate de tert-butyle

Le composé est obtenu selon le procédé du stade 1 de l'exemple 1 en remplaçant l'acide (3-méthoxyphényl)boronique par l'acide [4-(méthylthio)phényl]boronique.

### Stade 2 : Dichlorhydrate de méthyl{1-[({5-[4-(méthylthio)phényl]pyridin-3-yl}oxy)-méthyl]cyclopropyl}amine

Le composé est obtenu selon le procédé du stade 2 de l'exemple 44 en utilisant le composé obtenu au stade 1 précédent.
Point de fusion (cap) : 232-235°C
Spectrométrie de masse (ESI) m/z = 301,1367 Th ([M+H]⁺)

### Exemple 49 :

### Dichlorhydrate de [1-({[5-(4-éthylphényl)pyridin-3-yl]oxy}méthyl)cyclopropyl]-méthylamine

### Stade 1 : [1-({[5-(4-Ethylphényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (4-éthylphényl)boronique.

### Stade 2 : Dichlorhydrate de [1-({[5-(4-éthylphényl)pyridin-3-yl]oxy}méthyl)cyclopropyl]-méthylamine

Le composé est obtenu selon le procédé du stade 2 de l'exemple 44 en utilisant le composé obtenu au stade 1 précédent.
Point de fusion (cap) : 197-200°C
Spectrométrie de masse (ESI) m/z = 283,1796 Th ([M+H]⁺)

### Exemple 50 :

### Dichlorhydrate de méthyl[1-({[5-(2-méthylphényl)pyridin-3-yl]oxy}méthyl)-cyclopropyl]amine

### Stade 1 : Méthyl[1-({[5-(2-méthylphényl)pyridin-3-yl]oxy}méthyl)cyclopropyl]carbamate de tert-butyle

Le composé est obtenu selon le procédé du stade 1 de l'exemple 1 en remplaçant l'acide (3-méthoxyphényl)boronique par l'acide (2-méthylphényl)boronique.

### Stade 2 : Dichlorhydrate de méthyl[1-({[5-(2-méthylphényl)pyridin-3-yl]-oxy}méthyl)cyclopropy]amine

Le composé est obtenu selon le procédé du stade 2 de l'exemple 44 en utilisant le composé obtenu au stade 1 précédent.
Point de fusion (cap) : 192-196°C
Spectrométrie de masse (ESI) m/z = 269,1660 Th ([M+H]⁺)

### Exemple 51 :

### Dichlorhydrate de [1-({[5-(2,5-difluorophényl)pyridin-3-yl]oxy}méthyl)cyclopropyl]-méthylamine

### Stade 1 : [1-({[5-(2,5-Difluorophényl)pyridin-3-yl]oxy}méthyl)cyclopropyl]-méthylcarbamate de tert-butyle

Le composé est obtenu selon le procédé du stade 1 de l'exemple 1 en remplaçant l'acide (3-méthoxyphényl)boronique par l'acide (2,5-difluorophényl)boronique.

### Stade 2 : Dichlorhydrate de [1-({[5-(2,5-diflurorophényl)pyridin-3-yl]oxy}méthyl) cyclopropyl]méthylamine

Le composé est obtenu selon le procédé du stade 2 de l'exemple 44 en utilisant le composé obtenu au stade 1 précédent.
Point de fusion (cap) : 188-192°C
Spectrométrie de masse (ESI) m/z = 291 Th ([M+H]⁺)

### Exemple 52 :

### Dichlorhydrate de [1-({[5-(3,5-dichlorophényl)pyridin-3-yl]oxy}méthyl)cyclopropyl]-méthylamine

### Stade 1 : [1-({[5-(3.5-Dichlorophényl)pyridïn-3-yl]oxy}méthyl)cyclopropyl]-méthylcarbamate de tert-butyle

Le composé est obtenu selon le procédé du stade 1 de l'exemple 1 en remplaçant l'acide (3-méthoxyphényl)boronique par l'acide (3,5-dichlorophényl)boronique.

### Stade 2 : Dichlorhydrate de [1-({[5-(3,5-dichlorophényl)pyridin-3-yl]oxyl}méthyl)cyclopropyl]méthylamine

Le composé est obtenu selon le procédé du stade 2 de l'exemple 44 en utilisant le composé obtenu au stade 1 précédent.
Point de fusion (cap) : 215-220°C
Spectrométrie de masse (ESI) m/z = 323 Th ([M+H]⁺)

### Exemple 53 :

### Chlorhydrate de méthyl[1-({[5-(3,4,5-triméthoxyphényl)pyridin-3-yl]oxy}méthyl) cyclopropyl]amine

### Stade 1 : Méthyl[1-({[5-(3,4,5-trirnéthoxyphényl)pyridin-3-yl]oxy}méthyl)cyclopropyl]-carbamate de tert-butyle

Le composé est obtenu selon le procédé du stade 1 de l'exemple 1 en remplaçant l'acide (3-méthoxyphényl)boronique par l'acide (3,4,5-triméthoxyphényl)boronique.

### Stade 2 : Chlorhydrate de méthyl[1-({[5-(3,4,5-triméthoxyphényl)pyridin-3-yl]oxy}-méthyl)cyclopropyl]amine

Le composé est obtenu selon le procédé du stade 2 de l'exemple 44 en utilisant le composé obtenu au stade 1 précédent.
Point de fusion cap) : 116-120°C
Spectrométrie de masse (ESI) m/z = 345,1801 Th ([M+H]⁺)

### Exemple 54 :

### Dichlorhydrate de [1-({[5-(2,5-dichlorophényl)pyridin-3-yl]oxy}méthyl)cyclopropyl]-méthylamine

### Stade 1 : [1-({[5-(2,5-Dichlorophényl)pyridin-3-yl]oxy}méthyl)cyclopropyl]-méthylcarbamate de tert-butyle

Le composé est obtenu selon le procédé du stade 1 de l'exemple 1 en remplaçant l'acide (3-méthoxyphényl)boronique par l'acide (2,5-dichlorophényl)boronique.

### Stade 2 : Dichlorhydrate de [1-({[5-(2,5-dichlorophényl)pyridin-3-yl]oxy}méthyl)cyclopropyl]méthylamine

Le composé est obtenu selon le procédé du stade 2 de l'exemple 44 en utilisant le composé obtenu au stade 1 précédent.
Point de fusion (cap) : 195-200°C
Spectrométrie de masse (ESI) m/z = 323 Th ([M+H]⁺)

### Exemple 55 :

### Dichlorhydrate de [1({[5-(2,6-dichlorophényl)pyridin-3-yl]oxy}méthyl)cyclopropyl]-méthylamine

### Stade 1 : [1-({[5-(2,6-Dichlorophényl)pyridin-3-yl]oxy}méthyl)cyclopropyl]-méthylcarbamate de tert-butyle

Le composé est obtenu selon le procédé du stade 1 de l'exemple 1 en remplaçant l'acide (3-méthoxyphényl)boronique par l'acide (2,6-dichlorophényl)boronique.

### Stade 2 : Dichlorhydrate de [1-({[5-(2,6-dichlorophényl)pyridin-3-yl]oxy}méthyl)cyclopropyl]méthylamine

Le composé est obtenu selon le procédé du stade 2 de l'exemple 44 en utilisant le composé obtenu au stade 1 précédent.
Spectrométrie de masse (ESI) m/z = 323,0699 Th ([M+H]⁺)

### Exemple 56 :

### Chlorhydrate de {1-[({5-[3,5-bis(trifluorométhyl)phényl]pyridin-3-yl}oxy)méthyl] cyclopropyl}méthylamine

### Stade 1 : [1-[({5-[3,5-Bis(trifluorométhyl)phényl]pyridin-3-yl}oxy)méthyl]cyclopropyl}-méthylcarbamate de tert-butyle

Le composé est obtenu selon le procédé du stade 1 de l'exemple 1 en remplaçant l'acide (3-méthoxyphényl)boronique par l'acide [3,5-bis(trifluorométhyl)phényl]boronique.

### Stade 2 : Chlorhydrate de {1-[({5-[3,5-bis(trifluorométhyl)phényl]phényl]pyridin-3-yl}oxy) méthyl]cyclopropyl}méthylamine

Le composé est obtenu selon le procédé du stade 2 de l'exemple 44 en utilisant le composé obtenu au stade 1 précédent.
Point de fusion (cap) : 132-136°C
Spectrométrie de masse (ESI) m/z = 391,1249 Th ([M+H]⁺)

### Exemple 57 :

### Dichlorhydrate de méthyl[1-({[5-(2,3,4-triméthoxyphényl)pyridin-3-yl]oxy}méthyl)cyclopropyl]amine

### Stade 1: Méthyl[1-({[5-(2,3,4-triméthoxyphényl)pyridin-3-yl]oxy}méthyl)cyclopropyl]-carbamate de tert-butyle

Le composé est obtenu selon le procédé du stade 1 de l'exemple 1 en remplaçant l'acide (3-méthoxyphényl)boronique par l'acide (2,3,4-triméthoxyphényl)boronique.

### Stade 2 : Dichlorhydrate de méthyl[1-({[2,3,4-triméthoxyphényl)pyridin-3-yl]-oxy}méthylcyclopropyl]amine

Le composé est obtenu selon le procédé du stade 2 de l'exemple 44 en utilisant le composé obtenu au stade 1 précédent.
Spectrométrie de masse (ESI) m/z = 345,1783 Th ([M+H]⁺)

### Exemple 58 :

### Dichlorhydrate de N-[3-(5-{[1-(méthylamino)cyclopropyl]méthoxy}pyridin-3-yl)-phényl]-acétamide

### Stade 1 : {1-[({5-[3-(Acétylamino)phényl]pyridin-3-yl}oxy)méthyl]cyclopropyl}méthylcarbamate de tert-butyle

Le composé est obtenu selon le procédé du stade 1 de l'exemple 1 en remplaçant l'acide (3-méthoxyphényl)boronique par l'acide [3-(acétylamino)phényl]boronique.

### Stade 2 : Dichlorhydrate de N-[3-(5-{[1-(méthylamino)cyclopropyl]méthoxy}pyridin-3-yl)phényl]acétamide

1,05 g du produit obtenu au stade 1 précédent sont dissout dans 5 ml d'éthanol et 15 ml d'une solution d'acide chlorhydrique 5N dans l'éthanol sont ajoutés. Après 20 heures d'agitation le milieu réactionnel est concentré au trois-quarts et dilué à l'éther. Le précipité est repris par une solution saturée de carbonate de sodium et extrait à l'acétate d'éthyle. La phase organique est séchée sur sulfate de sodium puis évaporée. Le mélange obtenu est chromatographié sur 40 g de silice pour isoler 0,13 g de la base du produit attendu. La base est reprise dans l'éthanol, après ajout d'une solution d'acide chlorhydrique dans l'éther et filtration on reprend le solide collecté et on lyophilise pour obtenir 0,14 g de produit attendu.
Spectrométrie de masse (ESI) m/z = 312,1704 Th ([M+H]⁺)

### Exemple 59 :

### Trichlorhydrate de [1-({[5-(3-aminophényl)pyridin-3-yl]oxy}méthyl)cyclopropyl]-méthylamine

### Stade 1 : [1-({[5-(3-Aminophényl)pyridin-3-yl]oxy}méthyl)cyclopropyl]méthylcarbamate de tert-butyle

Le composé est obtenu selon le procédé du stade 1 de l'exemple 1 en remplaçant l'acide (3-méthoxyphényl)boronique par l'acide (3-aminophényl)boronique.

### Stade 2 : Trichlorhydrate de [1-({[5-(3-aminophényl)pyridin-3-yl]oxy}méthyl)-cyclopropyl]méthylamine

Le composé est obtenu selon le procédé du stade 2 de l'exemple 44 en utilisant le composé obtenu au stade 1 précédent.
Point de fusion (cap) : 185-190°C
Spectrométrie de masse (ESI) m/z = 270,1604 Th ([M+H]⁺)

### Exemple 60 :

### Dichlorhydrate de [1-({[5-(3-aminophényl)-6-fluoropyridin-3-yl]oxy}méthyl)-cyclopropyl]méthylamine

### Stade 1: [1-({[5-(3-Aminophényl)-6-fluoropyridin-3-yl]oxy}méthyl)cyclopropyl]methy/- carbamate de tert-butyle

Le composé est obtenu selon le procédé du stade 1 de l'exemple 1 en utilisant le composé de la préparation 4 à la place du composé de la préparation 1 et en remplaçant l'acide (3-méthoxyphényl)boronique par l'acide (3-aminophényl)boronique.

### Stade 2 : Dichlorhydrate de [1-({[5-(3-aminophényl)-6-fluoropyridin-3-yl]oxy}méthyl)cyclopropyl]méthylamine

75 ml de dioxanne puis 10 ml d'une solution d'acide chlorhydrique 4N dans le dioxanne sont ajoutés à 1,03 g du produit obtenu au stade 1 précédent. 50 ml d'éthanol sont ajoutés pour parvenir à une homogénéisation complète du milieu réactionnel. Après 20 h d'agitation les solvants sont évaporés, le résidu est repris par une solution de carbonate de sodium et extrait avec du dichlorométhane. Après séchage sur sulfate de sodium, la phase organique est concentrée et le résidu obtenu est chromatographié sur silice (dichlorométhane/méthanol : 97/3) pour obtenir 0,53 g de base du produit attendu. Après addition d'une solution d'acide chlorhydrique dans l'éther et filtration on obtient 0,5 g de produit attendu.
Point de fusion (cap) : 158-161°C
Spectrométrie de masse (ESI) m/z = 288,1488 Th ([M+H]⁺)

### Exemple 61 :

### Trichlorhydrate de [1-({[5-(3-aminophényl)-6-méthylpyridin-3-yl]oxy}méthyl)-cyclopropyl]méthylamine

### Stade 1 : [1-({[5-(3-Aminophényl)-6-méthylpyridin-3-yl]oxy}méthyl)cyclopropyl]méthylcarbamate de tert-butyle

Le composé est obtenu selon le procédé du stade 1 de l'exemple 1 en utilisant le composé de la préparation 3 à la place du composé de la préparation 1 et en remplaçant l'acide (3-méthoxyphényl)boronique par l'acide (3-aminophényl)boronique.

### Stade 2 : Trichlorhydrate de [1-({[5-(3-aminophényl)-6-méthylpyridin-3-yl]oxy}-méthyl)cyclopropyl]méthylamine

40 ml de dioxanne puis 10 ml d'une solution d'acide chlorhydrique 4N dans le dioxanne sont ajoutés à 1,05 g du produit obtenu au stade 1 précédent. 40 ml d'éthanol sont ajoutés pour parvenir à une homogénéisation complète du milieu réactionnel. Après 20 h d'agitation, les solvants sont évaporés, le résidu est repris par le minimun d'éthanol. Après dilution à l'éther et filtration, on obtient 0,71g de produit attendu.
Point de fusion (cap) : 222-228°C
Spectrométrie de masse (ESI) m/z = 284,1783 Th ([M+H]⁺)

### Exemple 62 :

### Dichlorhydrate de 4-(2-chloro-5-{[1-(méthylamino)cyclopropyl]méthoxy}-pyridin-3-yl)benzoate d'éthyle

### Stade 1 : 4-[5-({1-[(tert-Butoxycarbonyl)(méthyl)amino]cyclopropyl}méthoxy)-2-chloro-pyridin-3-yl]benzoate d'éthyle

Le composé est obtenu selon le procédé du stade 1 de l'exemple 1 en utilisant le composé de la préparation 2 à la place du composé de la préparation 1 et en remplaçant l'acide (3-méthoxyphényl)boronique par l'acide (4-éthoxycarbonylphényl)boronique.

### Stade 2 : Dichlorhydrate de 4-(2-chloro-5-{[1-(méthylamino)cyclopropyl]-méthoxy}pyridin-3-yl)benzoate d'éthyle

5 ml d'une solution d'acide chlorhydrique 4N dans le dioxanne sont ajoutés à une solution de 0,5 g du produit obtenu au stade 1 précédent dans 5 ml de dioxanne et 5 ml de d'éthanol. Après 20 heures d'agitation la déprotection est complète et le milieu réactionnel est concentré. Le résidu obtenu est chromatographié sur colonne RP18 12-25 µ (eau/acide trifluoroacétique : 1000/2,5 à eau/acétonitrile/acide trifluoroacétique : 450/550/2.5). Les fractions de chromatographie sont réunies, puis l'acétonitrile est évaporé. La solution aqueuse résiduelle est neutralisée puis saturée avec de l'hydrogénocarbonate de sodium solide puis extraite à l'acétate d'éthyle. Après séchage sur sulfate de sodium et concentration de la phase organique; la base obtenue est mise en solution dans l'éthanol et une solution d'acide chlorhydrique 4N dans le dioxanne est ajoutée. Après concentration et cristallisation on obtient, après lavage à l'éther et séchage, 0,25 g de produit attendu.
Point de fusion (cap) : 146-148°C
Spectrométrie de masse (ESI) m/z = 361,1357 Th ([M+H]⁺)

### Exemple 63 :

### Dichlorhydrate de 3-(2-chloro-5-{[1-(méthylamino)cyclopropyl]méthoxy}-pyridin-3-yl)benzoate d'éthyle

### Stade 1 : 3-[5-({1-[(tert-Butoxycarbonyl)(méthyl)amino]cyclopropyl}méthoxy)-2-chloropyridin-3-yl]benzoate d'éthyle

Le composé est obtenu selon le procédé du stade 1 de l'exemple 1 en utilisant le composé de la préparation 2 à la place du composé de la préparation 1 et en remplaçant l'acide (3-méthoxyphényl)boronique par l'acide (3-éthoxycarbonylphényl)boronique.

### Stade 2 : Dichlorhydrate de 3-(2-chloro-5-{[1-(méthylamino)cyclopropyl]méthoxy}-pyridin-3-yl)benzoate d'éthyle

Le composé est obtenu selon le procédé du stade 2 de l'exemple 62 en utilisant le composé obtenu au stade 1 précédent. Le produit final est repris dans l'eau puis lyophilisé. Spectrométrie de masse (ESI) m/z = 361,1328 Th ([M+H]⁺)

### Exemple 64 :

### Dichlorhydrate de [1-({[6-chloro-5-(4-méthoxyphényl)pyridin-3-yl]oxy}méthyl)cyclopropyl]amine

### Stade 1 : [1-({[6-Chloro-5-(4-méthoxyphényl)pyridin-3-yl]oxy}méthyl)cyclopropyl]-carbamate de tert-butyle

Le composé est obtenu selon le procédé du stade 1 de l'exemple 1 en utilisant le composé de la préparation 2 à la place du composé de la préparation 1 et en remplaçant l'acide (3-méthoxyphényl)boronique par l'acide (4-méthoxyphényl)boronique.
Point de fusion (cap) : 131°C

### Stade 2 : Dichlorhydrate de [1-({[6-chloro-5-(4-méthoxyphényl)pyridin-3-yl]oxy}méthyl)-cyclopropyl]amine

2 ml d'une solution d'acide chlorhydrique 4N dans le dioxanne sont ajoutés à une solution de 1,64 g de produit obtenu au stade 1 précédent dans 40 ml de méthanol. Après 20 h d'agitation les solvants sont évaporés et le résidu est trituré dans l'éther puis filtré. Le solide est repris par 20 ml d'une solution d'acide chlorhydrique 1,5 N dans le méthanol. On chauffe jusqu'à dissolution puis on laisse revenir à température ambiante le milieu réactionnel qui cristallise. Après filtration et séchage, on obtient 0,95 g de produit attendu
Point de fusion (cap) : 207-211 °C
Spectrométrie de masse (ESI) m/z = 305,1046 Th ([M+H]⁺)

### Exemple 65 :

### Chlorhydrate de [1-({[6-chloro-5-(4-méthoxyphényl)pyridin-3-yl]oxy}méthyl)-cyclopropyl] diméthylamine

Une solution de 1,28 g du composé du stade 1 de l'exemple 11 dans 12,5 ml d'acide formique est agitée 2 h à température ambiante. 12, 5 ml de solution à 40 % de formaldéhyde dans l'eau sont ajoutés puis le milieu réactionnel est chauffé 2 h à 70°C. 2,5 ml d'acide formique et 2,5 ml de la solution de formaldéhyde supplémentaires sont ajoutés au milieu réactionnel et le chauffage à 70°C est poursuivi 2 h. Le milieu réactionnel est concentré, puis 15 ml d'une solution aqueuse saturée de carbonate de potassium sont ajoutés et on extrait au dichlorométhane. Le dichlorométhane est séché sur sulfate de sodium et évaporé. Le résidu obtenu est chromatographié sur silice (dichlorométhane/méthanol : 97/3), pour isoler 0,63 g de base. 1,1 ml d'une solution d'acide chlorhydrique 4N dans le dioxanne sont ajoutés à une solution de la base précédemment isolée dans 5 ml d'éthanol. Le milieu réactionnel est dilué avec 100 ml d'éther et agité 30 mn. Après filtration et séchage on obtient 0,57 g de produit attendu.
Point de fusion (cap) : 208-210°C
Spectrométrie de masse (ESI) m/z = 333,1377 Th ([M+H]⁺)

### Exemple 66 :

### Chlorhydrate de [1-({[6-chloro-5-(chlorométhyl)pyridin-3-yl]oxy}méthyl) cyclopropyl]méthylamine

### Stade 1 : 1-({[6-Chloro-5-(chlorométhyl)pyridin-3-yl]oxy}méthyl)cyclopronyl]méthylcarbamate de tert-butyle

1,65 g de triphénylphosphine sont ajoutés à une solution de 2 g du composé obtenu au stade 1 de l'exemple 14 dans 10 ml de tétrachlorure de carbone. Le milieu est porté au reflux jusqu'à absence de matière première en chromatographie sur couche mince puis refroidi à température ambiante et filtré. Le filtrat est concentré et le résidu obtenu est chromatographié sur silice (dichlorométhane/tétrahydrofuranne : 97/3) pour obtenir 1,8 g de produit attendu.

### Stade 2 : Chlorhydrate de [1-({[6-chloro-5-(chlorométhyl)pyridin-3-yl]oxyl}méthyl)cyclopropyl]méthylamine

Le composé est obtenu selon le procédé du stade 2 de l'exemple 2 en utilisant le composé obtenu au stade 1 précédent.
Point de fusion (cap) : 136-140°C

### Exemple 67 :

### Chlorhydrate de 4-(2-chloro-5-{[1-(méthylamino)cyclopropyl]méthoxy}pyridine-3-yl) benzamide

1,2 g de carbonate de potassium sont ajoutés à une solution de 2,2 g du composé obtenu au stade 1 de l'exemple 18 dans 30 ml de diméthylsulfoxyde. Le milieu réactionnel est refroidi à à 0-5°C par un mélange d'eau et de glace puis 3,6 ml d'une solution aqueuse à 30% de peroxyde d'hydrogène sont additionnés goutte à goutte. Après 45 min d'agitation le milieu réactionnel est dilué à l'eau puis filtré. Le solide collecté est lavé à l'eau et repris dans l'acétate d'éthyle. La solution d'acétate d'éthyle est séchée sur sulfate de sodium puis concentrée. 2,1 g d'intermédiaire brut, [1-({[5-(aminocarbonyl)-6-chloropyridin-3-yl]oxy}méthyl)cyclopropyl]méthylcarbamate) de tert-butyle, sont repris dans 10 ml d'éthanol puis 15 ml d'une solution d'acide chlorhydrique 4N dans le dioxanne sont ajoutés. Après 20 h d'agitation, le milieu réactionnel est dilué à l'éther puis filtré. Le solide collecté est chromatographié sur colonne RP18, 12-25µ, (eau/acide trifluoroacétique : 1000/2,5 à eau/acétonitrile/acide trifluoroacétique : 675/325/2,5). Les fractions de chromatographie sont réunies, puis l'acétonitrile est évaporé. La solution aqueuse résiduelle est neutralisée puis saturée avec de l'hydrogénocarbonate de sodium solide puis extraite au dichlorométhane. Après séchage sur sulfate de sodium et concentration de la phase organique, la base obtenue est mise en solution dans 25 ml d'éthanol et 1 ml d'une solution d'acide chlorhydrique 4N dans le dioxanne est ajoutée. Après concentration et cristallisation dans l'éther, on filtre et on sèche pour obtenir 1,1 g de produit attendu.
Point de fusion (cap) : 130°C
Spectrométrie de masse (ESI) m/z = 332,1184 Th ([M+H]⁺)

### Exemple 68 :

### Dichlorhydrate de l'acide 3-(5-{[1-(méthylamino)cyclopropyl]méthoxy}pyridin-3-yl)-benzoique

### Stade 1 : Acide 3-[5-({1-[(tert-butoxycarbonyl)(méthyl)amino]cyclopropyl}méthoxy)pyridin-3-yl]benzoique

25 ml d'une solution aqueuse 0,4 M de carbonate de sodium et 0,58 g d'acide (3-carboxyphényl)boronique sont ajoutés à une solution de 1g du composé de la préparation 1 dans 25 ml d'acétonitrile. Après 45 mn d'agitation sous argon, 0,15 g de tetrakis(triphenylphosphine)palladium sont additionnés puis le mélange réactionnel est chauffé 5h30 à 80°C. Le milieu réactionnel est filtré à chaud et le pH du filtrat refroidi est ajusté à 5,5 sous pHmètre, par addition d'une solution aqueuse 1N d'acide chlorhydrique. Le milieu réactionnel est extrait à l'acétate d'éthyle. La phase organique est séchée sur sulfate de sodium puis concentrée. Une chromatographie sur silice (dichlorométhane/méthanol : 97/3) permet d'isoler 0,9 g du produit attendu.

### Stade 2 : Dichlorhydrate de l'acide 3-(5-{[1-(méthylamino)cyclopropyl]méthoxy}pyridin-3-yl)benzoique

5 ml d'une solution d'acide chlorhydrique 4N dans le dioxanne sont ajoutés à une solution de 0,9 g du composé obtenu au stade 1 précédent dans 10 ml de dioxanne. Le milieu est agité 20 h, dilué à l'éther puis filtré. Le solide collecté est repris dans 25 ml d'eau pour obtenir après lyophilisation 0,595 g de produit attendu.
Spectrométrie de masse (ESI) m/z = 299,1374 Th ([M+H]⁺)

### Exemple 69 :

### Dichlorhydrate de l'acide 4-(5-{[1-(méthylamino)cyclopropyl]méthoxy}pyridin-4-yl)-benzoique

### Stade 1 : Acide 4-[5-({1-[(tert-butoxycarbonyl)(méthyl)amino]cyclopropyl}-méthoxy)pyridin-3-yl]benzoique

Le composé est obtenu selon le procédé du stade 1 de l'exemple 68 en remplaçant l'acide (3-carboxyphényl)boronique par l'acide (4-carboxyphényl)boronique.

### Stade 2 : Dichlorhydrate de l'acide 4-(5-{[1-(méthylamino)cyclopropyl]méthoxy}pyridin-4-yl)benzoique

Le composé est obtenu selon le procédé du stade 2 de l'exemple 68 en utilisant le composé obtenu au stade 1 précédent.
Spectrométrie de masse (ESI) m/z = 299,1372 Th ([M+H]⁺)

### Exemple 70 :

### Chlorhydrate de l'acide 3-(2-chloro-5-{[1-(méthylamino)cyclopropyl]méthoxy} pyridin-3-yl)benzoique

### Stade 1 : Acide 3-[5-({1-[(tert-butoxycarbonyl)(méthyl)amino]cyclopropyl}méthoxy)-2-chloropyridin-3-yl]benzoique

Le composé est obtenu selon le procédé du stade 1 de l'exemple 68 en utilisant le composé de la préparation 2 à la place du composé de la préparation 1.

### Stade 2 : Chlorhydrate de l'acide 3-(2-chloro-5-{[1-(méthylamino)cyclopropyl]méthoxy}-pyridin-3-yl)benzoipue

Le composé est obtenu selon le procédé du stade 2 de l'exemple 68 en utilisant le composé obtenu au stade 1 précédent.
Spectrométrie de masse (ESI) m/z = 333,1004 Th ([M+H]⁺)

### Exemple 71 :

### Chlorhydrate de l'acide 4-(2-chloro-5-{[1-(méthylamino)cyclopropyl]méthoxy} pyridin-3-yl)benzoique

### Stade 1 : Acide 4-[5-({1-[(tert-butoxycarbonyl)(méthyl)amino]cycloproyl}méthoxy)-2-chloropyridin-3-yl]benzoique

Le composé est obtenu selon le procédé du stade 1 de l'exemple 68 en utilisant le composé de la préparation 2 à la place du composé de la préparation 1 et en remplaçant l'acide (3-carboxyphényl)boronique par l'acide (4-carboxyphényl)boronique.

### Stade 2 : Chlorhydrate de l'acide 4-(2-chloro-5{[1-(méthylamino)cyclopropyl]méthoxy}-pyridin-3-yl)benzoique

Le composé est obtenu selon le procédé du stade 2 de l'exemple 68 en utilisant le composé obtenu au stade 1 précédent.
Spectrométrie de masse (ESI) m/z = 333,1003 Th ([M+H]⁺)

### Exemple 72 :

### Dichlorhydrate de l'acide 3-(2-fluoro-5-{[1-(méthylamino)cyclopropyl]méthoxy}-pyridin-3-yl)benzoique

### Stade 1 : Acide 3-[5-({1-[(tert-butoxycarbonyl)(méthyl)amino]cyclopronyl}méthoxy)-2-fluoropyridin-3-yl]benzoique

Le composé est obtenu selon le procédé du stade 1 de l'exemple 68 en utilisant le composé de la préparation 4 à la place du composé de la préparation 1.

### Stade 2 : Dichlorhydrate de l'acide 3-(2-fluoro-5-{[1-(méthylamino)cyclopropyl ]méthoxy}pyridin-3-yl)benzoique

Le composé est obtenu selon le procédé du stade 2 de l'exemple 68 en utilisant le composé obtenu au stade 1 précédent.
Spectrométrie de masse (ESI) m/z = 317,1315 Th ([M+H]⁺)

### Exemple 73 :

### Dichlorhydrate de l'acide 4-(2-fluoro-5-{[1-(méthylamino)cyclopropyl]méthoxy}-pyridin-3-yl)benzoique

### Stade 1 : Acide 4-[5-({1-[(tert-butoxycarbonyl)(méthyl)amino]cyclopropyl}méthoxy)-2-fluoropyridin-3-yl]benzoique

Le composé est obtenu selon le procédé du stade 1 de l'exemple 68 en utilisant le composé de la préparation 4 à la place du composé de la préparation 1 et en remplaçant l'acide (3-carboxyphényl)boronique par l'acide (4-carboxyphényl)boronique.

### Stade 2 : Dichlorhydrate de l'acide 4-(2-fluoro-5-{[1-(méthylamino)cyclopropyl] méthoxy}pyridin-3-yl)benzoique

Le composé est obtenu selon le procédé du stade 2 de l'exemple 1 en utilisant le composé obtenu au stade 1 précédent.
Point de fusion (cap) : 211-215°C
Spectrométrie de masse (ESI) m/z = 317,1324 Th ([M+H]⁺)

### Exemple 74 :

### Dichlorhydrate de l'acide 3-(2-méthyl-5-{[1-(méthylamino)cyclopropyl]méthoxy}-pyridin-3-yl)benzoique

### Stade 1 : Acide 3-[5-({1-[(tert-butoxycarbony/)(méthyl)amino]cyc/opropyl}méthoxy)-2-méthylpyridin-3-yl]benzoique

Le composé est obtenu selon le procédé du stade 1 de l'exemple 68 en utilisant le composé de la préparation 3 à la place du composé de la préparation 1.

### Stade 2 : Dichlorhydrate de l'acide 3-(2-méthy/-5-{[1-(méthylamino)cyclopropyl] méthoxy}pyridin-3-yl)benzoique

Le composé est obtenu selon le procédé du stade 2 de l'exemple 1 en utilisant le composé obtenu au stade 1 précédent.
Point de fusion (cap) : 210-215°C
Spectrométrie de masse (ESI) m/z = 313,1515Th ([M+H]⁺)

### Exemple 75:

### Dichlorhydrate de l'acide 4-(2-méthyl-5-{[1-(méthylamino)cyclopropyl]méthoxy} pyridin-3-yl)benzoique

### Stade 1 : Acide 4-[5-({1-[(tert-butoxycarbonyl)(méthyl)amino]cyclopropyl}méthoxy)-2-méthylpyridin-3-yl]benzoique

Le composé est obtenu selon le procédé du stade 1 de l'exemple 68 en utilisant le composé de la préparation 3 à la place du composé de la préparation 1 et en remplaçant l'acide (3-carboxyphényl)boronique par l'acide (4-carboxyphényl)boronique.

### Stade 2 : Dichlorhydrate de l'acide 4-(2-méthyl-5-{[1-(méthylamino)cyclopropyl] méthoxy}pyridin-3-yl)benzoique

Le composé est obtenu selon le procédé du stade 2 de l'exemple 1 en utilisant le composé obtenu au stade 1 précédent.
Point de fusion (cap) : 228-235°C
Spectrométrie de masse (ESI) m/z = 313,1580 Th ([M+H]⁺)

### Exemple 76 :

### Dichlorhydrate de (1-{[(2-chloro-3,3'-bipyridin-5-yl)oxy]méthyl}cyclopropyl)-méthylamine

### Stade 1 : (1-{[(2-Chloro-3,3'-bipyridin-5-yl)oxy]méthyl}cyclopropyl)méthylcarbamate de tert-butyle

0,87 g de tetrakis(triphenylphosphine)palladium sont ajoutés sous azote à une solution de 2,91 g du composé de la préparation 2 dans 45 ml de toluène. Le milieu est agité 20 minutes puis une solution de 3 g de 3-(1,1,1-tributylstannyl)pyridine dans 6 ml de toluène est additionnée et le milieu réactionnel est porté 20 h au reflux. Une seconde fraction de 0,87g de Pd(PPh₃)₄ est ajoutée et le reflux est poursuivi 24 h. Après refroidissement le milieu réactionnel est dilué avec 120 ml de toluène puis lavé avec une solution aqueuse à 50% de carbonate de potassium. La phase organique est séchée sur sulfate de sodium et concentrée. Une chromatographie sur silice (dichlorométhane/butanone : 97/3 à dichlorométhane/butanone : 80/20) permet d'obtenir 1,76 g de produit attendu.

### Stade 2 : Dichlorhydrate de (1-{[(2-chloro-3,3'-bipyridin-5-yl)oxy]méthyl}cyclopropyl)-méthylamine

24,6 ml d'une solution d'acide chlorhydrique 4N dans le dioxanne sont ajoutés à une solution de 2,46 g du composé obtenu au stade 1 précédent dans 24,6 ml de méthanol. Après 20 h d'agitation, les solvants sont évaporés et le résidu repris par une solution aqueuse à 50% de carbonate de potassium et extrait au dichlorométhane. La phase organique est séchée sur sulfate de sodium et concentrée. Le résidu est chromatographié sur silice (dichlorométhane/méthanol : 97/3) pour isolé 1,05 g de base. La base est reprise dans 120 ml d'éthanol et une solution d'acide chlorhydrique dans l'éthanol est ajoutée jusqu'à pH acide. Le milieu réactionnel est concentré puis trituré dans l'éther pour obtenir après cristallisation, filtration et séchage 1,15 g de produit attendu.
Point de fusion (cap) : 210-215°C
Spectrométrie de masse (ESI) m/z = 290,1037 Th ([M+H]⁺)

### Exemple 77 :

### Dichlorhydrate de (1-{[(2-chloro-3,4'-bipyridin-5-yl)oxy]méthyl}cyclopropyl)-méthylamine

### Stade 1 : (1-{[(2-Chloro-3,4'-bipyridin-5-yl)oxy]méthyl}cyclopropyl)methylcarbamate de tert-butyle

Le composé est obtenu selon le procédé du stade 1 de l'exemple 76 en remplaçant la 3-(1,1,1-tributylstannyl)pyridine par la 4-(1,1,1-tributylstannyl)pyridine.

### Stade 2 : Dichlorhydrate de (1-{[(2-chloro-3,4'-bipyridin-5-yl)oxy]méthyl}cyclopropyl)méthylamino

Le composé est obtenu selon le procédé du stade 2 de l'exemple 76 en utilisant le composé obtenu au stade 1 précédent.
Point de fusion (cap) : 125-130°C
Spectrométrie de masse (ESI) m/z = 290,1035 Th ([M+H]⁺)

### Exemple 78 :

### Dichlorhydrate de (1-{[(2-fluoro-3,3'-bipyridin-5-yl)oxy]méthyl}cyclopropyl)-méthylamine

### Stade 1 : (1-{[(2-Fluoro-3,3'-bipyridin-5-yl)oxy]méthyl}cyclopropyl)méthylcarbamate de tert-butyle

0,76 g de tetrakis(triphenylphosphine)palladium (sont ajoutés sous azote à une solution de 2,5g du composé de la préparation 4 dans 40 ml de toluène. Le milieu est agité 20 minutes puis une solution de 2,6, g de 3-(1,1,1-tributylstannyl)pyridine dans 5 ml de toluène sont rajoutés et le milieu réactionnel est porté 20 h au reflux. Après refroidissement le milieu réactionnel est dilué avec du toluène puis lavé avec une solution aqueuse à 50% de carbonate de potassium. La phase organique est séchée sur sulfate de sodium et concentrée. Une chromatographie sur silice (dichlorométhane/butanone : 97/3 à dichlorométhane/butanone : 90/10) permet d'obtenir 1,66 g de produit attendu.

### Stade 2 : Dichlorhydrate de (1-{[(2-fluoro-3,3'-bipyridin-5-yl)oxy]méthyl}cyclopropyl)-méthylamine

16 ml d'une solution d'acide chlorhydrique 4N dans le dioxanne sont ajoutés à une solution de 1,6 g du composé obtenu au stade 1 précédent dans 100 ml d'éthanol Après 20 h d'agitation, les solvants sont évaporés, le résidu est dissout dans un minimum d'éthanol et de l'éther est ajouté. On agite 20 h , les solvants sont décantés et de l'éther est à nouveau ajouté au résidu solide pour obtenir après cristallisation, filtration et séchage 0,7 g de composé attendu.
Spectrométrie de masse (ESI) m/z = 274,1365 Th ([M+H]⁺)

### Exemple 79 :

### Dichlorhydrate de (1-{[(2-fluoro-3,4'-bipyridin-5-yl)oxy]méthyl}cyclopropyl)-méthylamine

### Stade 1: (1-{[(2-Fluoro-3,4'-bipyridin-5-yl)oxy]methyl}cyclopropyl)methylcarbamate de tert-butyle

Le composé est obtenu selon le procédé du stade 1 de l'exemple 78 en remplaçant la 3-(1,1,1-tributylstannyl)pyridine par la 4-(1,1,1-tributylstannyl)pyridine.

### Stade 2 : Dichlorhydrate de (1-{[(2-fluoro-3,4'-bipyridin-5-yl)oxy]méthyl}cyclopropyl)-méthylamine

24 ml d'une solution d'acide chlorhydrique 4N dans le dioxanne sont ajoutés à une solution de 2,4 g du composé obtenu au stade 1 précédent dans 120 ml d'éthanol Après 20 h d'agitation, les solvants sont évaporés et le résidu repris par une solution aqueuse à 50% de carbonate de potassium et extrait au dichlorométhane. La phase organique est séchée sur sulfate de sodium et concentrée. Le résidu est chromatographié sur silice (dichlorométhane /méthanol : 97/3). La base est dissoute dans un minimum d'éthanol et une solution d'acide chlorhydrique dans l'éther est ajoutée jusqu'à pH acide. Le milieu réactionnel est concentré puis trituré dans l'éther pour obtenir après cristallisation, filtration et séchage 1 g de produit attendu.
Spectrométrie de masse (ESI) m/z = 274,1344 Th ([M+H]⁺)

### Exemple 80 :

### Trichlorhydrate de méthyl(1-{[(2-méthyl-3,3'-bipyridin-5-yl)oxy]méthyl}-cyclopropyl)amine

### Stade 1 : Méthyl(1-{[(2-méthyl-3,3'-bipyridin-5-yl)oxy]méthyl}cyclopropyl)carbamate de tert-butyle

Le composé est obtenu selon le procédé du stade 1 de l'exemple 78 en utilisant le composé de la préparation 3 à la place du composé de la préparation 4.

### Stade 2 : Trichlorhydrate de méthyl(1-{[(2-méthyl-3,3'-bipyridin-5-yl)oxy]méthyl}cyclopropylamine

Le composé est obtenu selon le procédé du stade 2 de l'exemple 78 en utilisant le composé obtenu au stade 1 précédent.
Spectrométrie de masse (ESI) m/z = 270,1619 Th ([M+H]⁺)

### Exemple 81 :

### Trichlorhydrate de méthyl(1-{[(2-méthyl-3,4'-bipyridin-5-yl)oxy]méthyl}cyclopropyl)amine

### Stade 1: Méthyl(1-{[(2-méthyl-3,4'-bipyridin-5-yl)oxy]méthyl}cyclopropyl)carbamate de tert-butyle

Le composé est obtenu selon le procédé du stade 1 de l'exemple 78 en utilisant le composé de la préparation 3 à la place du composé de la préparation 4 et en remplaçant la 3-(1,1,1-tributylstannyl)pyridine par la 4-(1,1,1-tributylstannyl)pyridine.

### Stade 2 : Trichlorhydrate de méthyl(1-{[(2-méthyl-3,4'-bipyridin-5-yl)oxy]méthyl}cyclopropyl)amine

Le composé est obtenu selon le procédé du stade 2 de l'exemple 78 en utilisant le composé obtenu au stade 1 précédent.
Point de fusion (cap) : 130-138°C
Spectrométrie de masse (ESI) m/z = 270,1609 Th ([M+H]⁺)

### Exemple 82 :

### Dichlorhydrate de [1-({[5-(4-aminophényl)-6-chloropyridin-3-yl]oxy}méthyl)-cyclopropyl]méthylamine

### Stade 1 : [1-({[5-(4-Amlnophényl)-6-chloropyridin-3-yl]oxy}méthyl)cyclopropyl]méthylcarbamate de tert-butyle

0,67 g de tetrakis(triphenylphosphine)palladium sont ajoutés sous azote à une solution de 1 g du composé de la préparation 2 et de 4-(4,4,5,5-tétraméthyl-1,3,2-dioxaborolan-2-yl)aniline dans 30 ml de tétrahydrofuranne. Le milieu est agité 20 minutes puis une solution de 1,65 g de carbonate de sodium dans 10 ml d'eau est ajoutée et le milieu réactionnel est chauffé 20 h à 60°C. Les solvants sont évaporés puis le résidu est repris par une solution de carbonate de sodium et extrait au dichlorométhane. La phase organique est séchée sur sulfate de sodium et concentrée. Une chromatographie sur silice (dichlorométhane/acétate d'éthyle : 98/2 à 90/10) permet d'obtenir 0,87 g du produit attendu.

### Stade 2 : Dichlorhydrate de [1-({[5-(4-aminophényl)-6-chloropyridin-3-yl]oxy} méthyl)cyclopropyl]méthylamine

Le composé est obtenu selon le procédé du stade 2 de l'exemple 2 en utilisant le composé obtenu au stade 1 précédent.
Point de fusion (cap) : 240-243°C
Spectrométrie de masse (ESI) m/z = 304 Th ([M+H]⁺)

### Exemple 83 :

### Dichlorhydrate de [1-({[5-(4-aminophényl)-6-fluoropyridin-3-yl]oxy}méthyl) cyclopropyl]méthylamine

### Stade 1 : [1-({[5-(4-Aminophényl)-6-fluoropyridin-3-yl]oxy}méthyl)cyclopropyl]méthylcarbamate de tert-butyle

Le composé est obtenu selon le procédé du stade 1 de l'exemple 82 en utilisant le composé de la préparation 4 à la place du composé de la préparation 2.

### Stade 2 : Dichlorhydrate de [1-({[5-(4-aminophényl)-6-fluoropyridin-3-yl]oxy}méthyl)-cyclopropyl]méthylamine

Le composé est obtenu selon le procédé du stade 2 de l'exemple 22 en utilisant le composé obtenu au stade 1 précédent.
Point de fusion (cap) : 225-230°C
Spectrométrie de masse (ESI) m/z = 288,1521 Th ([M+H]⁺)

### Exemple 84 :

### Dichlorhydrate de 4-{5-[(1-aminocyclopropyl)méthoxy]-2-chloropyridin-3-yl}phénol

### Stade 1 : [1-({[6-chloro-5-(4-hydroxyphenyl)pyridin-3-yl]oxy}methyl)cyclopropyl] carbamate de tert-butyle

Le composé est obtenu selon le procédé du stade 1 de l'exemple 82 en utilisant le composé de la préparation 5 à la place du composé de la préparation 2 et en remplaçant le 4-(4,4,5,5-tétraméthyl-1,3,2-dioxaborolan-2-yl)aniline par le 4-(4,4,5,5-tétraméthyl-1,3,2-dioxaborolan-2-yl)phénol.

### Stade 2 : Dichlorhydrate de 4-{5-[(1-aminocyclopropyll)méthoxy]-2-chloropyridin-3-yl}-phénol

Le composé est obtenu selon le procédé du stade 2 de l'exemple 1 en utilisant le composé obtenu au stade 1 précédent.
Point de fusion (cap) : décomposition > 200°C
Spectrométrie de masse (ESI) m/z = 291,0894 Th ([M+H]⁺)

### Exemple 85 :

### Dichlorhydrate de 4-(2-chloro-5-{[1-(méthylamino)cyclopropyl]méthoxy}pyridin-3-yl)phénol

### Stade 1 : 1-({[6-Chloro-5-(4-hydroxyphényl)pyridin-3-yl]oxy}méthyl)cyclopropyl]-méthylcarbamate de tert-butyle

Le composé est obtenu selon le procédé du stade 1 de l'exemple 82 en remplaçant la 4-(4,4,5,5-tétraméthyl-1,3,2-dioxaborolan-2-yl)aniline par le 4-(4,4,5,5-tétraméthyl-1,3,2-dioxaborolan-2-yl)phénol.

### Stade 2 : Dichlorhydrate de 4-(2-chloro-5-{[1-(méthylamino)cyclopropyl]méthoxy}-pyridin-3-yl)phénol

Le composé est obtenu selon le procédé du stade 2 de l'exemple 1 en utilisant le composé obtenu au stade 1 précédent.
Point de fusion (cap) : 216-220°C
Spectrométrie de masse (ESI) m/z = 305,1062 Th ([M+H]⁺)

### Exemple 86 :

### Chlorhydrate de 2-chloro-N-[4-(2-chloro-5-{[1-(méthylamino)cyclopropyl]méthoxy}-pyridin-3-yl)phényl]acétamide

### Stade 1 : (1-{[(6-Chloro-5-{4-[(chloroacétyl)amino]phényl}pyridin-3-yl)oxy]méthyl}-cyclopropyl)méthylcarbamate de tert-butyle

0,44 g de chlorure de chloracétyle sont additionnés à 10 °C à une solution de 1,5 g du composé obtenu au stade 1 de l'exemple 82 et de 0,54 ml de triéthylamine dans 20 ml de tétrahydrofuranne. Le milieu réactionnel est agité 20 h à température ambiante puis concentré. Le résidu est repris dans un mélange éther et eau puis concentrée. La phase organique est décantée, puis séchée sur sulfate de sodium et concentrée pour obtenir 1,7 g de produit attendu.

### Stade 2 : Chlorhydrate de 2-chloro-N-[4-(2-chloro-5-{[1-(méthylamino)cyclopropyl]-méthoxy}pyridin-3-yl)phényl]acétamide

Le composé est obtenu selon le procédé du stade 2 de l'exemple 1 en utilisant le composé obtenu au stade 1 précédent.
Point de fusion (cap) : 140-144°C
Spectrométrie de masse (ESI) m/z = 380,0928 Th ([M+H]⁺)

### Exemple 87 :

### Chlorhydrate de [1-({[6-chloro-5-(4-isothiocyanatophényl)pyridin-3-yl]oxy}méthyl)-cyclopropyl]méthylamine

### Stade 1 : [1-({[6-Chloro-5-(4-isothiocyanatophényl)pyridin-3-yl]oxy}méthyl)-cyclopropyl]méthylcarbamate de tert-butyle

Une solution de 1,05 g d'hydrogénocarbonate de sodium dans 20 ml d'eau est ajoutée à une solution de 1,01 g du composé obtenu au stade 1 de l'exemple 82 dans 20 ml de tétrahydrofuranne. 1,38 g de thiophosgène sont ensuite additionnés goutte à goutte puis le mélange réactionnel orange est agité une heure à température ambiante. 30 ml d'une solution aqueuse saturée d'hydrogénocarbonate de sodium sont ajoutés et le milieu réactionnel est extrait au dichlorométhane. La phase organique est séchée sur sulfate de sodium et concentrée. Une chromatographie sur silice (dichlorométhane/tétrahydrofuranne : 98/2) permet d'obtenir 1 g du produit attendu.

### Stade 2 : Chlorhydrate de [1-({[6-chloro-5-(4-isothiocyanatophénvl)pyridin-3-yl]oxy}-méthyl)cyclopropyl]méthylamine

Le composé est obtenu selon le procédé du stade 2 de l'exemple 1 en utilisant le composé obtenu au stade 1 précédent.
Point de fusion (cap) : 172-176°C
Spectrométrie de masse (ESI) m/z = 346,0770 Th ([M+H]⁺)

### Exemple 88 :

### Dichlorhydrate de méthyl{1-[({5-[3-(2H-tétrazol-5-yl)phényl]pyridin-3-yl}oxy)-méthyl]cyclopropyl}amine

### Stade 1: Méthyl{1-[({5-[3-(2H-tétrazol-5-yl)phényl]pyridin-3-yl}oxy)méthyl]-cyclopropyl}carbamate de tert-butyle

1,2 g d'azidotriméthylétain est additionné à une solution 0,7 g du composé obtenu au stade 1 de l'exemple 21 dans 20 ml de toluène. Le mélange réactionnel est porté 20 h au reflux puis concentré. Une chromatographie sur silice (dichlorométhane/méthanol : 95/5) permet d'obtenir 0,7 g de produit attendu.

### Stade 2 : Dichlorhydrate de méthyl{1-[({5-[3-(2H-tétrazol-5-y/)phényl]pyridin-3-yl}oxy)-méthyl]cyclopropyl}amine

10 ml d'une solution d'acide chlorhydrique 4N dans le dioxanne sont ajoutés à une solution de 0,65 g du composé obtenu au stade 1 précédent dans 10 ml d'éthanol. Après 20 h d'agitation le milieu réactionnel est dilué avec de l'éther puis filtré. Le solide collecté est repris dans l'eau et la solution aqueuse est lyophilisée pour obtenir 0,49 g de produit attendu.
Spectrométrie de masse (ESI) m/z = 323,1595Th ([M+H]⁺)

### Exemple 89 :

### Dichlorhydrate de méthyl{1-[({5-[4-(2H-tétrazol-5-yl)phényl]pyridin-3-yl}oxy)-méthyl]cyclopropyl}amine

### Stade 1 : Méthyl{1-[({5-[4-(2H-tétrazol-5-yl)phényl]pyridin-3-yl}oxy)méthyl]-cyclopropylcarbamate de tert-butyle

Le composé est obtenu selon le procédé du stade 1 de l'exemple 88 en utilisant le composé du stade 1 de l'exemple 20 à la place du composé du stade 1 de l'exemple 21.

### Stade 2 : Dichlorhydrate de méthyl{1-[({5-[4-(2H-tétrazol-5-yl)phényl]pyridin-3-yl}oxy)-méthyl]cyclopropyl}amine

Le composé est obtenu selon le procédé du stade 2 de l'exemple 88 en utilisant le composé obtenu au stade 1 précédent.
Spectrométrie de masse (ESI) m/z = 323,1610 Th ([M+H]⁺)

### Exemple 90 :

### Dichlorhydrate de {1-[({6-chloro-5-[4-(2H-tétrazol-5-yl)phényl]pyridin-3-yl}oxy)-méthyl]cyclopropyl}méthylamine

### Stade 1 : {1-[({6-Chloro-5-[4-(2H-tétrazol-5-yl)phényl]pyridin-3-yl}oxy)méthyl]cyclopropyl}méthylcarbamate de tert-butyle

Le composé est obtenu selon le procédé du stade 1 de l'exemple 88 en utilisant le composé du stade 1 de l'exemple 18 à la place du composé du stade 1 de l'exemple 21.

### Stade 2 : Dichlorhydrate de {1-[({6-chloro-5-[4-(2H-tétrazol-5-yl)phényl]pyridin-3-yl}-oxy)méthyl]cyclopropyl}méthylamine

6 ml d'une solution d'acide chlorhydrique 4N dans le dioxanne sont ajoutés à une solution de 0,84 g du composé obtenu au stade 1 précédent dans 15 ml d'éthanol. Après 20 h d'agitation le milieu réactionnel est dilué avec de l'éther puis filtré pour obtenir après séchage du solide 0,75 g de produit attendu.
Point de fusion (car) : décomposition > 160°C
Spectrométrie de masse (ESI) m/z = 357,1216 Th ([M+H]⁺)

### Exemple 91 :

### Dichlorhydrate de {1-[({6-chloro-5-[3-(2H-tétrazol-5-yl)phényl]pyridin-3-yl}oxy)-méthyl]cyclopropyl}méthylamine

### Stade 1 : 3-(2-Chloro-5-{[1-(méthylamino)cyclopropyl]méthoxy}pyridin-3-yl)benzonitrile

Le composé est obtenu selon le procédé du stade 1 de l'exemple 1 en utilisant le composé de la préparation 2 à la place du composé de la préparation 1 et en remplaçant l'acide (3-méthoxyphényl)boronique par l'acide (3-cyanophényl)boronique.

### Stade 2 : {1-[({6-Chloro-5-[3-(2H-tétrazol-5-yl)phényl]pyridin-3-yl}oxy)méthyl]cyclopropyl}méthylcarbamate de tert-butyle

Le **c**omposé est obtenu selon le procédé du stade 1 de l'exemple 88 en utilisant le composé obtenu au stade 1 précédent.

### Stade 3 : Dichlorhydrate de {1-[({6-chloro-5-[3-(2H-tétrazol-5-yl)phényl]pyridin-3-yl}-oxy)méthyl]cyclopropyl}méthylamine

Le composé est obtenu selon le procédé du stade 2 de l'exemple 88 en utilisant le composé obtenu au stade 2 précédent.
Spectrométrie de masse (ESI) m/z = 357,1230 Th ([M+H]⁺)

### Exemple 92 :

### Dichlorhydrate de (1S,2R),(1R,2S)-N,2-diméthyl-1-({[5-(2-méthylphényl)pyridin-3-yl]oxy}méthyl)cyclopropanamine

0,17 g de tetrakis(triphenylphosphine)palladium sont ajoutés sous azote à une solution de 1 g du composé de la préparation 6 dans 20 ml de toluène. Le milieu est agité 20 minutes puis une solution de 0,61 g d'acide (2-méthylphényl)boronique dans 10 ml d'éthanol et 10 ml d'une solution aqueuse saturée d'hydrogénocarbonate de sodium sont ajoutés. Le milieu réactionnel est porté 12 h à 80 °C puis filtré et décanté. La phase organique est séchée sur sulfate de sodium et concentrée. Le résidu est chromatographié sur sur colonne RP 18, 12-25µ, (eau/acide trifluoroacétique : 1000/2,5 à eau/acétonitrile/acide trifluoroacétique : 800/200/2,5). Les fractions de chromatographie sont réunies, puis l'acétonitrile est évaporé. La solution aqueuse résiduelle est neutralisée puis saturée avec de l'hydrogénocarbonate de sodium solide puis extraite au dichlorométhane. Après séchage sur sulfate de sodium et concentration de la phase organique, 0,57 g de base obtenue sont mis en solution dans 10 ml d'éthanol et 1,35 ml d'une solution d'acide chlorhydrique 4N dans le dioxanne est ajoutée. Après concentration et cristallisation dansl'éther, on filtre et on sèche pour obtenir 0,51 g du produit attendu.
Point de fusion (cap) : 198-201°C
Spectrométrie de masse (ESI) m/z = 282 Th ([M+H]⁺)

### Exemple 93 :

### Dichlorhydrate de (1S,2R),(1R,2S)-1-[({5-[3,5-bis(trifluorométhyl)phényl]pyridin-3-yl}oxy)méthyl]-N,2-diméthylcyclopropanamine

Le composé est obtenu selon le procédé de l'exemple 92 en remplaçant l'acide (2-méthylphényl)boronique par l'acide [3,5-bis(trifluorométhyl)phényl]boronique.
Point de fusion (cap) : 117-120°C
Spectrométrie de masse (ESI) m/z = 405,1418 Th ([M+H]⁺)

### Exemple 94 :

### Dichlorhydrate de (1S,2R),(1R,2S)-N,2-diméthyl-1-({[5-(2,3,4-triméthoxyphényl)-pyridin-3-yl]oxy}méthyl)cyclopropanamine

Le composé est obtenu selon le procédé de l'exemple 92 en remplaçant l'acide (2-méthylphényl)boronique par l'acide (2,3,4-triméthoxyphényl)boronique.
Point de fusion (cap) : 196-199°C
Spectrométrie de masse (ESI) m/z = 359,1964 Th ([M+H]⁺)

### Exemple 95 :

### Dichlorhydrate de [1-({[5,6-bis(4-chlorophényl)pyridin-3-yl]oxy}méthyl)cyclopropyl]-méthylamine

### Stade 1 : [1-({[5,6-bis(4-Chlorophényl)pyridin-3-yl]oxy}méthyl)cyclopropyl]-méthylcarbamate de tert-butyle

Le composé, produit de disubstitution, est obtenu lors du procédé du stade 1 de l'exemple 13

### Stade 2 : Dichlorhydrate de [1-({[5,6-bis(4-chlorophényl)pyridin-3-yl]oxy}méthyl)cyclopropyl]méthylamine

Le composé est obtenu selon le procédé du stade 2 de l'exemple 2 en utilisant le composé obtenu au stade 1 précédent.
Point de fusion (cap) : 220-225°C
Spectrométrie de masse (ESI) m/z = 399,1033 Th ([M+H]⁺)

### Exemple 96 :

### Dichlorhydrate de [1-({[5,6-bis(4-nitrophényl)pyridin-3-yl]oxy}méthyl)cyclopropyl]-méthylamine

### Stade 1: [1-({[5,6-bis(4-Nitrophényl)pyridin-3-yl]oxy}méthyl)cyclopropyl]-méthylcarbamate de tert-butyle

Le composé, produit de disubstitution, est obtenu lors du procédé du stade 1 de l'exemple 12.

### Stade 2 : Dichlorhydrate de [1-({[5,6-bis(4-nitrophényl)pyridin-3-yl]oxy}méthyl)cyclopropyl]méthylamine

Le composé est obtenu selon le procédé du stade 2 de l'exemple 2 en utilisant le composé obtenu au stade 1 précédent.
Point de fusion (cap) : 215-220°C
Spectrométrie de masse (ESI) m/z = 421,1500 Th ([M+H]⁺)

### Exemple 97 :

### Dichlorhydrate de 4,4'-(5-{[1-(méthylamino)cyclopropyl]méthoxy}pyridine-2,3-diyl)-dibenzonitrile

### Stade 1 : [1-({5,6-bis(4-cyanophényl)pyridin-3-yl]oxy}méthyl)cyclopropyl]-méthylcarbamate de tert-butyle

Le composé, produit de disubstitution, est obtenu lors du procédé du stade 1 de l'exemple 18.

### Stade 2 : Dichlorhydrate de 4,4'-(5-{[1-(méthylamino)cyclopropyl]méthoxy}pyridine-2,3-diyl)dibenzonitrile

Le composé est obtenu selon le procédé du stade 2 de l'exemple 2 en utilisant le composé obtenu au stade 1 précédent.
Point de fusion (cap) : 205-208°C
Spectrométrie de masse (ESI) m/z = 381,1718 Th ([M+H]⁺)

### Exemple 98 :

### Trichlorhydrate de [1-({[5-(4-aminophényl)-6-méthylpyridin-3-yl]oxy}méthyl) cyclopropyl]méthylamine

### Stade 1 : [1-({[5-(4-Aminophényl)-6-méthylpyridin-3-yl]oxy}méthyl)cyclopropyl]méthylcarbamate de tert-butyle

Le composé est obtenu selon le procédé du stade 1 de l'exemple 1 en utilisant le composé de la préparation 3 à la place du composé de la préparation 1 et en remplaçant l'acide (3-méthoxyphényl)boronique par l'acide (4-aminophényl)boronique.

### Stade 2 : Trichlorhydrate de [1-({[5-(4-aminophényl)-6-méthylpyridin-3-yl]oxy}-méthyl)cyclopropyl]méthylamine

40 ml de dioxanne puis 10 ml d'une solution d'acide chlorhydrique 4N dans le dioxanne sont ajoutés à 1,05 g du produit obtenu au stade 1 précédent. 40 ml d'éthanol sont ajoutés pour parvenir à une homogénéisation complète du milieu réactionnel. Après 20 h d'agitation, les solvants sont évaporés et le résidu est repris par le minimun d'éthanol. Après dilution à l'éther, filtration et séchage, on obtient 0,9g de produit attendu.
Point de fusion (cap) : 245-250°C
Spectrométrie de masse (ESI) m/z = 284,169 Th ([M+H]⁺)

### Exemple 99 :

### Dichlorhydrate de l'acide 3-[5-({[1-(méthylamino)cyclopropyl]méthyl}-amino)pyridin-3-yl]benzoique

### Stade 1: Acide 3-{5-[({1-[(tert-butoxycarbonyl)(méthyl)amino]cyclopropyl}-méthyl)amino]pyridin-3-yl}benzoique

On ajoute successivement une solution contenant 3,96 g de carbonate de sodium dans 94 mL d'eau puis 2,05 g d'acide 3-carboxyphénylboronique dans un mélange contenant 140 mL d'acétonitrile et 4,68 g du composé de la préparation 8. On agite 1 heure sous azote puis on ajoute 0,55 g de tétrakis(triphénylphosphine)palladium. On agite 1 heure à 20°C puis 20heures au reflux. On concentre à sec, reprend dans 40 mL d'eau, et extrait à l'éther plusieurs fois. On acidifie la phase aqueuse par de l'acide chlorhydrique N jusqu'à pH 5,5. On extrait au dichlorométhane, sèche sur sulfate de sodium et concentre à sec. Une chromatographie sur silice (dichlorométhane/méthanol : 95/5) permet d'obtenir 2,4 g de produit attendu.
Point de fusion (cap) : 109°C

### Stade 2 : Dichlorhydrate de l'acide 3-[5-({[1-(méthylamino)cyclopropyl]-méthyl}amino)pyridin-3-yl]benzoique

On dissous 2,26 g du composé obtenu au stade 1 précédent dans 44 mL de dioxane. On ajoute 44 mL d'acide chlorhydrique 4 M dans le dioxane puis après une heure d'agitation 7,3 mL d'eau. On agite 20 heures. On essore, lave le précipité à l'éther et sèche à 60°C sous 0,5 torr. On obtient 1,96 g de produit attendu.
Point de fusion (cap) : 248-250°C
Spectrométrie de masse (ESI) (H₂O/CH₃CN) m/z = 298,1527 Th ([M+H]⁺)

### Exemple 100 :

### Dichlorhydrate de l'acide 4-[5-({[1-méthylamino)cyclopropyl]méthyl}amino)pyridin-3-yl]benzoique

### Stade 1 : Acide 4-{5-[({1-[(tert-butoxycarbonyl)(méthyl)amino]cyclopropyl}-méthyl)amino]pyridin-3-yl}benzoique

Le composé est obtenu selon le procédé du stade 1 de l'exemple 99 en remplaçant l'acide 3-carboxyphénylboronique par l'acide 4-carboxyphénylboronique.
Point de fusion (cap) : 120°C

### Stade 2 : Dichlorhydrate de l'acide 4-[5-({[1-méthylamino)cyclopropyl]méthyl}-amino)pyridin-3-yl]benzoique

Le composé est obtenu selon le procédé du stade 2 de l'exemple 99 en utilisant le composé obtenu au stade 1 précédent.
Point de fusion (cap) : 256-260°C
Spectrométrie de masse (ESI) (H₂O/CH₃CN) m/z = 298,1522 Th ([M+H]⁺)

### Exemple 101 :

### Dichlorhydrate de l'acide 4-[5-(méthyl{[1-(méthylamino)cyclopropyl]méthyl}amino)-pyridin-3-yl]benzoique

### Stade 1 : Acide 4-{5-[({1-[(tert-butoxycarbonyl)(méthyl)amino]cyclopropyl}méthyl)-(méthyl)amino]pyridin-3-yl}benzoïque

Le composé est obtenu selon le procédé du stade 1 de l'exemple 99 en utilisant le composé de la préparation 9 à la place du composé de la préparation 8 et en remplaçant l'acide 3-carboxyphénylboronique par l'acide 4-carboxyphénylboronique.

### Stade 2 : Dichlorhydrate de l'acide 4-[5-(méthyl{[1-(méthylamino)cyclopropyl]méthyl}-amino)pyridin-3-yl]benzoique

Le composé est obtenu selon le procédé du stade 2 de l'exemple 99 en utilisant le composé obtenu au stade 1 précédent.
Spectrométrie de masse (ESI) (H₂O/CH₃CN) m/z = 312,1676 Th ([M+H]⁺)

### Exemple 102 :

### Dichlorhydrate de l'acide 3-[5-(méthyl{[1-(méthylamino)cyclopropyl]méthyl}amino)-pyridin-3-yl]benzoique

### Stade 1 : Acide 3-{5-[({1-[tert-butoxycarbonyl)(méthyl)amino]cyclopropyl}méthyl)-(méthyl)amino]pyridin-3-yl]benzoique

Le composé est obtenu selon le procédé du stade 1 de l'exemple 99 en utilisant le composé de la préparation 9 à la place du composé de la préparation 8.

### Stade 2 : Dichlorhydrate de l'acide 3-[5-(méthyl{[1-(méthylamino)cyclopropyl]méthyl}amino)pyridin-3-yl]benzoique

Le composé est obtenu selon le procédé du stade 2 de l'exemple 99 en utilisant le composé obtenu au stade 1 précédent.
Spectrométrie de masse (ESI) (H₂O/CH₃CN) m/z = 312,1689 Th ([M+H]+)

### Exemple 103 :

### Trichlorhydrate de 2-chloro-N-{[1-(méthylamino)cyclopropyl]méthyl}-3,3'-bipyridin-5-amine

### Stade 1 : (1-{[(2-Chloro-3,3'-bipyridin-5-yl)amino]méthyl}cyclopropyl)méthylcarbamate de tert-butyle

On agite pendant 1 heure à 20°C un mélange composé de 1 g du composé de la préparation 10, 25 mL de toluène et 0,15 g de tétrakis(triphénylphosphine)palladium. On ajoute successivement 0,39 g d'acide pyridine 3-boronique, 12,5 mL d'éthanol et 12,5 mL de solution saturée aqueuse d'hydrogénocarbonate de sodium. On porte 20 heures à 80°C sous agitation efficace. Après refroidissement, on ajoute du toluène, décante, sèche la phase organique sur sulfate de sodium et concentre à sec. Une chromatographie sur silice (dichlorométhane/tétrahydrofurane : 97/3) permet d'obtenir 0,99 g de produit attendu.

### Stade 2 : Trichlorhydrate de 2-chloro-N-{[1-(méthylamino)cyclopropyl]méthyl}-3,3'-bipyridin-5-amine

On agite pendant 20 heures à température ambiante 1,0 g de produit obtenu au stade 1 précédent avec 50 mL d'éthanol et 10 mL d'acide chlorhydrique 4N dans le dioxane. On dilue avec de l'éther puis essore et sèche à 60°C sous 1 torr. On obtient 0,78 g de produit souhaité.
Point de fusion (cap) : 180-185°C
Spectrométrie de masse (ESI) (H₂O/CH₃CN) m/z = 289,1219 Th ([M+H]⁺)

### Exemple 104 :

### Dichlorhydrate de l'acide 3-[2-chloro-5-({[1-(méthylamino)cyclopropyl]méthyl}amino)pyridin-3-yl]benzoique

### Stade 1 : Acide 3-{5[({1-[(tert-butoxycarbonyl)(méthyl)amino]cyclopropyl}méthyl)-amino]-2-chloropyridin-3-yl}benzoiqu

Le composé est obtenu selon le procédé du stade 1 de l'exemple 99 en utilisant le composé de la préparation 10 à la place du composé de la préparation 8.

### Stade 2 : Dichlorhydrate de l'acide 3-[2-chloro-5-({[1-(méthylamino)cyclopropyl] méthyl}amino)pyridin-3-yl]benzoique

Le composé est obtenu selon le procédé du stade 2 de l'exemple 99 en utilisant le composé obtenu au stade 1 précédent.
Point de fusion (cap) : 140-145°C
Spectrométrie de masse (ESI) (H₂O/CH₃CN) m/z = 332,1 Th ([M+H]+)

### Exemple 105 :

### Dichlorhydrate de l'acide 4-[2-chloro-5-({[1-(méthylamino)cyclopropyl]méthyl}-amino)pyridin-3-yl]benzoique

### Stade 1 : Acide 4-{5-[({1-(tert-butoxycarbonyl)méthyl)amino]cyclopropyl}méthyl)-amino]-2-chloropyridin-3-yl}benzoique

Le composé est obtenu selon le procédé du stade 1 de l'exemple 99 en utilisant le composé de la préparation 10 à la place du composé de la préparation 8 et en remplaçant l'acide 3-carboxyphénylboronique par l'acide 4-carboxyphénylboronique.

### Stade 2 : Dichlorhydrate de l'acide 4-[2-chloro-5-({[1-(méthylamino)cyclopropyl]-méthyl}amino)pyridin-3-yl]benzoique

Le composé est obtenu selon le procédé du stade 2 de l'exemple 99 en utilisant le composé obtenu au stade 1 précédent.
Point de fusion (cap) : 175-182°C
Spectrométrie de masse (ESI) (H₂O/CH₃CN) m/z = 332,1147 Th ([M+H]+)

### Exemple 106 :

### Dichlorhydrate de 2-chloro-N-{[1-(méthylamino)cyclopropyl]méthyl}-3,4'-bipyridin-5-amine

### Stade 1 : (1-{[(2-Chloro-3,4'-bipyridin-5-yl)amino]méthyl}cyclopropyl)méthylcarbamate de tert-butyle

Le composé est obtenu selon le procédé du stade 1 de l'exemple 103 en remplaçant l'acide pyridine 3-boronique par l'acide pyridine 4-boronique.

### Stade 2 : Dichlorhydrate de 2-chloro-N-{[1-(méthylamino)cyclopropyl]méthyl}-3,4'-bipyridin-5-amine

Le composé est obtenu selon le procédé du stade 2 de l'exemple 103 en utilisant le composé obtenu au stade 1 précédent.
Point de fusion (cap) : 100-110°C
Spectrométrie de masse (ESI) (H₂O/CH₃CN) m/z = 289,1219 Th ([M+H]⁺)

### Exemple 107 :

### Dichlorhydrate de 6-chloro-5-(4-chlorophényl)-N-{[1-(méthylamino)cyclopropyl]-méthyl}pyridin-3-amine

### Stade 1 : [1-({[6-Chloro-5-(4-chlorophényl)pyridin-3-yl]amino}méthyl)cyclopropyl]-méthylcarbamate de tert-butyle

Le composé est obtenu selon le procédé du stade 1 de l'exemple 103 en remplaçant l'acide 3-pyridinboronique par l'acide (4-chlorophényl)boronique.

### Stade 2 : Dichlorhydrate de 6-chloro-5-(4-chlorophényl)-N-{[1-(méthylamino)-cyclopropyl]méthyl}pyridin-3-amine

On ajoute 18 mL d'acide chlorhydrique 4N dans le dioxane à 2 g de produit issu du stade 1 précédent en solution dans 80 mL de dioxane. On agite 16 heures à 20°C et on dilue avec 80 mL d'éther. On agite 1 heure, essore et sèche le précipité à 50°C sous 1 torr. On obtient 1,57g de produit attendu.
Point de fusion (cap) : 128-139°C
Spectrométrie de masse (ESI) (H₂O/CH₃CN) m/z = 332,0876 Th ([M+H]⁺)

### Exemple 108 :

### Dichlorhydrate de 5-(4-aminophényl)-6-chloro-N-{[1-(méthylamino)cyclopropyl]-méthyl}pyridin-3-amine

### Stade 1 : [1-({[5-(4-Aminophényl)-6-chloropyridin-3-yl]amino}méthyl) cyclopropyl]-méthylcarbamate de tert-butyle

Sous atmosphère d'azote, on agite pendant 30 minutes un mélange constitué de 2,0 g du composé de la préparation 10, 0,25 g de tétrakis(triphénylphosphine)palladium et 1,33 g de 4-(4,4,5,5-tétraméthyl-1,3,2-dioxa-borolan-2-yl)aniline dans 60 ml de THF. On ajoute 3,2 g de carbonate de potassium dissous dans 20 mL d'eau. On agite 20 heures à 60°C. On concentre à sec. On reprend dans une solution aqueuse de carbonate de sodium et on extrait au dichlorométhane. On sèche la phase dichlorométhane sur sulfate de sodium et concentre à sec. Une chromatographie sur silice (dichlorométhane/butanone : 93/7) permet d'obtenir 1,67 g du produit attendu.

### Stade 2 : Dichlorhydrate de 5-(4-aminophényl)-6-chloro-N-{[1-(méthylamino)-cyclopropyl]méthyl}pyridin-3-amine

Le composé est obtenu selon le procédé du stade 2 de l'exemple 103 en utilisant le composé obtenu au stade 1 précédent.
Point de fusion (cap) : 190-196°C
Spectrométrie de masse (ESI) (H₂O/CH₃CN) m/z = 303,1 Th ([M+H]⁺)

### Exemple 109 :

### Chlorhydrate de 4-[2-chloro-5-({[1-(méthylamino)cyclopropyl]méthyl}amino)pyridin-3-yl]benzonitrile

### Stade 1 : [1-({[6-Chloro-5-(4-cyanophényl)pyridin-3-yl]amino}méthyl)cyclopropyl]-méthylcarbamate de tert-butyle

Le composé est obtenu selon le procédé du stade 1 de l'exemple 103 en remplaçant l'acide pyridine 3-boronique par l'acide (4-cyanophényl)boronique.

### Stade 2 : Chlorhydrate de 4-[2-chloro-5-({[1-(méthylamino)cyclopropyl]méthyl}amino)-pyridin-3-yl]benzonitrile

Le composé est obtenu selon le procédé du stade 2 de l'exemple 107 en utilisant le composé obtenu au stade 1 précédent.
Spectrométrie de masse (ESI) (H₂O/CH₃CN) m/z = 313,1 Th ([M+H]⁺)

### Exemple 110 :

### Dichlorhydrate de l'acide 4-[2-chloro-5-(méthyl{[1-(méthylamino)cyclopropyl]-méthyl}amino)pyridin-3-yl]benzoique

### Stade 1 : Acide 4-{5-[({1-[(tert-butoxycarbonyl)(méthyl)amino]cyclopropyl}méthyl-(méthyl)amino]-2-chloropyridin-3-yl}benzoique

Le composé est obtenu selon le procédé du stade 1 de l'exemple 99 en utilisant le composé de la préparation 11 à la place du composé de la préparation 8 et en remplaçant l'acide 3-carboxyphénylboronique par l'acide 4-carboxyphénylboronique.

### Stade 2 : Dichlorhydrate de l'acide 4-[2-chloro-5-(méthyl{[1-(méthylamino)cyclopropyl]-méthyl}amino)pyridin-3-yl]benzoique

Le composé est obtenu selon le procédé du stade 2 de l'exemple 99 en utilisant le composé obtenu au stade 1 précédent.
Point de fusion (cap) : 138-145°C
Spectrométrie de masse (ESI) (H₂O/CH₃CN) m/z = 346,1 Th ([M+H]⁺)

### Exemple 111 :

### Dichlorhydrate de 2-chloro-N-méthyl-N-{[1-(méthylamino)cyclopropyl]méthyl}-3,4'-bipyridin-5-amine

### Stade 1 : (1-{[(2-Chloro-3,4'-bipyridin-5-yl)(méthyl)amino]méthyl} cyclopropyl)méthylcarbamate de tert-butyle

Le composé est obtenu selon le procédé du stade 1 de l'exemple 103 en utilisant le composé de la préparation 11 à la place du composé de la préparation 10 et en remplaçant l'acide pyridine 3-boronique par l'acide pyridine 4-boronique.

### Stade 2 : Dichlorhydrate de 2-chloro-N-méthyl-N-{[1-(méthylamino)cyclopropyl]-méthyl}-3,4'-bipyridin-5-amine

Le composé est obtenu selon le procédé du stade 2 de l'exemple 103 en utilisant le composé obtenu au stade 1 précédent.
Point de fusion (cap) : 110-120°C
Spectrométrie de masse (ESI) (H₂O/CH₃CN) m/z = 303,1 Th ([M+H]⁺)

### Exemple 112 :

### Dichlorhydrate de l'acide 4-[2-méthyl-5-({[1-(méthylamino)cyclopropyl]méthyl}-amino)pyridin-3-yl]benzoique

### Stade 1 : Acide 4-{5-[({1-[(tert-butoxycarbonyl)(méthyl)amino]cyclopropyl}méthyl)-amino]-2-méthylpyridin-3-yl}benzoique

Le composé est obtenu selon le procédé du stade 1 de l'exemple 99 en utilisant le composé de la préparation 12 à la place du composé de la préparation 8 et en remplaçant l'acide 3-carboxyphénylboronique par l'acide 4-carboxyphénylboronique.
Point de fusion (cap) : 146°C

### Stade 2 : Dichlorhydrate de l'acide 4-[2-méthyl-5-({[1-(méthylamino)cyclopropyl]-méthyl}amino)pyridin-3-yl]benzoique

Le composé est obtenu selon le procédé du stade 2 de l'exemple 99 en utilisant le composé obtenu au stade 1 précédent.
Point de fusion (cap) : 241-245°C
Spectrométrie de masse (ESI) (H₂O/CH₃CN) m/z = 312,2 Th ([M+H]⁺)

### Exemple 113 :

### Trichlorhydrate de 2-méthyl-N-{[1-(méthylamino)cyclopropyl]méthyl}-3,4'-bipyridin-5-amine

### Stade 1 : Méthyl(1-{[(2-méthyl-3,4'-bipyridin-5-yl)amino]méthyl}cyclopropyl)carbamate de tert-butyle

Le composé est obtenu selon le procédé du stade 1 de l'exemple 103 en utilisant le composé de la préparation 12 à la place du composé de la préparation 10 et en remplaçant l'acide pyridine 3-boronique par l'acide pyridine 4-boronique.

### Stade 2 : Trichlorhydrate de 2-méthyl-N-{[1-(méthylamino)cyclonropyl]méthyl}-3,4'-bipyridin-5-amine

Le composé est obtenu selon le procédé du stade 2 de l'exemple 103 en utilisant le composé obtenu au stade 1 précédent.
Point de fusion (cap) : 240-248°C
Spectrométrie de masse (ESI) (H₂O/CH₃CN) m/z = 269,2 Th ([M+H]⁺)

### Exemple 114 :

### Trichlorhydrate de 5-(4-aminophényl)-6-méthyl-N-{[1-(méthylamino)cyclopropyl]-méthyl}pyridin-3-amine

### Stade 1 : [1-({[5-(4-aminophenyl)-6-méthylpyridin-3-yl]amino}méthyl)cyclopropyl]-méthylcarbamate de tert-butyle

Le composé est obtenu selon le procédé du stade 1 de l'exemple 108 en utilisant le composé de la préparation 12 à la place du composé de la préparation 10.

### Stade 2 : Trichlorhydrate de 5-(4-aminophényl)-6-méthyl-N-{[1-(méthylamino)-cyclopropyl]méthyl}pyridin-3-amine

Le composé est obtenu selon le procédé du stade 2 de l'exemple 103 en utilisant le composé obtenu au stade 1 précédent.
Point de fusion (cap) : 190-200°C
Spectrométrie de masse (ESI) (H₂O/CH₃CN) m/z = 283,2 Th ([M+H]⁺)

### Exemple 115 :

### Dichlorhydrate de l'acide 4-[2-méthyl-5(méthyl{[1-(méthylamino)cyclopropyl]-méthyl}amino)pyridin-3-yl]benzoique

### Stade 1 : Acide 4-{5-[({1-[(tert-butoxycarbonyl)(méthyl)amino]cyclopropyl}méthyl-(méthyl)amino]-2-méthylpyridin-3-yl}benzoique

Le composé est obtenu selon le procédé du stade 1 de l'exemple 99 en utilisant le composé de la préparation 13 à la place du composé de la préparation 9 et en remplaçant l'acide 3-carboxyphénylboronique par l'acide 4-carboxyphénylboronique.

### Stade 2 : Dichlorhydrate d'acide 4-[2-méthyl-5(méthyl{[1-(méthylamino)cyclopropyl]-méthyl}amino)pyridin-3-yl]benzoique

Le composé est obtenu selon le procédé du stade 2 de l'exemple 99 en utilisant le composé obtenu au stade 1 précédent.
Spectrométrie de masse (ESI) (H₂O/CH₃CN) m/z = 326,1870 Th ([M+H]⁺)

### Exemple 116 :

### Trichlorhydrate de N,2-diméthyl-N-{[1-(méthylamino)cyclopropyl]méthyl}-3,4'-bipyridin-5-amine

### Stade 1 : Méthyl (1-{[méthyl(2-méthyl-3,4'-bipyridin-5-yl)amino]méthyl}cyclopropyl)-carbamate de tert-butyle

Le composé est obtenu selon le procédé du stade 1 de l'exemple 103 en utilisant le composé de la préparation 13 à la place du composé de la préparation 10 et en remplaçant l'acide pyridine 3-boronique par l'acide pyridine 4-boronique.

### Stade 2 : Trichlorhydrate de N,2-diméthyl-N-{[1-(méthylamino)cyclopropyl]méthyl}-3,4'-bipyridin-5-amine

Le composé est obtenu selon le procédé du stade 2 de l'exemple 103 en utilisant le composé obtenu au stade 1 précédent.
Point de fusion (cap) : 182-187°C
Spectrométrie de masse (ESI) (H₂O/CH₃CN) m/z = 283,2 Th ([M+H]⁺)

### Exemple 117 :

### Trichlorhydrate de 5-(4-aminophényl)-N,6-diméthyl-N-{[1-(méthylamino)-cyclopropyl]méthyl}pyridin-3-amine

### Stade 1 : (1-{[[5-(4-Aminophényl)-6-méthylpyridin-3-yl](méthyl)amino]méthyl}cyclopropyl)méthylcarbamate de tert-butyle

Le composé est obtenu selon le procédé du stade 1 de l'exemple 108 en utilisant le composé de la préparation 13 à la place du composé de la préparation 10.

### Stade 2 : Trichlorhydrate de 5-(4-aminophényl)-N,6-diméthyl-N-{[1-(méthylamino)-cyclopropyl]méthyl}pyridin-3-amine

Le composé est obtenu selon le procédé du stade 2 de l'exemple 103 en utilisant le composé obtenu au stade 1 précédent.
Point de fusion (cap) : 235-240°C
Spectrométrie de masse (ESI) (H₂O/CH₃CN) m/z = 297,2 Th ([M+M]⁺)

### ETUDES PHARMACOLOGIQUES DES COMPOSES DE L'INVENTION

### EXEMPLE A:

### Déplacement de la fixation de l,[¹²⁵I]-α-bungarotoxine sur les récepteurs nicotiniques de l'organe électrique de torpille

Cette étude, réalisée selon la méthode décrite dans J. Pharmacol. Exp. Ther., 1994, 271; 624-631, a pour objectif d'évaluer l'affinité des composés de la présente invention sur les récepteurs nicotiniques de « type musculaire ».
Des membranes (1-5 µg/ml) d'organe électrique de torpille sont incubées (1h, 22°C) en présence d'une gamme de concentration (0,01-10 µM) de chaque composé de l'invention (dilution à partir d'une solution mère à 10 mM dans du DMSO) en présence d'[¹²⁵I]-α-bungarotoxine (A.S. : 7,4 TBq/mmol : 0,2 nM) dans du tampon Krebs (Tris-HCl 50 mM, KCl 5 mM, MgCl₂ 1 mM, CaCl₂ 2 mM, NaCl 100 mM, pH 7.4) avec 0,01 % de BSA, volume final de 500 µl. La liaison non-spécifique est déterminée par l'incubation de membranes en présence d'α-bungarotoxine (1 µM).
Les résultats indiquent que les composés de la présente invention ne possèdent aucune affinité significative vis-à-vis des récepteurs nicotiniques de « type musculaire » jusqu'à la concentration de 10 µM.

### EXEMPLE B :

### Déplacement de la fixation d'[³H]-épibatidine sur les récepteurs nicotiniques de cellules IMR32

Cette étude, réalisée selon la technique décrite dans Molec. Pharmacol., 1995, 48 ; 280-287, a pour but de déterminer l'affinité des composés de la présente invention sur les récepteurs nicotiniques de « type ganglionnaire » (American Soc. Neuroscience, 2000, 26, 138).
Des membranes (250 µg/ml) de cellules neuroblastome IMR-32 sont incubées (2h, 20°C) en présence d'une gamme de concentration (0,01-10 µM) de chaque composé de l'invention (dilution à partir d'une solution mère à 10 mM dans du DMSO) et d'(±)-[³H]-épibatidine (A.S. : 2464 GBq/mmol: 1,5 nM) dans du tampon phosphate (NaH₂PO₄ 20 mM, pH 7,4), volume final de 250 µl. La liaison non-spécifique est déterminée par l'incubation de membranes en présence de 300 µM de (-)nicotine.
Les résultats montrent que les composés de la présente invention n'ont aucune affinité significative sur les récepteurs nicotiniques de « type ganglionnaire », jusqu'à la concentration de 10 µM.

### EXEMPLE C :

### Déplacement de la fixation d'[³H]-oxotrémorine-M sur les récepteurs muscariniques de cerveau de rat

Cette étude, réalisée selon la méthode décrite dans Naumyn-Schmiederberg's Arch. Pharmacol., 2001, 363, 429-438, a pour objectif de déterminer l'affinité des composés de la présente invention sur les récepteurs muscariniques.
Des membranes (250 µg/ml) de cerveau de rat sont incubées (2h, 20°C) en présence d'une gamme de concentration (0,01-10 µM) de chaque composé de l'invention (dilution à partir d'une solution mère à 10 mM dans du DMSO) et d'[³H]-oxotrémorine-M (A.S. : 3174 GBq/mmol : 2 nM) dans du tampon phosphate (NaH₂PO₄ 20 mM, pH 7,4), volume final de 250 µl. La liaison spécifique est déterminée par l'incubation des membranes en présence d'atropine (1 µM). L'affinité des composés de la présente invention vis-à-vis des récepteurs muscariniques est caractérisée par la détermination du Kᵢ.
Les résultats démontrent que la plupart des composés de la présente invention, jusqu'à la concentration de 10 µM, sont dépourvus d'affinité vis-à-vis des récepteurs muscariniques.

### EXEMPLE D :

### Déplacement de la fixation de l'[¹²⁵I]-α-bungarotoxine sur les récepteurs nicotiniques de « type α7 » de cerveau de rat

Cette étude, réalisée selon la méthode décrite dans Molec. Pharmacol., 1986, 30 ; 427-436, a pour objectif de déterminer l'affinité des composés de la présente invention vis-à-vis des récepteurs centraux nicotiniques de type α7.
Des membranes (1000 µg/ml) de cerveau de rat sont incubées (5h, 37°C) en présence d'une gamme de concentration (0,01-10 µM) de chaque composé de la présente invention (dilution à partir d'une solution mère à 10 mM dans du DMSO) et d' [¹²⁵I]-α-bungarotoxine (A.S. : 7,4 TBq/mmol : 1 nM) dans du tampon Krebs (Tris-HCl 50 mM, Cl 5 mM, MgCl₂ 1 mM, CaCl₂ 2 mM, NaCl 100 mM, pH 7,4) avec 0,05 % de BSA, volume final de 500 µl. La liaison non-spécifique est déterminée par l'incubation de membranes en présence d'α-bungarotoxine (1 µM).
L'affinité des composés de la présente invention vis-à-vis des récepteurs nicotiniques de type α7 est caractérisée par la détermination du Kᵢ.
Les résultats indiquent que la plupart des composés de la présente invention, jusqu'à la concentration de 10 µM, sont dépourvus d'affinité vis-à-vis des récepteurs nicotiniques centraux de type α7. Certains composés de l'invention présentent un Kᵢ de l'ordre de 10 µM.

### EXEMPLE E :

### Déplacement de la fixation de [³H]-cytisine sur les récepteurs nicotiniques de « type α4β2 » de cerveau de rat

Cette étude, réalisée selon la technique décrite dans Molec. Pharmacol., 1990, 39 ; 9-12, a pour objectif de déterminer l'affinité des composés de la présente invention vis-à-vis des récepteurs nicotiniques centraux de type α4β2.
Des membranes (250 µg/ml) de cerveau de rat sont incubées (2h, 20°C) en présence d'une gamme de concentration (0,01-10 µM) de chaque composé de la présente invention (dilution à partir d'une solution mère à 10 mM dans du DMSO) et de [³H]-cytisine (A.S. 1184 GBq/mmol : 2 nM) dans du tampon phosphate (NaH₂PO₄ 20 mM, pH 7,4), volume final de 250µl. La liaison non-spécifique est déterminée par l'incubation de membranes en présence de 10 µM de (-)nicotine. L'affinité des composés de la présente invention vis-à-vis des récepteurs nicotiniques centraux de type α₄β₂ est caractérisée par la détermination du Kᵢ.
Les résultats obtenus montrent que les composés de la présente invention présentent une forte affinité vis-à-vis des récepteurs nicotiniques centraux de type α₄β₂ avec des Kᵢ de l'ordre de 1 nM.

Ces résultats, ainsi que ceux obtenus dans les exemples A à D indiquent que les composés de la présente invention sont de puissants ligands nicotiniques centraux spécifiques des récepteurs de type α₄β₂.

**TABLEAU 1**

| **Affinité (Ki, nM) des composés de la présente invention pour les récepteurs de type α₄β₂** | |
|---|---|
| Exemples | Ki (nM) |
| 1 | 7,9 |
| 2 | 8,5 |
| 6 | 2,3 |
| 10 | 0,7 |
| 11 | 0,8 |
| 26 | 0,3 |
| 32 | 0,4 |
| 52 | 1,4 |

### EXEMPLE F :

### Mesure in vivo de la libération d'acétylcholine par microdialyse intra-corticale chez le rat Wistar vigile

L'administration systémique de nicotine et d'agonistes nicotiniques induit une augmentation *in vivo* d'acétylcholine dans diverses régions cérébrales (Neurochem. Res., 1996, 21, 1181-1186 ; Eur. J. Pharmacol., 1998, 351, 181-188 ; Br. J. Pharmacol., 1999, 127, 1486-1494). Une sonde de microdialyse est implantée au niveau du cortex préfrontal médian de rats mâles Wistar. Six ou sept jours après l'implantation des sondes, celles-ci sont perfusées par du Ringer (NaCl 147 mM, KCl 2.7 mM, CaCl₂ 1.2 mM, MgCl₂ 1 mM, néostigmine 20 nM) à un débit de 1 µl/min chez l'animal libre de se mouvoir. Après 2 heures de stabulation, le produit étudié est administré par voie intrapéritonéale. Un groupe d'animaux témoins reçoit le solvant du produit. Puis, les dialysats (30 µl) sont collectés toutes les 30 minutes pendant 4h afin de mesurer les concentrations extra-synaptiques corticales d'acétylcholine par HPLC en détection ampérométrique. Les résultats sont exprimés en pg d'acétylcholine/dialysat et les comparaisons inter-groupes sont effectuées par une analyse de variance à 2 facteurs (traitement x temps) avec mesures répétées sur le temps.

Les résultats obtenus montrent que les composés de la présente invention augmentent la libération corticale *in vivo* d'acétylcholine de manière dose-dépendante pour des doses actives allant de 1 à 10 mg/kg IP et indiquent le caractère agoniste α4β2 des composés de la présente invention. Par exemple, le composé de l'exemple 1 augmente la libération d'acétylcholine (+ 72 %) à la dose de 10 mg/kg IP, et celui de l'exemple 18 de +104 % à la même dose.

### EXEMPLE G :

### Torsions abdominales induites à la phényl-p-benzoquinone (PBQ) chez la souris NMRI

L'administration intrapéritonéale d'une solution alcoolique de PBQ provoque des crampes abdominales chez la Souris (Proc. Soc. Exp. Biol., 1957, 95, 729-731). Ces crampes sont caractérisées par des contractions répétées de la musculature abdominale, accompagnées d'une extension des membres postérieurs. La plupart des analgésiques antagonisent ces crampes abdominales (Brit. J. Pharmacol. Chem., 1968, 32, 295-310). A t=0 minute, les animaux sont pesés et le produit étudié est administré par voie IP. Un groupe d'animaux témoins reçoit le solvant du produit. A t=30 minutes, une solution alcoolique de PBQ (0,2 %) est administrée par voie IP sous un volume de 0,25 ml/souris. Immédiatement après l'administration de la PBQ, les animaux sont placés dans des cylindres en plexiglass (L = 19,5 cm ; D.I. = 5 cm). De t=35 minutes à t=45 minutes, la réaction des animaux est observée et l'expérimentateur note le nombre total de crampes abdominales par animal. Les résultats sont exprimés par le pourcentage d'inhibition du nombre de crampes abdominales mesuré chez les animaux témoins à la dose active du composé étudié.

Les résultats obtenus montrent une inhibition allant de -80 %, pour des doses actives allant de 10 mg/kg IP. Ceci montre que les composés de l'invention sont pourvus de propriétés antalgiques. Par exemple, les composés 81 et 106, inhibent à la dose de 10 mg/kg IP, les torsions abdominales de -90% et -87%, respectivement.

### EXEMPLE H :

### Reconnaissance sociale chez le Rat Wistar

Initialement décrit en 1982 (J. Comp. Physiol., 1982, 96, 1000-1006), le test de la reconnaissance sociale a été ensuite proposé par différents auteurs (Psychopharmacology, 1987, 91, 363-368 ; Psychophamacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation, par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester par voie intrapéritonéale et, 2 heures plus tard, est remis en présence (5 minutes) du jeune rat. Le comportement de reconnaissance sociale est alors à nouveau observé et la durée en est mesurée. Le critère de jugement est la différence (T2-T1), exprimée en secondes, des temps de « reconnaissance » des 2 rencontres.

Les résultats obtenus montrent une différence (T2-T1) comprise entre -16 et -26 s pour des doses allant de 3 à 10 mg/kg IP. Ceci montre que les composés de l'invention augmentent la mémorisation de façon très importante, et à faible dose. Les résultats obtenus montrent une différence (T2-T1) comprise entre -16 et -26 pour des doses allant de 3 à 10 mg/kg IP pour le composé de l'exemple 1.

### EXEMPLE I :

### Compositions pharmaceutiques pour 1000 comprimés dosés à 100 mg

| | |
|---|---|
| Composé de l'exemple 1 | 10 g |
| Hydroxypropylméthylcellulose | 10 g |
| Amidon de blé | 15 g |
| Lactose | 90 g |
| Stéarate de magnésium | 2 g |

## Revendications

1. Composés de formule (I) : 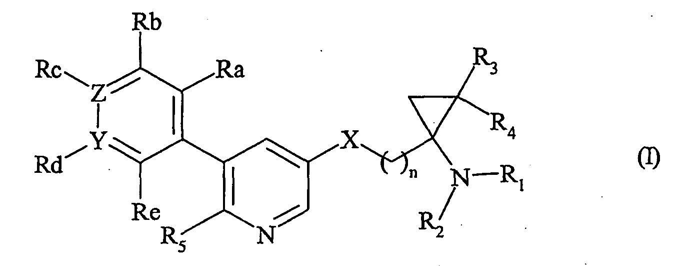 dans laquelle :
n représente un nombre entier compris entre 1 et 6 inclus,
X représente un atome d'oxygène ou un groupement NR₆,
Y représente un atome de carbone ou un atome d'azote, étant entendu que lorsque Y représente un atome d'azote, Rd est absent,
Z représente un atome de carbone ou un atome d'azote, étant entendu que lorsque Z représente un atome d'azote, Rc est abent,
R₁ et R₂, identiques ou différents, indépendamment l'un de l'autre, représentent un atome d'hydrogène, ou un groupement alkyle (C₁-C₆) linéaire ou ramifié ou arylalkyle (C₁-C₆) linéaire ou ramifié,
R₃ et R₄, identiques ou différents, indépendamment l'un de l'autre, représentent un atome d'hydrogène, ou un groupement alkyle (C₁-C₆) linéaire ou ramifié,
R₅ représente un atome d'hydrogène, ou un groupement alkyle (C₁-C₆) linéaire ou ramifié, halogène, hydroxy, alkoxy (C₁-C₆) linéaire ou ramifié, cyano, nitro, acyle (C₂-C₆) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, amino éventuellement substitué par un ou deux groupements alkyle (C₁-C₆) linéaire ou ramifié, aryle ou hétéroaryle,
R₆ représente un atome d'hydrogène, ou un groupement alkyle (C₁-C₆) linéaire ou ramifié ou arylalkyle (C₁-C₆) linéaire ou ramifié,
Ra, Rb, Rc, Rd et Re, identiques ou différents, indépendamment l'un de l'autre, représentent un atome d'hydrogène, ou un groupement alkyle (C₁-C₆) linéaire ou ramifié, halogène, halogénoalkyle (C₁-C₆) linéaire ou ramifié, hydroxy, alkoxy (C₁-C₆) linéaire ou ramifié, hydroxyalkyle (C₁-C₆) linéaire ou ramifié, cyano, nitro, carboxy, isothiocyanate, acyle (C₂-C₆) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, alkylthio (C₁-C₆) linéaire ou ramifié, alkyle(C₁-C₆)carbonylamino la partie alkyle étant linéaire ou ramifié, halogénoalkyle(C₁-C₆)carbonylamino la partie alkyle étant linéaire ou ramifié, aminocarbonyle, amino éventuellement substitué par un ou deux groupements alkyle (C₁-C₆) linéaire ou ramifié, tétrazolyle,
par groupement aryle, on comprend un groupement phényle, biphényle, naphtyle, dihydronaphtyle, tétrahydronaphtyle, indanyle et indényle, chacun de ces groupements étant éventuellement substitués par un ou plusieurs groupements, identiques ou différents, choisis parmi halogène, alkyle (C₁-C₆) linéaire ou ramifié, hydroxy, cyano, nitro, alkoxy (C₁-C₆) linéaire ou ramifié, acyle (C₂-C₇) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, et amino éventuellement substitué par un ou deux groupements alkyle (C₁-C₆) linéaire ou ramifié,
par groupement hétéroaryle, on comprend un système monocyclique aromatique ou bicyclique de 5 à 12 chaînons, contenant de un à trois hétéroatomes, identiques ou différents, choisis parmi oxygène, azote et soufre, et dont l'un des cycles, dans le cas d'un système bicyclique, possède un caractère aromatique, l'autre cycle pouvant être aromatique ou partiellement hydrogéné, chacun de ces groupements pouvant être éventuellement substitué par un ou plusieurs groupements identiques ou différents, choisis parmi les substituants précédemment définis dans le cas d'un groupement aryle.

2. Composés de formule (I) selon la revendication 1, **caractérisés en ce qu'**ils représentent des composés de formule (I/A) : 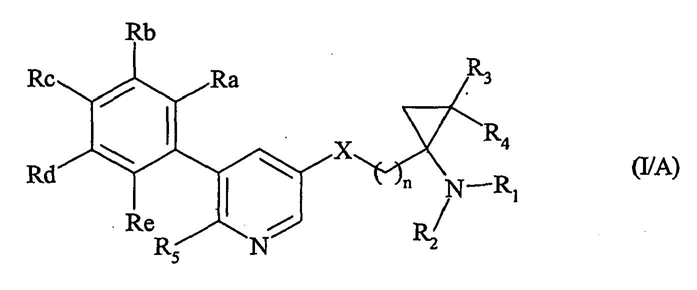 dans laquelle R₁, R₂, R₃, R₄, R₅, Ra, Rb, Rc, Rd, Re, X et n sont tels que définis précédemment.

3. Composés de formule (I) selon la revendication 1, **caractérisés en ce qu'**ils représentent des composés de formule (I/B) : 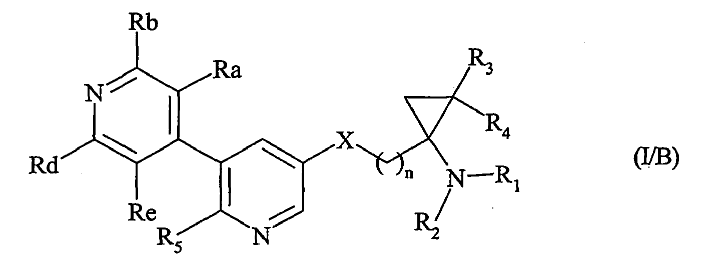 dans laquelle R₁, R₂, R₃, R₄, R₅, Ra, Rb, Rd, Re, X et n sont tels que définis précédemment.

4. Composés de formule (1) selon la revendication 1, **caractérisés en ce qu'**ils représentent des composés de formule (I/C) : 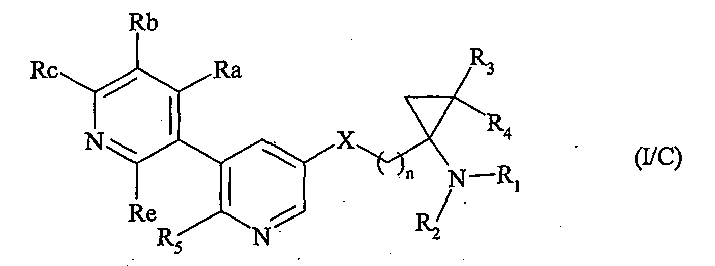 dans laquelle R₁, R₂, R₃, R₄, R₅, Ra, Rb, Rc, Re, X et n sont tels que définis précédemment.

5. Composés de formule (I) selon la revendication 1, **caractérisés en ce que** n est un entier prenant la valeur 1, leurs énantiomères, diastéréoisomères, ainsi que leurs sels d'addition à un acide ou à une base pharmaceutiquement acceptable.

6. Composés de formule (I) selon la revendication 1, **caractérisés en ce que** R₁ et R₂, identiques ou différents, indépendamment l'un de l'autre, représentent un atome d'hydrogène et un groupement alkyle (C₁-C₆) linéaire ou ramifié, leurs énantiomères, diastéréoisomères, ainsi que leurs sels d'addition à un acide ou à une base pharmaceutiquement acceptable.

7. Composés de formule (I) selon la revendication 1, **caractérisés en ce que** R₃ et R₄, identiques ou différents, représentent chacun un atome d'hydrogène ou un groupement méthyl, leurs énantiomères, diastéréoisomères, ainsi que leurs sels d'addition à un acide ou à une base pharmaceutiquement acceptable.

8. Composés de formule (I) selon la revendication 1, **caractérisés en ce que** R₅ représente un atome d'hydrogène, un atome d'halogène ou un groupement méthyl, leurs énantiomères, diastéréoisomères, ainsi que leurs sels d'addition à un acide ou à une base pharmaceutiquement acceptable.

9. Composés de formule (I) selon la revendication 1, **caractérisés en ce que** R₆ représente un atome d'hydrogène et un groupement méthyl, leurs énantiomères, diastéréoisomères, ainsi que leurs sels d'addition à un acide ou à une base pharmaceutiquement acceptable.

10. Composés de formule (I) selon la revendication 1 qui sont le :
Dichlorhydrate de [1-({[5-(3-méthoxyphényl)pyridin-3-yl]oxy}méthyl)cyclopropyl]-méthylamine,
Dichlorhydrate de [1-({[6-chloro-5-(3-méthoxyphényl)pyridin-3-yl]oxy}méthyl)-cyclopropyl]méthylamine,
Dichlorhydrate de [1-({[5-(4-méthoxyphényl)pyridin-3-yl]oxy}méthyl)cyclopropyl]-méthylamine,
Chlorhydrate de [1-({[5-(4-chlorophényl)pyridin-3-yl]oxy}méthyl)cyclopropyl]-méthylamine,
Chlorhydrate de [1-({[6-chloro-5-(4-fluorophényl)pyridin-3-yl]oxy}méthyl)-cyclopropyl]méthylamine,
Dichlorhydrate de {1-[({6-chloro-5-[4-(méthylthio)phényl]pyridin-3-yl}oxy)méthyl]-cyclopropyl}méthylamine,
Chlorhydrate de [1-({[6-chloro-5-(3,5-dichlorophényl)pyridin-3-yl]oxy}méthyl)-cyclopropyl]méthylamine,
Chlorhydrate de N-[3-(2-chloro-5-{[1-(méthylamino)cyclopropyl]méthoxy}pyridin-3-yl)-phényl]acétamide,
Dichlorhydrate de 4-(2-chloro-5-{[1-(méthylamino)cyclopropyl]méthoxy}-pyridin-3-yl)benzoate d'éthyle,
Chlorhydrate de 4-(2-chloro-5-{[1-(méthylamino)cydopropyl]méthoxy}pyridine-3-yl)benzamide,
Chlorhydrate de l'acide 4-(2-chloro-5-{[1-(uiéthylamino)cyclopropyl]méthoxy}pyridin-3-yl)benzoique,
Dichlorhydrate de (1-{[(2-chloro-3,4'-bipyridin-5-yl)oxy]méthyl}cyclopropyl)-méthylamine,
Dichlorhydrate de {1-[({6-chloro-5-[4-(2H-tétrazol-5-yl)phényl]pyridin-3-yl}oxy)-méthyl]cyclopropyl}méthylamine,
Dichlorhydrate de [1-({[5,6-bis(4-chlorophényl)pyridin-3-yl]oxy}méthyl)cyclopropyl]-méthylamine,
Trichlorhydrate de 5-(4-aminophényl)-6-méthyl-N-{[1-(méthylamino)cydopropyl]-méthyl}pyridin-3-amine,
leurs énantiomères, diastéréoisomères, ainsi que leurs sels d'addition à un acide ou à une base pharmaceutiquement acceptable.

11. Procédé de préparation des composés de formule (I), **caractérisé en ce qu'**on utilise comme produit de départ un composé de formule (II) : 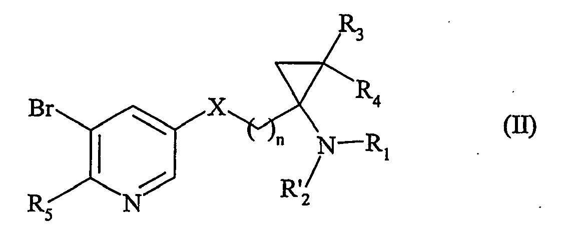 dans laquelle R'₂ représente un atome d'hydrogène, un groupement méthyle ou un groupement tert-butoxycarbonyl et R₁, R₃, R₄, R₅, X et n sont tels que définis dans la formule (I), composés de formule (II) qui sont mis à réagir avec un composé de formule (III) : 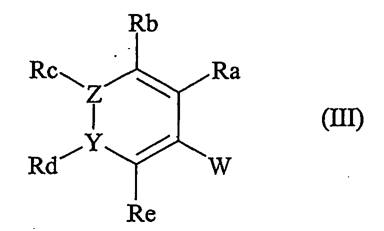 dans laquelle W représente un groupement -Sn(C₄H₉)₃, -B(OH)₂ et 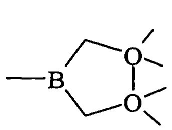 et Ra, Rb, Rc, Rd, Re, Y et Z sont tels que définis dans la formule (I), en présence de Pd(PPh₃)₄ en milieu basique pour conduire aux composés de formule (IV) : 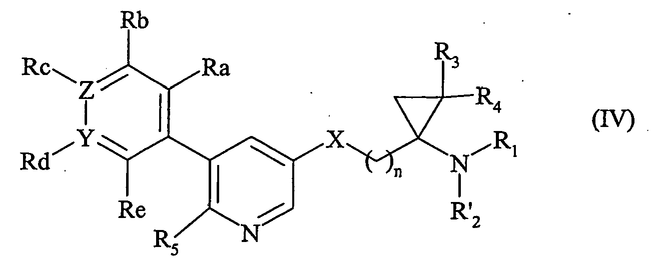 dans laquelle R₁, R'₂, R₃, R₄, R₅, X, Y, Z, Ra, Rb, Rc, Rd, Re et n sont tels que définis précédemment,
composés de formule (IV) qui sont mis en présence d'acide chlorhydrique, dans le cas où R'₂ représente un groupement tert-butoxycarbonyl, pour conduire aux composés de formule (I/a), cas particulier des composés de formule (I) : 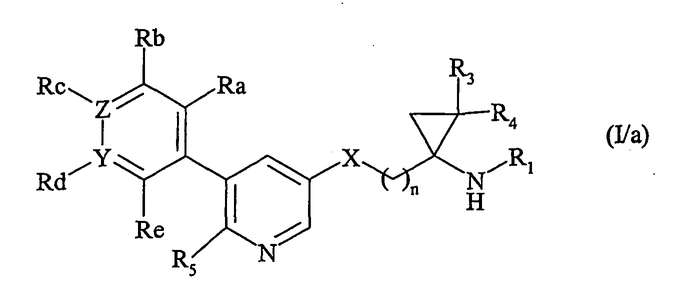 dans laquelle R₁, R₃, R₄, R₅, X, Y, Z, Ra, Rb, Rc, Rd, Re et n sont tels que définis précédemment,
composés de formule (I/a) qui sont mis à réagir avec un composé de formule (V) :
R"₂ - L₂ (V)
dans laquelle R"₂ représente un groupement alkyle (C₁-C₆) linéaire ou ramifié ou arylalkyle (C₁-C₆) linéaire ou ramifié, et L₂ représente un groupement partant usuel de la chimie organique, en milieu basique pour conduire aux composés de formule (I/b), cas particulier des composés de formule (I) : 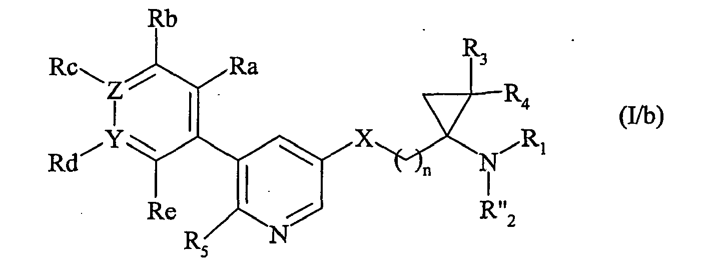 dans laquelle R₁, R"₂, R₃, R₄, R₅, X, Y, Z, Ra, Rb, Rc, Rd, Re et n sont tels que définis précédemment,
l'ensemble des composés de formules (I/a) et (I/b) formant l'ensemble des composés de l'invention qui sont purifiés, le cas échéant, selon des techniques classiques de purification, qui peuvent être séparés en leurs différents isomères, selon une technique classique de séparation et qui sont transformés, le cas échéant, en leurs sels d'addition à un acide ou à une base pharmaceutiquement acceptable.

12. Compositions pharmaceutiques contenant comme principe actif au moins un composé selon l'une quelconque des revendications 1 à 10, seul ou en combinaison avec un ou plusieurs excipients ou véhicules inertes, non toxiques, pharmaceutiquement acceptables.

13. Compositions pharmaceutiques selon la revendication 12 contenant au moins un principe actif selon l'une quelconque des revendications 1 à 10 utiles comme ligand nicotinique spécifique des récepteurs α4β2.

14. Compositions pharmaceutiques selon la revendication 12 contenant au moins un principe actif selon l'une quelconque des revendications 1 à 10 utiles dans le traitement des déficits de mémoire associés au vieillissement cérébral et aux maladies neurodégénératives, ainsi que pour le traitement des troubles de l'humeur, du syndrome de Tourette, du syndrome d'hyperactivité avec déficits attentionnels, du sevrage tabagique et de la douleur.

15. Compositions pharmaceutiques selon la revendication 12 contenant au moins un principe actif selon l'une quelconque des revendications 1 à 10, utiles dans le traitement des déficits de mémoire associés à la maladie d'Alzheimer, la maladie de Parkinson, la maladie de Pick, la maladie de Korsakoff ou les démences frontales et sous-corticales.

## Claims

1. Compounds of formula (I) : 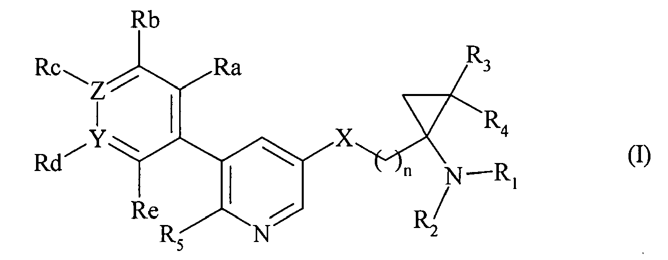 wherein :
n represents an integer of from 1 to 6 inclusive,
X represents an oxygen atom or an NR₆ group,
Y represents a carbon atom or a nitrogen atom, wherein when Y represents a nitrogen atom Rd is absent,
Z represents a carbon atom or a nitrogen atom, wherein when Z represents a nitrogen atom Rc is absent,
R₁ and R₂, which may be identical or different, each independently of the other represent a hydrogen atom, a linear or branched (C₁-C₆)alkyl group or an aryl-(C₁-C₆)alkyl group in which the alkyl moiety may be linear or branched,
R₃ and R₄, which may be identical or different, each independently of the other represent a hydrogen atom or a linear or branched (C₁-C₆)alkyl group,
R₅ represents a hydrogen atom, a linear or branched (C₁-C₆)alkyl, halogen, hydroxy, linear or branched (C₁-C₆)alkoxy, cyano, nitro, linear or branched (C₂-C₆)acyl, linear or branched (C₁-C₆)alkoxycarbonyl, linear or branched (C₁-C₆)trihaloalkyl or linear or branched (C₁-C₆)trihaloalkoxy group, or an amino group optionally substituted by one or two linear or branched (C₁-C₆)alkyl groups, or represents an aryl or heteroaryl group,
R₆ represents a hydrogen atom, a linear or branched (C₁-C₆)alkyl group or an aryl-(C₁-C₆)alkyl group in which the alkyl moiety may be linear or branched,
Ra, Rb, Rc, Rd and Re, which may be identical or different, each independently of the others represent a hydrogen atom, a linear or branched (C₁-C₆)alkyl, halogen, linear or branched (C₁-C₆)haloalkyl, hydroxy, linear or branched (C₁-C₆)alkoxy, linear or branched (C₁-C₆)hydroxyalkyl, cyano, nitro, carboxy, isothiocyanate, linear or branched (C₂-C₆)acyl, linear or branched (C₁-C₆)alkoxycarbonyl, linear or branched (C₁-C₆)trihaloalkyl, linear or branched (C₁-C₆)trihaloalkoxy or linear or branched (C₁-C₆)alkylthio group, a (C₁-C₆)alkylcarbonylamino group in which the alkyl moiety may be linear or branched, a halo-(C₁-C₆)alkylcarbonylamino group in which the alkyl moiety may be linear or branched, an aminocarbonyl group, an amino group optionally substituted by one or two linear or branched (C₁-C₆)alkyl groups, or a tetrazolyl group,
there being understood by aryl group a phenyl, biphenyl, naphthyl, dihydronaphthyl, tetrahydronaphthyl, indanyl or indenyl group, each of those groups being optionally substituted by one or more identical or different groups selected from halogen atoms, linear or branched (C₁-C₆)alkyl, hydroxy, cyano, nitro, linear or branched (C₁-C₆)alkoxy, linear or branched (C₂-C₇)acyl, linear or branched (C₁-C₆)alkoxycarbonyl, linear or branched (C₁-C₆)trihaloalkyl and linear or branched (C₁-C₆)trihaloalkoxy groups and amino groups optionally substituted by one or two linear or branched (C₁-C₆)alkyl groups,
there being understood by heteroaryl group an aromatic monocyclic system or a bicyclic system having from 5 to 12 chain members and containing from one to three identical or different hetero atoms selected from oxygen, nitrogen and sulphur, wherein one of the rings, in the case of the bicyclic system, has an aromatic character while the other ring may be aromatic or partially hydrogenated, and wherein each of those groups may optionally be substituted by one or more identical or different groups selected from the substituents defined above in the case of an aryl group.

2. Compounds of formula (I) according to claim 1, **characterised in that** they are compounds of formula (I/A) : 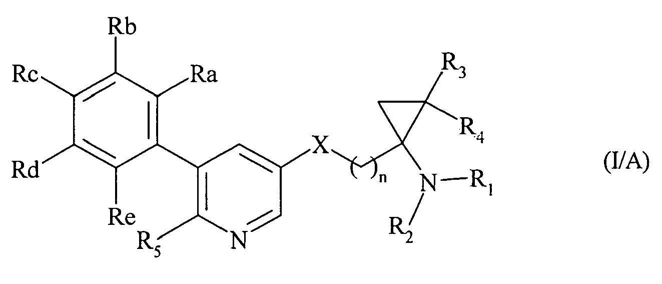 wherein R₁, R₂, R₃, R₄, R₅, Ra, Rb, Rc, Rd, Re, X and n are as defined hereinbefore.

3. Compounds of formula (I) according to claim 1, **characterised in that** they are compounds of formula (I/B) : 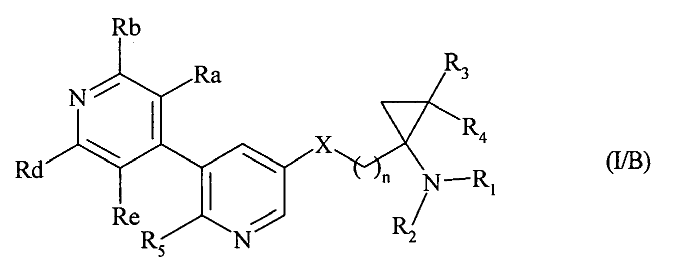 wherein R₁, R₂, R₃, R₄, R₅, Ra, Rb, Rd, Re, X and n are as defined hereinbefore.

4. Compounds of formula (I) according to claim 1, **characterised in that** they are compounds of formula (I/C) : 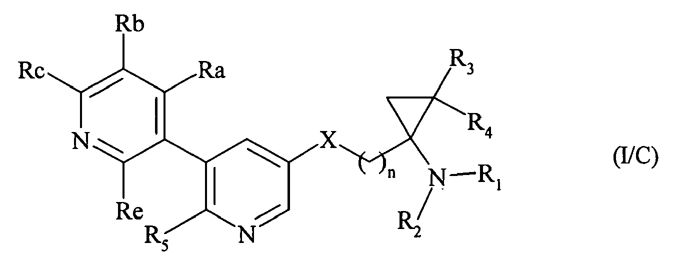 wherein R₁, R₂, R₃, R₄, R₅, Ra, Rb, Rc, Re, X and n are as defined hereinbefore.

5. Compounds of formula (I) according to claim 1, **characterised in that** n is an integer having the value 1, their enantiomers, diastereoisomers, and also addition salts thereof with a pharmaceutically acceptable acid or base.

6. Compounds of formula (I) according to claim 1, **characterised in that** R₁ and R₂, which may be identical or different, each independently of the other represent a hydrogen atom or a linear or branched (C₁-C₆)alkyl group, their enantiomers, diastereoisomers, and also addition salts thereof with a pharmaceutically acceptable acid or base.

7. Compounds of formula (I) according to claim 1, **characterised in that** R₃ and R₄, which may be identical or different, each represent a hydrogen atom or a methyl group, their enantiomers, diastereoisomers, and also addition salts thereof with a pharmaceutically acceptable acid or base.

8. Compounds of formula (I) according to claim 1, **characterised in that** R₅ represents a hydrogen atom, a halogen atom or a methyl group, their enantiomers, diastereoisomers, and also addition salts thereof with a pharmaceutically acceptable acid or base.

9. Compounds of formula (I) according to claim 1, **characterised in that** R₆ represents a hydrogen atom or a methyl group, their enantiomers, diastereoisomers, and also addition salts thereof with a pharmaceutically acceptable acid or base.

10. Compounds of formula (I) according to claim 1 which are:
[1-({[5-(3-methoxyphenyl)pyridin-3-yl]oxy}methyl)cyclopropyl]methylamine dihydrochloride,
[1-({[6-chloro-5-(3-methoxyphenyl)pyridin-3-yl]oxy}methyl)cyclopropyl]methylamine dihydrochloride,
[1-({[5-(4-methoxyphenyl)pyridin-3-yl]oxy}methyl)cyclopropyl]methylamine dihydrochloride,
[1-({[5-(4-chlorophenyl)pyridin-3-yl]oxy}methyl)cyclopropyl]methylamine hydrochloride,
[1-({[6-chloro-5-(4-fluorophenyl)pyridin-3-yl]oxy}methyl)cyclopropyl]methylamine hydrochloride,
{1-[({6-chloro-5-[4-(methylthio)phenyl]pyridin-3-yl}oxy)methyl]cyclopropyl}methylamine dihydrochloride,
[1-({[6-chloro-5-(3,5-dichlorophenyl)pyridin-3-yl]oxy}methyl)cyclopropyl]methylamine hydrochloride,
N-[3-(2-chloro-5-{[1-(methylamino)cyclopropyl]methoxy} pyridin-3-yl)phenyl] acetamide hydrochloride,
ethyl 4-(2-chloro-5-{[1-(methylamino)cyclopropyl]methoxy}pyridin-3-yl)benzoate dihydrochloride,
4-(2-chloro-5-{[1-(methylamino)cyclopropyl]methoxy}pyridin-3-yl)benzamide hydrochloride,
4-(2-chloro-5-{[1-(methylamino)cyclopropyl]methoxy}pyridin-3-yl)benzoic acid hydrochloride,
(1-{[(2-chloro-3,4'-bipyridin-5-yl)oxy]methyl}cyclopropyl)methylamine dihydrochloride,
{1-[({6-chloro-5-[4-(2H-tetrazol-5-yl)phenyl]pyridin-3-yl}oxy)methyl]cyclopropyl}methylamine dihydrochloride,
[1-({[5,6-bis(4-chlorophenyl)pyridin-3-yl]oxy}methyl)cyclopropyl]methylamine dihydrochloride,
5-(4-aminophenyl)-6-methyl-N-{[1-(methylamino)cyclopropyl]methyl}pyridin-3-amine trihydrochloride,
their enantiomers, diastereoisomers, and also addition salts thereof with a pharmaceutically acceptable acid or base.

11. Process for the preparation of compounds of formula (I), **characterised in that** there is used as starting material a compound of formula (II) : 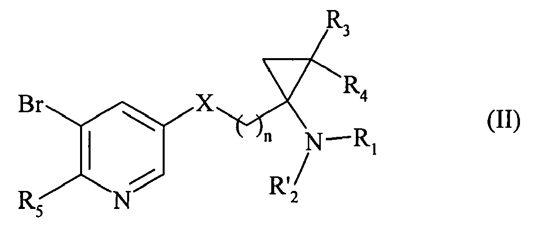 wherein R'₂ represents a hydrogen atom, a methyl group or a *tert*-butoxycarbonyl group and R₁, R₃, R₄, R₅, X and n are as defined for formula (I), which compounds of formula (II) are reacted with a compound of formula (III) : 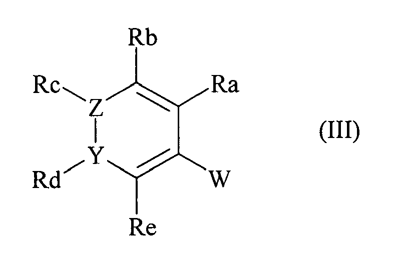 wherein W represents an -Sn(C₄H₉)₃, -B(OH)₂ or 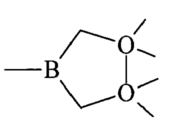 group, and Ra, Rb, Rc, Rd, Re, Y and Z are as defined for formula (I), in the presence of Pd(PPh₃)₄, in basic medium, to yield compounds of formula (IV) : 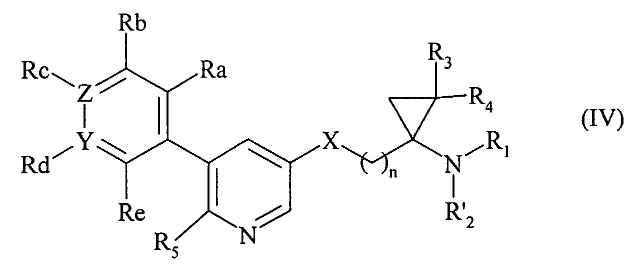 wherein R₁, R'₂, R₃, R₄, R₅, X, Y, Z, Ra, Rb, Rc, Rd, Re and n are as defined hereinbefore,
which compounds of formula (IV), when R'₂ represents a *tert*-butoxycarbonyl group, are placed in the presence of hydrochloric acid to yield compounds of formula (I/a), a particular case of the compounds of formula (I) : 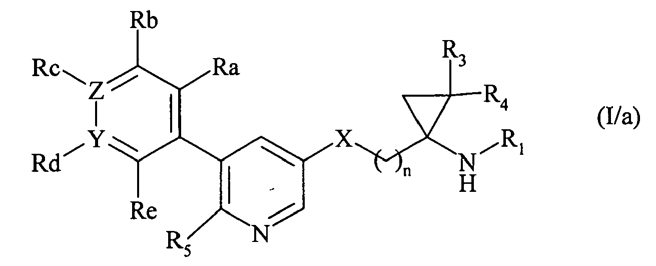 wherein R₁, R₃, R₄, R₅, X, Y, Z, Ra, Rb, Rc, Rd, Re and n are as defined hereinbefore,
which compounds of formula (I/a) are reacted with a compound of formula (V) :
R"₂-L₂ (V)
wherein R"₂ represents a linear or branched (C₁-C₆)alkyl group or an aryl-(C₁-C₆)alkyl group in which the alkyl moiety may be linear or branched, and L₂ represents a leaving group customary in organic chemistry, in basic medium, to yield compounds of formula (I/b), a particular case of the compounds of formula (I) : 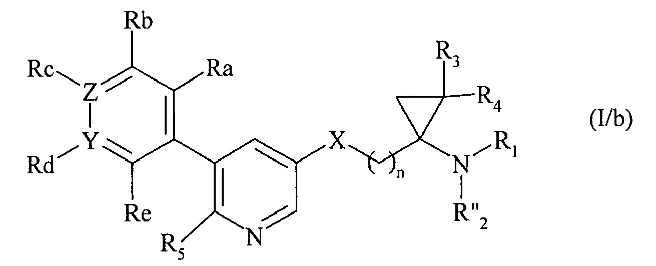 wherein R₁, R"₂, R₃, R₄, R₅, X, Y, Z, Ra, Rb, Rc, Rd, Re and n are as defined hereinbefore,
the totality of the compounds of formulae (I/a) and (I/b) constituting the totality of the compounds of the invention, which are purified, where appropriate, according to conventional purification techniques, which may be separated into their different isomers according to a conventional separation technique, and which are converted, where appropriate, into addition salts with a pharmaceutically acceptable acid or base.

12. Pharmaceutical compositions comprising as active ingredient at least one compound according to any one of claims 1 to 10, alone or in combination with one or more pharmaceutically acceptable, inert, non-toxic excipients or carriers.

13. Pharmaceutical compositions according to claim 12 comprising at least one active ingredient according to any one of claims 1 to 10 for use as a specific nicotinic ligand of α4β2 receptors.

14. Pharmaceutical compositions according to claim 12 comprising at least one active ingredient according to any one of claims 1 to 10 for use in the treatment of deficiencies of memory associated with cerebral ageing and with neurodegenerative diseases, and also for the treatment of mood disorders, Tourette's syndrome, attention-deficit hyperactivity syndrome, tobacco withdrawal and pain.

15. Pharmaceutical compositions according to claim 12 comprising at least one active ingredient according to any one of claims 1 to 10 for use in the treatment of deficiencies of memory associated with Alzheimer's disease, Parkinson's disease, Pick's disease, Korsakoff's disease or frontal lobe and subcortical dementias.

## Patentansprüche

1. Verbindungen der Formel (I): in der:
n eine ganze Zahl mit einem Wert zwischen 1 und 6 einschließlich bedeutet,
X ein Sauerstoffatom oder eine Gruppe NR₆ bedeutet,
Y ein Kohlenstoffatom oder ein Stickstoffatom bedeutet, mit der Maßgabe, dass, wenn Y ein Stickstoffatom darstellt, Rd nicht vorhanden ist,
Z ein Kohlenstoffatom oder ein Stickstoffatom bedeutet, mit der Maßgabe, dass, wenn Z ein Stickstoffatom darstellt, Rc nicht vorhanden ist.
R₁ und R₂, die gleichartig oder verschieden sind, unabhängig voneinander ein Wasserstoffatom, eine geradkettige oder verzweigte (C₁-C₆)-Alkylgruppe oder eine geradkettige oder verzweigte Aryl-(C₁-C₆)-alkylgruppe bedeuten.
R₃ und R₄, die gleichartig oder verschieden sind, unabhängig voneinander ein Wasserstoffatom oder eine geradkettige oder verzweigte (C₁-C₆)-Alkylgruppe bedeuten,
R₅ ein Wasserstoffatom, eine geradkettige oder verzweigte (C₁-C₆)-Alkylgruppe, Halogen, eine Hydroxygruppe, eine geradkettige oder verzweigte (C₁-C₆)-Alkoxygruppe, eine Cyanogruppe, eine Nitrogruppe, eine geradkettige oder verzweigte (C₂-C₆)-Acylgruppe, eine geradkettige oder verzweigte (C₁-C₆)-Alkoxycarbonylgruppe, eine geradkettige oder verzweigte (C₁₋C₆)-Trihalogenalkylgruppe, eine geradkettige oder verzweigte (C₁-C₆)-Trihalogenalkoxygruppe, eine gegebenenfalls durch eine oder zwei geradkettige oder verzweigte (C₁-C₆)-Alkylgruppen substituierte Aminogruppe, eine Arylgruppe oder eine Heteroarylgruppe bedeutet,
R₆ ein Wasserstoffatom, eine geradkettige oder verzweigte (C₁-C₆)-Alkylgruppe oder eine geradkettige oder verzweigte Aryl-(C₁-C₆)-alkylgruppe bedeutet,
Ra, Rb, Rc, Rd und Re, die gleichartig oder verschieden sind, unabhängig voneinander ein Wasserstoffatom oder eine geradkettige oder verzweigte (C₁-C₆)-Alkylgruppe, ein Halogenatom, eine geradkettige oder verzweigte (C₁-C₆)-Halogenalkylgruppe, eine Hydroxygruppe, eine geradkettige oder verzweigte (C₁-C₆)-Alkoxygruppe, eine geradkettige oder verzweigte (C₁-C₆)-Hydroxyalkylgruppe, eine Cyanogruppe, eine Nitrogruppe, eine Carboxygruppe, eine Isothiocyanatgruppe, eine geradkettige oder verzweigte (C₂-C₆)-Acylgruppe, eine geradkettige oder verzweigte (C₁-C₆)-Alkoxycarbonylgruppe, eine geradkettige oder verzweigte (C₁-C₆)-Trihalogenalkylgruppe, eine geradkettige oder verzweigte (C₁-C₆)-Trihalogenalkoxygrupe, eine geradkettige oder verzweigte (C₁-C₆)-Alkylthiogruppe, eine (C₁-C₆)-Alkylcarbonylaminogruppe, deren Alkylrest geradkettig oder verzweigt ist, eine (C₁-C₆)-Halogenalkylcarbonylaminogruppe, deren Alkylrest geradkettig oder verzweigt ist, eine Aminocarbonylgruppe, eine gegebenenfalls durch eine oder zwei geradkettige oder verzweigte (C₁-C₆)-Alkylgruppen substituierte Aminogruppe oder eine Tetrazolylgruppe bedeuten,
wobei man unter einer Arylgruppe eine Phenyl-, Biphenyl-, Naphthyl-, Dihydronaphthyl-, Tetrahydronaphthyl-, Indanyl- oder Indenylgruppe versteht, wobei jede dieser Gruppen gegebenenfalls durch eine oder mehrere gleichartige oder verschiedenartige Gruppen substituiert ist ausgewählt aus Halogen, geradkettigem oder verzweigtem (C₁-C₆)-Alkyl, Hydroxy, Cyano, Nitro, geradkettigem oder verzweigtem (C₁-C₆)-Alkoxy, geradkettigem oder verzweigtem (C₂-C₇)-Acyl, geradkettigem oder verzweigtem (C₁-C₆)-Alkoxcarbonyl, geradkettigem oder verzweigtem (C₁-C₆)-Trihalogenalkyl, geradkettigem oder verzweigtem (C₁-C₆)-Trihalogenalkoxy und gegebenenfalls durch eine oder zwei geradkettige oder verzweigte (C₁-C₆)-Alkylgruppen substituiertem Amino.
man unter einer Heteroarylgruppe ein monocyclisches aromatisches oder bicyclisches System mit 5 bis 12 Kettengliedern versteht, das ein bis drei gleichartige oder verschiedenartige Heteroatome aufweist ausgewählt aus Sauerstoff, Stickstoff und Schwefel, wobei einer der Ringe im Fall eines bicyclischen Systems einen aromatischen Charakter besitzt und der andere Ring aromatisch oder teilweise hydriert sein kann, wobei jede dieser Gruppen gegebenenfalls durch eine oder mehrere gleichartige oder verschiedenartige Gruppen substituiert ist, ausgewählt aus den oben für die Arylgruppe definierten Substituenten.

2. Verbindungen der Formel (I) nach Anspruch 1, **dadurch gekennzeichnet, dass** sie der Formel (I/A) entsprechen: 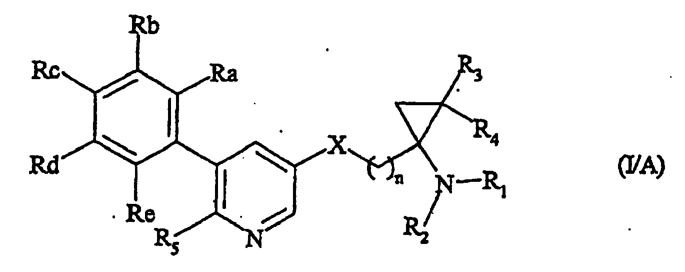 in der R₁, R₂, R₃, R₄. R₅, Ra, Rb, Rc, Rd, Re, X und n die oben angegebenen Bedeutungen besitzen.

3. Verbindungen der Formel (I) nach Anspruch 1, **dadurch gekennzeichnet, dass** sie der Formel (I/B) entsprechen: 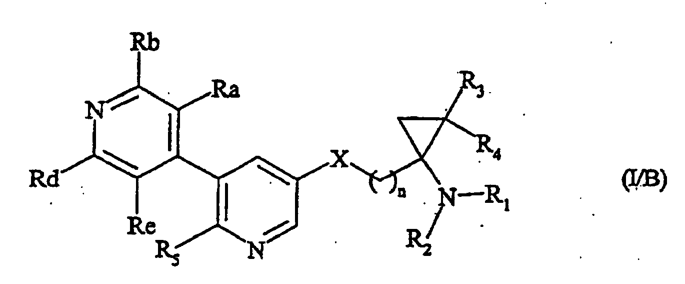 in der R₁, R₂, R₃, R₄, R₅, Ra, Rb, Rd, Re, X und n die oben angegebenen Bedeutungen besitzen.

4. Verbindungen der Formel (I) nach Anspruch 1, **dadurch gekennzeichnet, dass** sie der Formel (I/C) entsprechen: 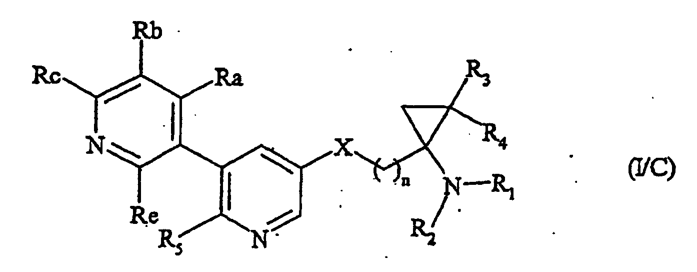 in der R₁, R₂, R₃, R₄, R₅, Ra, Rb, Rc, Re, X und n die oben angegebenen Bedeutungen besitzen.

5. Verbindungen der Formel (I) nach Anspruch 1, **dadurch gekennzeichnet, dass** n eine ganze Zahl mit einem Wert von 1 ist, deren Enantiomere, Diastereoisomere sowie deren Additionssalze mit einer pharmazeutisch annehmbaren Säure oder Base.

6. Verbindungen der Formel (I) nach Anspruch 1, **dadurch gekennzeichnet, dass** R₁ und R₂, die gleichartig oder verschieden sind, unabhängig voneinander ein Wasserstoffatom oder eine geradkettige oder verzweigte (C₁-C₆)-Alkylgruppe bedeuten, deren Enantiomere, Diastereoisomere sowie deren Additionssalze mit einer pharmazeutisch annehmbaren Säure oder Base.

7. Verbindungen der Formel (I) nach Anspruch 1, **dadurch gekennzeichnet, dass** R₃ und R₄, die gleichartig oder verschieden sind, unabhängig voneinander ein Wasserstoffatom oder eine Methylgruppe bedeuten, deren Enantiomere, Diastereoisomere sowie deren Additionssalze mit einer pharmazeutisch annehmbaren Säure oder Base.

8. Verbindungen der Formel (I) nach Anspruch 1, **dadurch gekennzeichnet, dass** R₅ ein Wasserstoffatom, ein Halogenatom oder eine Methylgruppe bedeutet, deren Enantiomere, Diastereoisomere sowie deren Additionssalze mit einer pharmazeutisch annehmbaren Säure oder Base.

9. Verbindungen der Formel (I) nach Anspruch 1, **dadurch gekennzeichnet, dass** R₆ ein Wasserstoffatom oder eine Methylgruppe bedeutet, deren Enantiomere, Diastereoisomere sowie deren Additionssalze mit einer pharmazeutisch annehmbaren Säure oder Base.

10. Verbindungen der Formel (I) nach Anspruch 1, nämlich:
[1-({[5-(3-Methoxyphenyl)-pyridin-3-yl]-oxy}-methyl)-cyclopropyl]-methylamin-Dihydrochlorid,
[1-({[6-Chlor-5-(3-methoxyphenyl)-pyridin-3-yl]-oxy}-methyl)-cyclopropyl]-methylamin-Dihydrochlorid,
[1-({[5-(4-Methoxyphenyl)-pyridin-3-yl]-oxy}-methyl)-cyclopropyl]-methylamin-Dihydrochlorid,
[1-({[5-(4-Chlorphenyl)-pyridin-3-yl]-oxy}-methyl)-cyclopropyl]-methylamin-Hydrochlorid,
[1-({[6-Chlor-5-(4-fluorphenyl)-pyridin-3-yl]-oxy}-methyl)-cyclopropyl]-methylamin-Hydrochlorid,
{1-[({6-Chlor-5-[4-(methylthio)-phenyl]-pyridin-3-yl}-oxy)-methyl]-cyclopropyl}methylamin-Dihydrochlorid,
[1-({[6-Chlor-5-(3,5-dichlorphenyl)-pyridin-3-yl]-oxy}-methyl)-cyclopropyl]-methylamin-Hydrochlorid,
N-[3-(2-Chlor-5-{[1-(methylamino)-cyclopropyl]-methoxy}-pyridin-3-yl)-phenyl]-acetamid-Hydrochlorid,
4-(2-Chlor-5-{[1-(methylamino)-cyclopropyl]-methoxy}-pyridin-3-yl)-benzoesäureethylester-Dihydrochlorid,
4-(2-Chlor-5-{[1-(methylamino)-cyclopropyl]-methoxy}-pyridin-3-yl)-benzamid-Hydrochlorid,
4-(2-Chlor-5-{[1-(methylamino)-cyclopropyl]-methoxy}-pyridin-3-yl)-benzoesäure-Hydrochlorid,
(1-{[2-Chlor-3,4'-bipyridin-5-yl)-oxy]-methyl}-cyclopropyl)-methylamin-Dihydrochlorid,
{1-[({6-Chlor-5-[4-(2H-tetrazol-5-yl)-phenyl]-pyridin-3-yl}-ocy)-methyl]-cyclopropyl}-methylamin-Dihydrochlorid,
[1-({[5,6-Bis(4-chlorphenyl)-pyridin-3-yl]-oxy}-methyl)-cyclopropyl]-methylamin-Dihydrochlorid,
5-(4-Aminophenyl)-6-methyl-N-{[1-(methylamino)-cyclopropyl]-methyl}-pyridin-3-amin-Trihydrochlorid,
deren Enantiomere, Diastereoisomere sowie deren Additionssalze mit einer pharmazeutisch annehmbaren Säure oder Base.

11. Verfahren zur Herstellung der Verbindungen der Formel (I), **dadurch gekennzeichnet, dass** man als Ausgangsprodukt eine Verbindung der Formel (II) verwendet: 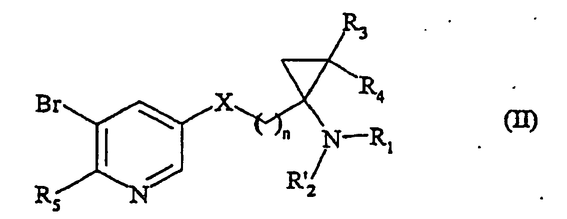 in der R'₂ ein Wasserstoffatom, eine Methylgruppe oder eine tert.-Butoxycarbonylgruppe bedeutet und R₁, R₃, R₄, R₅, X und n die bezüglich der Formel (I) angegebenen Bedeutungen besitzen, welche Verbindungen der Formel (II) mit einer Verbindung der Formel (III): 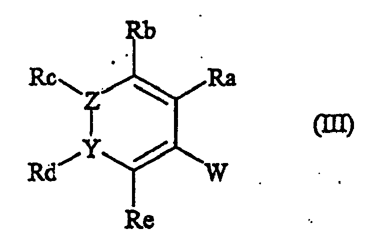 in der W eine Gruppe -Sn(C₄H₉)₃, -B(OH)₂ oder 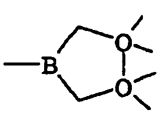 darstellt und Ra, Rb, Rc, Rd. Re, Y und Z die bezüglich der Formel (I) angegebenen Bedeutungen besitzen, in Gegenwart von Pd(PPH₃)₄ in basischem Medium umgesetzt werden zur Bildung der Verbindungen der Formel (IV): 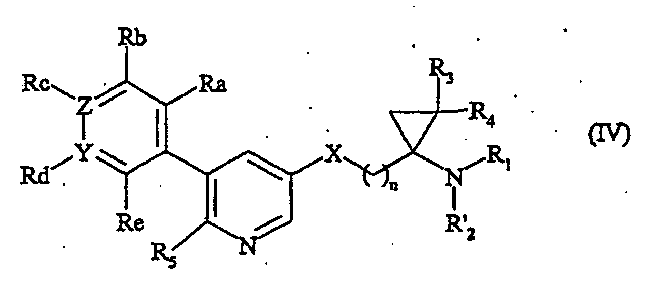 in der R₁, R'₂, R₃, R₄, R₅, X, Y, Z, Ra, Rb, Rc, Rd, Re und n die oben angegebenen Bedeutungen besitzen,
welche Verbindungen der Formel (IV) in dem Fall, dass R'₂ eine tert.-Butoxycarbonylgruppe bedeutet, mit Chlorwasserstoffsäure behandelt werden zur Bildung der Verbindungen der Formel (I/a), einem Sonderfall der Verbindungen der Formel (I): 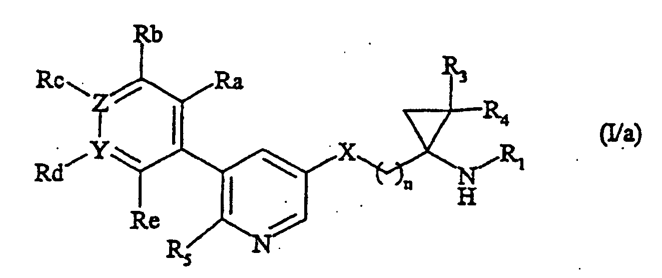 in der R₁, R₃, R₄, R₅, X, Y, Z, Ra, Rb, Rc, Rd, Re und n die oben angegebenen Bedeutungen besitzen,
welche Verbindungen der Formel (I/a) mit einer Verbindung der Formel (V):
R"₂ - L₂ (V)
in der R"₂ eine geradkettige oder verzweigte (C₁-C₆)-Alkylgruppe oder eine geradkettige oder verzweigte Aryl-(C₁-C₆)-alkylgruppe bedeutet und L₂ eine in der organischen Chemie übliche austretende Gruppe darstellt, in basischem Medium umgesetzt werden zur Bildung der Verbindungen der Formel (I/b), einem Sonderfall der Verbindungen der Formel (I): 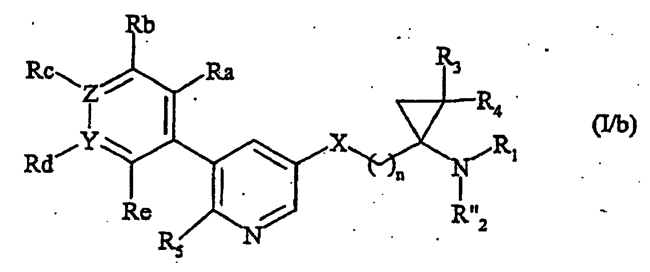 in der R₁, R"₂, R₃, R₄, R₅, X. Y, Z. Ra, Rb, Rc, Rd, Re und n die oben angegebenen Bedeutungen besitzen,
wobei die Verbindungen der Formeln (I/a) und (I/b), die die Gesamtheit der erfindungsgemäßen Verbindungen bilden, gegebenenfalls mit Hilfe klassischer Reinigungsmethoden gereinigt werden, mit Hilfe einer klassischen Trennmethode in ihre verschiedenen Isomeren aufgetrennt werden können und gegebenenfalls in ihre Additionssalze mit einer pharmazeutisch annehmbaren Säure oder Base überführt werden können.

12. Pharmazeutische Zubereitungen, enthaltend als Wirkstoff mindestens eine Verbindung nach einem der Ansprüche 1 bis 10 allein oder in Kombination mit einem oder mehreren inerten, nichttoxischen und pharmazeutisch annehmbaren Hilfsstoffen oder Trägermaterialien.

13. Pharmazeutische Zubereitungen nach Anspruch 12, enthaltend mindestens einen Wirkstoff nach einem der Ansprüche 1 bis 10, nützlich als spezifischer nicotinischer Ligand für die Rezeptoren α4β2.

14. Pharmazeutische Zubereitungen nach Anspruch 12, enthaltend mindestens einen Wirkstoff nach einem der Ansprüche 1 bis 10, nützlich bei der Behandlung von mit dem Altern des Gehirns und neurodegenerativen Erkrankungen verknüpften Gedächtnisdefiziten sowie zur Behandlung von Gemütsstörungen, des Tourette-Syndroms, des Hyperaktivitätssyndroms mit Aufmerksamkeitsdefiziten, dem Tabakentzug und von Schmerzen.

15. Pharmazeutische Zubereitungen nach Anspruch 12, enthaltend mindestens einen Wirkstoff nach einem der Ansprüche 1 bis 10, für die Behandlung von mit der Alzheimerschen Krankheit verknüpften Gedächtnisdefiziten, der Parkinsonschen Krankheit, der Pickschen Krankheit, der Korsakoff-Krankheit oder frontalen und subkortikalen Demenzen.
